# EUROPEAN PATENT APPLICATION

(11) **EP 4 163 390 A1**
(43) Date of publication of application: **12.04.2023**
(21) Application number: 21817287.2
(22) Date of filing: 02.06.2021
(51) Int. Cl.: C12Q 1/6806, C12Q 1/6869

(54) **METHOD FOR ANALYZING TARGET NUCLEIC ACID FROM CELL**

(30) Priority: 03.06.2020 CN 202010495334; 05.06.2020 CN 202010506791
(71) Applicant: Tenk Genomics, Inc., Beijing 101300 (CN)
(72) Inventor: SHI, Weiyang, Qingdao, Shandong 266100 (CN)
(74) Representative: Böhm, Brigitte
(86) International application number: PCT/CN2021/097800
(87) International publication number: WO 2021/244557

(57) **Abstract**

The present application relates to a method for analyzing target nucleic acid from a cell, comprising: 1) providing the following discrete partition: target nucleic acid derived from a single cell and added with an oligonucleotide adapter sequence, and a solid support with at least one oligonucleotide tag attached, wherein each oligonucleotide tag comprises a first chain and a second chain, the first chain comprises a barcode sequence and a hybridization sequence located at the 3' end of the barcode sequence, the second chain comprises a first portion complementary to the hybridization sequence of the first chain and a second portion complementary to the oligonucleotide adapter sequence attached to the target nucleic acid, and the first chain and the second chain form a partially double-stranded structure, or the second chain and the target nucleic acid attached form a partially double-stranded structure; and 2) in the discrete partition, the oligonucleotide tag is linked to the target nucleic acid attached, thereby producing barcoded target nucleic acid.

## Description

### TECHNICAL FIELD

The present application relates to the field of biomedicine, in particular to a method for analyzing a target nucleic acid from a cell, and related preparations.

### BACKGROUND OF THE INVENTION

Currently, nucleic acid sequencing technologies have experienced rapid and great progress. Sequencing technologies produce a large number of sequence data, which can be used for the research and interpretation of genomes and genome regions, and provide information widely used in routine biological research and diagnosis. Genome sequencing can be used to obtain information of various biomedical backgrounds, including diagnostics, prognosis, biotechnology and forensic biology. Sequencing includes Maxam-Gilbert sequencing and chain termination method or de novo sequencing method (including shotgun sequencing and bridge PCR), or new generation methods including polymerase clone sequencing, 454 pyrosequencing, Illumina sequencing, SOLiD sequencing, Ion Torrent semiconductor sequencing, HeliScope single molecule sequencing, [image] sequencing, etc. For most sequencing applications, samples such as nucleic acid samples are treated before being introduced into the sequencer.

Traditional research methods of genome or transcriptome expression are usually carried out at multi-cell level. Therefore, the final signal value is the average of a plurality of cells, and the information of cell heterogeneity is lost. For example, currently, the analysis of mRNA content of cells by direct sequencing relies on the analysis of a large number of mRNA obtained from tissue samples containing millions of cells, which means that when gene expression is analyzed in a large number of mRNA, a lot of functional information presented in single cells will be lost or blurred; in addition, dynamic processes such as cell cycle can't be observed based on the overall average. Similarly, some cell types in complex tissues (e.g., brain) can be studied only by analyzing cells alone.

Currently, there are no suitable cell surface markers for isolated single cells to study, and even if there are suitable cell surface markers, a small number of single cells are still insufficient to capture the range of natural variation in gene expression. Therefore, there is a need for an analytical method that can be used for analyzing gene information in a large number of single cells.

### SUMMARY OF THE INVENTION

The present application provides a method for analyzing a target nucleic acid from a cell, including:
a) providing the discrete partition comprising the following:
   i. a target nucleic acid derived from a single cell, wherein at least a part of the target nucleic acids is added with oligonucleotide adapter sequences to become target nucleic acids attached; and
   ii. a solid support with at least one oligonucleotide tag attached, wherein each of the oligonucleotide tags comprises a first chain and a second chain, the first chain comprises a barcode sequence and a hybridization sequence located at the 3' end of the barcode sequence, the second chain comprises a first portion complementary to the hybridization sequence of the first chain and a second portion complementary to the oligonucleotide adapter sequence attached to the target nucleic acid, and the first chain and the second chain form a partially double-stranded structure, or the second chain and the target nucleic acid attached form a partially double-stranded structure; and
b) in the discrete partition, the oligonucleotide tag is linked to the target nucleic acid attached, thereby producing a barcoded target nucleic acid.

In certain embodiments, the oligonucleotide tag is releasably attached to the solid support.

In certain embodiments, it includes releasing the at least one oligonucleotide tag from the solid support, and linking the released oligonucleotide tag to the target nucleic acid attached in b), thereby producing a barcoded target nucleic acid.

In certain embodiments, the oligonucleotide tag is directly or indirectly attached to the solid support through the 5' end of its first chain.

In certain embodiments, a ligase is further comprised in the discrete partition, and the ligase links the oligonucleotide tag to the target nucleic acid attached.

In certain embodiments, the ligase includes T4 ligase.

In certain embodiments, in the barcoded target nucleic acid, the target nucleic acid sequence is located at the 3' end of the barcode sequence.

In certain embodiments, the solid support is a bead.

In certain embodiments, the bead is a magnetic bead.

In certain embodiments, the discrete partition is a hole or droplet.

In certain embodiments, the barcode sequence comprises a cell barcode sequence, and each oligonucleotide tag attached to the same solid support comprises the same cell barcode sequence.

In certain embodiments, the cell barcode sequence comprises at least 2 cell barcode segments separated by a linker sequence.

In certain embodiments, a) includes co-distributing the target nucleic acid derived from the single cell and the solid support attached with at least one oligonucleotide tag into the discrete partition.

In certain embodiments, b) includes linking the hybridization sequence of the first chain in the oligonucleotide tag to the oligonucleotide adapter attached to the target nucleic acid, thereby producing the barcoded target nucleic acid.

In certain embodiments, b) includes hybridizing the second part of the second chain in the oligonucleotide tag with the oligonucleotide adapter attached to the target nucleic acid, and linking the hybridization sequence of the first chain in the oligonucleotide tag to the oligonucleotide adapter attached to the target nucleic acid, thereby producing the barcoded target nucleic acid.

In certain embodiments, the target nucleic acid attached comprises a unique molecular identification region.

In certain embodiments, the unique molecular identification region is located between the oligonucleotide adapter sequence and the target nucleic acid sequence.

In certain embodiments, the oligonucleotide tag further comprises an amplification primer recognition region.

In certain embodiments, the amplification primer recognition region is a general amplification primer recognition region.

In certain embodiments, the method further includes:
c) obtaining the characterization result of the barcoded target nucleic acid; and
d) identifying the sequence of the target nucleic acid as deriving from the single cell based at least in part on the presence of the same cell barcode sequence in the characterization result obtained in c).

In certain embodiments, the method further includes, releasing the barcoded target nucleic acid from the discrete partition after b) and before c).

In certain embodiments, c) includes sequencing the barcoded target nucleic acid, thereby obtaining the characterization result.

In certain embodiments, the method further includes assembling continuous nucleic acid sequences of at least a part of the genome of the single cell from the sequences of the barcoded target nucleic acids.

In certain embodiments, the single cell is characterized based on the nucleic acid sequences of at least a part of the genome of the single cell.

In certain embodiments, each of the discrete partitions includes at most the target nucleic acids derived from the single cell.

In certain embodiments, the method further includes identifying the single nucleic acid sequence in the barcoded target nucleic acid as deriving from a given nucleic acid in the target nucleic acid based at least in part on the presence of the unique molecular identification region.

In certain embodiments, the target nucleic acid includes an exogenous nucleic acid including that linked to proteins, lipids and/or small molecule compounds which can bind to target molecules in the cell.

In certain embodiments, the method further includes determining the amount of a given nucleic acid in the target nucleic acid based on the presence of the unique molecular identification region.

In certain embodiments, it includes pretreatment of the cell before a).

In certain embodiments, the pretreatment includes fixing the cell.

In certain embodiments, the cell is immobilized using a fixing agent selected from one or more of the group consisting of formaldehyde, paraformaldehyde, methanol, ethanol, acetone, glutaraldehyde, osmic acid and potassium dichromate.

In certain embodiments, the pretreatment includes exposing the nucleus of the cell.

In certain embodiments, the pretreatment includes treating the cell with a detergent including Triton, Tween, SDS, NP-40 and/or digitonin.

In certain embodiments, the target nucleic acid includes one or more selected from the group consisting of DNA, RNA and cDNA.

In certain embodiments, it further includes amplifying the barcoded target nucleic acid after b) and before c).

In certain embodiments, it includes releasing the barcoded target nucleic acid from the discrete partition after b) and before c), and the amplification is performed after the barcoded target nucleic acid is released from the discrete partition.

In certain embodiments, an amplification primer is used in the amplification, and the amplification primer comprises a random guide sequence.

In certain embodiments, the random guide sequence is a random hexamer.

In certain embodiments, the amplification includes at least partially hybridizing the random guide sequence with the barcoded target nucleic acid and extending the random guide sequence in a template-oriented manner.

In certain embodiments, it includes releasing at least a part of the target nucleic acids from the single cell in the discrete partition to the outside of the cell, and linking the released target nucleic acids to the oligonucleotide tags in b), thereby producing the barcoded target nucleic acids.

In certain embodiments, it includes introducing at least a part of the oligonucleotide tags released from the solid support into the single cell, and linking them to the target nucleic acids in b), thereby producing barcoded target nucleic acids.

In certain embodiments, it includes co-distributing the target nucleic acid derived from the single cell and the solid support attached with at least one oligonucleotide tag into the discrete partition by using a microfluidic device.

In certain embodiments, the discrete partition is a droplet, and the microfluidic device is a droplet generator.

In certain embodiments, the microfluidic device includes a first input channel and a second input channel, which meet at a junction in fluid communication with an output channel.

In certain embodiments, the method further includes introducing a sample comprising the target nucleic acid into the first input channel, and introducing the solid support attached with at least one oligonucleotide tag into the second input channel, thereby generating a mixture of the sample and the solid support in the output channel.

In certain embodiments, the output channel is in fluid communication with a third input channel at a junction.

In certain embodiments, it further includes introducing oil into the third input channel, so that aqueous droplets in the water-in-oil emulsion are formed as the discrete partitions.

In certain embodiments, each of the discrete partitions includes at most the target nucleic acids from the single cell.

In certain embodiments, the first input channel and the second input channel form a substantially perpendicular angle with each other.

In certain embodiments, the target nucleic acid includes cDNA derived from RNA in the single cell.

In certain embodiments, the RNA includes mRNA.

In certain embodiments, it includes reverse transcription of the RNA before a) and generation of the target nucleic acid attached.

In certain embodiments, a reverse transcription primer is used in the reverse transcription, and the reverse transcription primer comprises the oligonucleotide adapter sequence and a polyT sequence in a 5' to 3' direction.

In certain embodiments, the reverse transcription includes hybridizing the polyT sequence with the RNA and extending the polyT sequence in a template-oriented manner.

In certain embodiments, the target nucleic acid includes DNA derived from the single cell.

In certain embodiments, the DNA includes genomic DNA, accessible chromatin DNA, protein-bound DNA regions and/or an exogenous nucleic acid that linked to proteins, lipids and/or small molecule compounds which can bind to target molecules in the cell.

In certain embodiments, it includes fragmenting the DNA derived from the single cell before a).

In certain embodiments, the target nucleic acid attached is generated after or during the fragmentation.

In certain embodiments, the fragmentation includes breaking with ultrasound, and then adding a sequence comprising the oligonucleotide adapter to the broken DNA, thereby obtaining the target nucleic acid attached.

In certain embodiments, the fragmentation includes interrupting with DNA endonuclease and DNA exonuclease, and then adding a sequence comprising the oligonucleotide adapter to the broken DNA, thereby obtaining the target nucleic acid attached

In certain embodiments, the fragmentation includes integrating the sequence comprising the oligonucleotide adapter into the DNA with a transposase-nucleic acid complex, and releasing the transposase to obtain the target nucleic acid attached.

In certain embodiments, the transposase-nucleic acid complex comprises transposase and a transposon terminal nucleic acid molecule, wherein the transposon terminal nucleic acid molecule comprises the oligonucleotide adapter sequence.

In certain embodiments, the transposase includes Tn5.

In certain embodiments, the DNA includes a protein-bound DNA region, and the transposase-nucleic acid complex further comprises a moiety that directly or indirectly recognizes the protein.

In certain embodiments, the moiety that directly or indirectly recognizes the protein includes one or more of the following groups: an antibody that specifically binds to the protein and Protein A or Protein G.

In another aspect, the present application further provides a composition, which comprises a plurality of solid supports each attached with at least one oligonucleotide tag, wherein each of the oligonucleotide tags comprises a first chain and a second chain, where the first chain comprises a barcode sequence and a hybridization sequence located at the 3' end of the barcode sequence, the second chain comprises a first portion complementary to the hybridization sequence of the first chain and a second portion complementary to the sequence in the nucleic acid to be tested, and the first chain and the second chain form a partially double-stranded structure, or the second chain and the target nucleic acid attached form a partially double-stranded structure; and the barcode sequence of the oligonucleotide tag comprises a common barcode domain and a variable domain, where the common barcode domains are the same in the oligonucleotide tags attached to the same solid support, and the common barcode domains are different between two or more solid supports in the plurality of solid supports.

In another aspect, the present application further provides a kit for analyzing a target nucleic acid from a cell, which comprises the composition described in this application.

In certain embodiments, the kit includes a transposase.

In certain embodiments, the kit further comprises at least one of a nucleic acid amplification agent, a reverse transcription agent, a fixing agent, a permeabilization agent, a ligation agent and a lysis agent.

A method for amplifying a target nucleic acid from a cell, comprising:
a) providing a discrete partition comprising: i. a target nucleic acid derived from a single cell, wherein at least a part of the target nucleic acids is added with an oligonucleotide adapter sequence to become a target nucleic acid attached; and ii. a solid support with at least one oligonucleotide tag attached, wherein each of the oligonucleotide tags comprises a first chain and a second chain, where the first chain comprises a barcode sequence and a hybridization sequence located at the 3' end of the barcode sequence, the second chain comprises a first portion complementary to the hybridization sequence of the first chain and a second portion complementary to the oligonucleotide adapter sequence attached to the target nucleic acid, and the first chain and the second chain form a partially double-stranded structure, or the second chain and the target nucleic acid attached form a partially double-stranded structure;
b) in the discrete partition, the oligonucleotide tag is linked to the target nucleic acid attached, thereby producing a barcoded target nucleic acid; and
c) amplifying the barcoded target nucleic acid.

In certain embodiments, the oligonucleotide tag is releasably attached to the solid support.

In certain embodiments, it includes releasing the at least one oligonucleotide tag from the solid support, and linking the released oligonucleotide tag to the target nucleic acid attached in b), thereby producing a barcoded target nucleic acid.

In certain embodiments, the oligonucleotide tag is directly or indirectly attached to the solid support through the 5' end of its first chain.

In certain embodiments, a ligase is further comprised in the discrete partition, and the ligase links the oligonucleotide tag to the target nucleic acid attached.

In certain embodiments, the ligase includes T4 ligase.

In certain embodiments, in the barcoded target nucleic acid, the target nucleic acid sequence is located at the 3' end of the barcode sequence.

In certain embodiments, the solid support is a bead.

In certain embodiments, the discrete partition is a hole or droplet.

In certain embodiments, the barcode sequence comprises a cell barcode sequence, and each oligonucleotide tag attached to the same solid support comprises the same cell barcode sequence.

In certain embodiments, the cell barcode sequence comprises at least 2 cell barcode segments separated by a linker sequence.

In certain embodiments, a) includes co-distributing the target nucleic acid derived from the single cell and the solid support attached with at least one oligonucleotide tag into the discrete partition.

In certain embodiments, b) includes linking the hybridization sequence of the first chain in the oligonucleotide tag to the oligonucleotide adapter attached to the target nucleic acid, thereby producing the barcoded target nucleic acid.

In certain embodiments, b) includes hybridizing the second part of the second chain in the oligonucleotide tag with the oligonucleotide adapter attached to the target nucleic acid, and linking the hybridization sequence of the first chain in the oligonucleotide tag to the oligonucleotide adapter attached to the target nucleic acid, thereby producing the barcoded target nucleic acid.

In certain embodiments, the target nucleic acid attached comprises a unique molecular identification region.

In certain embodiments, the unique molecular identification region is located between the oligonucleotide adapter sequence and the target nucleic acid sequence.

In certain embodiments, the oligonucleotide tag further comprises an amplification primer recognition region.

In certain embodiments, the amplification primer recognition region is a general amplification primer recognition region.

In certain embodiments, it includes releasing the barcoded target nucleic acid from the discrete partition after b) and before c), and the amplification is performed after the barcoded target nucleic acid is released from the discrete partition.

In certain embodiments, an amplification primer is used in the amplification, and the amplification primer comprises a random guide sequence.

In certain embodiments, the random guide sequence is a random hexamer.

In certain embodiments, the amplification includes at least partially hybridizing the random guide sequence with the barcoded target nucleic acid and extending the random guide sequence in a template-oriented manner.

In another aspect, the present application further provides a method for sequencing a target nucleic acid from a cell, including:
a) providing a discrete partition comprising: i. a target nucleic acid derived from a single cell, wherein at least a part of the target nucleic acids is added with an oligonucleotide adapter sequence to become a target nucleic acid attached; and ii. a solid support with at least one oligonucleotide tag attached, wherein each of the oligonucleotide tags comprises a first chain and a second chain, where the first chain comprises a barcode sequence and a hybridization sequence located at the 3' end of the barcode sequence, the second chain comprises a first portion complementary to the hybridization sequence of the first chain and a second portion complementary to the oligonucleotide adapter sequence attached to the target nucleic acid, and the first chain and the second chain form a partially double-stranded structure, or the second chain and the target nucleic acid attached form a partially double-stranded structure;
b) in the discrete partition, the oligonucleotide tag is linked to the target nucleic acid attached, thereby producing a barcoded target nucleic acid; and
c) sequencing the barcoded target nucleic acid.

In certain embodiments, the oligonucleotide tag is releasably attached to the solid support.

In certain embodiments, it includes releasing the at least one oligonucleotide tag from the solid support, and linking the released oligonucleotide tag to the target nucleic acid attached in b), thereby producing a barcoded target nucleic acid.

In certain embodiments, the oligonucleotide tag is directly or indirectly attached to the solid support through the 5' end of its first chain.

In certain embodiments, a ligase is further comprised in the discrete partition, and the ligase links the oligonucleotide tag to the target nucleic acid attached.

In certain embodiments, the ligase includes T4 ligase or T7 ligase.

In certain embodiments, in the barcoded target nucleic acid, the target nucleic acid sequence is located at the 3' end of the barcode sequence.

In certain embodiments, the solid support is a bead.

In certain embodiments, the discrete partition is a hole or droplet.

In certain embodiments, the barcode sequence comprises a cell barcode sequence, and each oligonucleotide tag attached to the same solid support comprises the same cell barcode sequence.

In certain embodiments, the cell barcode sequence comprises at least 2 cell barcode segments separated by a linker sequence.

In certain embodiments, a) includes co-distributing the target nucleic acid derived from the single cell and the solid support attached with at least one oligonucleotide tag into the discrete partition.

In certain embodiments, b) includes linking the hybridization sequence of the first chain in the oligonucleotide tag to the oligonucleotide adapter attached to the target nucleic acid, thereby producing the barcoded target nucleic acid.

In certain embodiments, b) includes hybridizing the second part of the second chain in the oligonucleotide tag with the oligonucleotide adapter attached to the target nucleic acid, and linking the hybridization sequence of the first chain in the oligonucleotide tag to the oligonucleotide adapter attached to the target nucleic acid, thereby producing the barcoded target nucleic acid.

In certain embodiments, the target nucleic acid attached comprises a unique molecular identification region.

In certain embodiments, the unique molecular identification region is located between the oligonucleotide adapter sequence and the target nucleic acid sequence.

In certain embodiments, the oligonucleotide tag further comprises an amplification primer recognition region.

In certain embodiments, the amplification primer recognition region is a general amplification primer recognition region.

In certain embodiments, it further includes assembling continuous nucleic acid sequences of at least a part of the genome of the single cell from the sequences of the barcoded target nucleic acids.

In certain embodiments, the single cell is characterized based on the nucleic acid sequences of at least a part of the genome of the single cell.

In certain embodiments, each of the discrete partitions includes at most the target nucleic acids derived from the single cell.

In certain embodiments, it further includes identifying the single nucleic acid sequence in the barcoded target nucleic acid as deriving from a given nucleic acid in the target nucleic acid based at least in part on the presence of the unique molecular identification region.

In certain embodiments, it further includes determining the amount of a given nucleic acid in the target nucleic acid based on the presence of the unique molecular identification region. Persons skilled in the art can recognize other aspects and advantages of theis application from the following detailed description. The following detailed description only shows and indicates exemplary embodiments of this application. As those skilled in the art will appreciate, the disclosure of this application enables persons skilled in the art to modify the disclosed embodiments without departing from the spirit and scope of the invention involved by this application. Correspondingly, the drawings and the description in the specification of this application are only exemplary, and not restrictive.

### BRIEF DESCRIPTION OF THE DRAWING

The specific features of the invention involved by this application are as shown in the appended claims. By reference to the exemplary embodiments detailed below and the accompanying drawings, the characteristics and advantages of the invention involved in this application can be better understood. The drawings are briefly described as follows:
Fig. 1 shows a schematic diagram of generating nucleotide tags suitable for non-transcriptome analysis by the PCR method in this application.
Fig. 2 shows a schematic diagram of generating nucleotide tags suitable for non-transcriptome analysis by the T4 ligase method in this application.
Fig. 3 shows a schematic diagram of generating nucleotide tags suitable for transcriptome analysis by the PCR method in this application.
Fig. 4 shows a schematic diagram of generating nucleotide tags suitable for non-transcriptome analysis by the T4 ligase method in this application.
Fig. 5 shows the distribution diagram of the fragment lengths in the ATAC sequencing results of the human 293T cells mediated by the Tn5 transposition reaction in this application.
Figs. 6A and 6B show the diagrams of the signal-enriched transcription start sites (TSS) in the ATAC sequencing results of the human 293T cells mediated by the Tn5 transposition reaction in this application.
Fig. 7 shows the diagram of the ratios of different types of sequences in the ATAC sequencing results of the human 293T cells mediated by the Tn5 transposition reaction in this application.
Fig. 8 shows a schematic diagram of the microfluidic chip in this application.
Fig. 9 shows the stackplot of the ATAC sequencing results in this application drawn according to the number of reads in each barcode.
Fig. 10 shows the distribution diagram of the unique mapped reads of the single cells in the ATAC sequencing results in this application.
Fig. 11 shows the distribution diagram of the ATAC data of cells in gene regions in this application.
Fig. 12 shows the diagram of the analysis results of the ATAC signal correlation of single cells in this application.
Fig. 13 shows the distribution results of the fragments of the Cut tag library in this application.
Fig. 14 shows the diagram of distribution position results of the Cut tag fragments at transcription start sites in this application.
Fig. 15 shows the diagram of the distribution ratio results of the Cut tag fragments in the genome in this application.
Fig. 16 shows the distribution results of the Cut tag results of single cells in this application.
Fig. 17 shows the diagram of the results of clearly distinguishing single cells of mixed cells according to the single cell transcriptomes in this application.
Fig. 18 shows the distribution results of transcripts and gene numbers detected in each cell in this application.
Fig. 19 shows the diagram of the results of clearly distinguishing single cells of mixed cells according to the single cell genomes in this application.
Fig. 20 shows the diagram of the results showing that single cell sequencing has different coverage in each cell and each genome site in this application.
Fig. 21 shows the diagram of the results of clearly distinguishing single cells of mixed cells according to the single cell DNA modifications in this application.
Fig. 22 shows the distribution results of the methylation modifications detected in each cell in this application.
Fig. 23 shows the distribution results of the 5hmC modifications detected in each cell in this application.
Fig. 24 shows the diagram of the results of the possibility of well distinguishing single cells of mixed cells according to both the transcriptomes and ATAC in this application.
Fig. 25 shows the diagram of the results of the possibility of well distinguishing single cells of mixed cells according to both the transcriptomes and cut tags in this application.
Fig. 26 shows the diagram of the results showing that both the transcriptomes and methylation sets of the same cell can be well matched with the gene models and known methylation sites in this application.
Fig. 27 shows a schematic diagram of a spatial lattice chip in this application.
Fig. 28 shows the diagram of the results of the number of genes after superimposing of the HE staining of the slices on the spatial lattice chip in this application.

### DETAILED DESCRIPTION

Hereinafter the embodiments of this application are described by specific examples. Persons skilled in the art can readily understand other advantages and effects of the invention of this application from the disclosure of the specification.

### Definition of terms

In this application, the term "sequencing" generally refers to a technology of obtaining the sequence information of nucleic acid molecules. For example, analyzing the base sequence of a specific DNA fragment (for example, the arrangement of adenine (A), thymine (T), cytosine (C) and guanine (G), etc.); and sequencing methods can include Sanger's dideoxy chain Termination Method, pyrosequencing methods, as well as the "synthetic parallel sequencing" platform or "sequencing by ligation" platform used by Illumina, Life Technologies, Roche, etc., and the sequencer of MGI/Complete Genomics, which are the new generation of sequencing; generally, sequencing methods can also include nanopore sequencing methods, e.g., the method developed by the Oxford nanopore technology company, PacBio's third-generation sequencer, or the method based on electronic detection, e.g., the Ion Torrent technology provided by Life Technologies.

In this application, the term "characterization result" usually refers to information description of nucleic acids and other related molecules obtained by sequencing or other biological analysis methods such as genomics and/or proteomics. For example, it can include sequence information of whole genome sequencing, accessible chromatin sequence and distribution information, binding information of nucleic acid sequences and its binding factors, mutation information of pathogenic genes, single nucleotide polymorphism (SNP), nucleotide methylation, transcriptome information (e.g., temporal or spatial change of gene expression level), etc.

In this application, the term "Protein A" generally refers to a cell-derived protein that can bind to the conserved region of the heavy chain of antibodies from different species (i.e., the recognition protein of antibodies). For example, it can bind to Fc fragments in serum IgG molecules of human and a plurality of mammals, wherein the mammals can include pigs, dogs, rabbits, humans, monkeys, murine, mice and cattle; and the subclasses of IgG bound to Protein A can mainly include IgG1, IgG2 and IgG4; Besides binding to IgG, Protein A can also bind to IgM and IgA in serum. For example, Protein A can include Protein A from *Staphylococcus aureus* (SPA). SPA is the main ingredient of cell wall antigen, and almost more than 90% of *Staphylococcus aureus* strains contain this ingredient, but the contents in different strains vary greatly. Using the function of protein A that can bind to antibodies, the target protein can be located and/or analyzed by forming a target protein-antibody-Protein A complex.

In this application, the term "solid support" generally refers to any material that is suitable or can be modified to be suitable for attaching oligonucleotide tags, barcode sequences, primers, etc. described herein. For example, a solid support includes an array of holes or depressions in a surface, which can be manufactured using a plurality of techniques, e.g., photolithography methods, stamping techniques, molding techniques and micro-etching techniques; and the composition and geometry of the solid support can be changed according to its use. For example, the solid support can be a planar structure (e.g., a slide glass, a chip, a microchip and/or an array among others); for example, the solid support or its surface can also be non-planar, e.g., the inner or outer surface of a pipe or container; and for example, the solid support can also include microspheres or beads.

In this application, "beads" or "microspheres" or "particles" usually refers to small discrete particles. Suitable bead compositions include, but not limited to, plastics, ceramics, glass, polystyrene, methylstyrene, acrylic polymers, paramagnetic materials, thorium oxide sol, carbon graphite, titanium dioxide, latex or cross-linked dextran (such as agarose), cellulose, nylon, cross-linked micelles and Teflon, and any other material outlined herein for solid supports can all be used, and Microsphere DetectionGuide of Bangs Laboratories, Fishers Ind. can be referred to; and in certain embodiments, the microspheres can be magnetic microspheres or beads.

In this application, the term "unique molecular identification region" can also be called "molecular barcode", "molecular label", "unique identifier (UID)", "unique molecular identifier (UMI)", etc., which usually refers to a unique sequence code linked to each original nucleotide fragment of the same sample. It can usually be designed as a completely random nucleotide chain (e.g., NNNNNNN), a partially degenerate nucleotide chain (e.g., NNNRNYN) or a designated nucleotide chain (e.g., when the template molecule is limited); and when it is introduced into a nucleic acid molecule, for example during the synthesis of the cDNA of the first chain, the subsequent amplification bias can be corrected by directly counting the unique molecular identifier (UMI) sequenced after the amplification. The design, incorporation and application of UMI can be carried out according to methods known in the art, for example as publicly exemplified by WO2012/142213, Islam et al. Nat. Methods (2014) 11:163-166, and Kivioja, T. et al. Nat. Methods (2012) 9:72-74, which are incorporated herein by reference in its entirety.

In this application, the term "amplification primer recognition region" generally refers to a nucleotide sequence that can be complementarily hybridized with the primer sequence for amplifying the target nucleic acid. Binding the primer to the amplification primer recognition region can initiate nucleotide extension, linking and/or synthesis, for example, increase the number of the target nucleic acid copies under the action of the polymerase chain reaction (i.e., amplification). In some embodiments, the amplification of sequences such as oligonucleotide tags and molecular unique identifiers can also be included.

In this application, the term "discrete partition" generally refers to spatial units comprising target substances to be analyzed, which are independent of each other. For example, droplets or pores; for example, the droplets formed by co-distributing a sample of a target nucleic acid with a solid support attached with an oligonucleotide tag; and in some embodiments, the discrete partition can also comprise other substances distributed according to different requirements, e.g., dyes, emulsifiers, surfactants, stabilizers, polymers, aptamers, reducing agents, initiators, biotin labels, fluorophores, buffers, acidic solutions, alkaline solutions, light-sensitive enzymes, pH-sensitive enzymes, aqueous buffers, detergents, ionic detergents, nonionic detergents, etc.

In this application, the term "releasably attached" generally means that the linking mode between an oligonucleotide tag and a solid support is releasable, cleavable or reversible, or can be destroyed or eliminated. For example, the linking of an oligonucleotide tag to a solid support comprises unstable bonds, e.g., chemically, thermally or light-sensitive bonds, e.g., disulfide bonds, UV-sensitive bonds, etc., which can be broken by corresponding treatment to achieve releasable attachment; for example, the linking of an oligonucleotide tag to a solid support comprises a specific base that can be recognized by a nuclease, e.g., dU, the linking of which can be cleaved by the action of a UNG enzyme; for example, the linking of an oligonucleotide tag to a solid support comprises an endonuclease recognition sequence, the linking of which can be cleaved by the action of a nuclease; and for example, the solid support is degradable, and the oligonucleotide tag is released by degradation of the solid support when a degradation condition is applied, so as to achieve the releasable attachment.

In this application, the term "linker" usually refers to a nucleotide sequence that links various functional sequences together, and can also include a molecular sequence (a nucleic acid, a polypeptides or other chemical linking structures, etc.) that links a oligonucleotide tag to a solid support, wherein the functional sequences can include cell barcode segments, barcode sequences, amplification primer recognition regions, sequencing primer recognition regions, unique molecular identifiers, etc. In certain embodiments, the nucleotide can be a fixing nucleotide sequence. In certain embodiments, the linker can further comprise chemical modification.

In this application, the term "random guide sequence" generally refers to a random primer that can exhibit quadruple degeneracy at each position. The random guide sequence recognizes and binds to the corresponding region of a target nucleic acid (including the target nucleic acid sequence itself and other nucleotide sequences attached), so as to achieve the synthesis and/or amplification of nucleotide sequences.

In this application, the term "barcode sequence" usually refers to a nucleotide sequence that can identify a target nucleic acid or its derivative or modified form.

In this application, the term "cell barcode sequence" generally refers to a nucleotide sequence that can be used to recognize the source of a target nucleic acid sample. Wherein the source can be, for example, from the same cell or different cells. In case nucleic acid samples are derived from a plurality of sources, different cell barcode sequences can be used to label the nucleic acid from each source, so that the source of the sample can be recognized. Barcodes (commonly also referred to as indexes, tags, etc.) are well known to those skilled in the art. Any suitable barcode or barcode group can be used, e.g., the cell barcode sequence described in the publication of US2013/0274117.

In this application, the term "cell barcode segment" generally refers to barcode nucleotide units that make up a cell barcode sequence, and N of the cell barcode segments can be formed into a cell barcode segment by PCR or the action of DNA ligase. N can be greater than or equal to 1, so that the resultant cell barcode sequence is sufficient to recognize the cell source of each nucleic acid sample derived from a plurality of sources.

In this application, the term "oligonucleotide adapter" generally refers to a nucleotide sequence attached to a target nucleic acid and comprising a hybridization sequence that can be complementary to the oligonucleotide tag. The nucleotide sequence can be a partially double-stranded structure, for example, it can have a prominent sequence that hybridizes with the oligonucleotide tag; in certain embodiments, the oligonucleotide adapter can further comprise a transposase (e.g., Tn5 transposase) binding sequence; in certain embodiments, the oligonucleotide adapter can further comprise an amplification primer recognition sequence; and in certain embodiments, the oligonucleotide adapter can further comprise a reverse transcription primer sequence.

In this application, the term "barcoded target nucleic acid" generally refers to a target nucleic acid to which at least a cell barcode sequence is attached.

In this application, the term "common barcode domain" generally refers to a barcode sequence used to recognize the source of a target nucleic acid. The common barcode domains comprised in the oligonucleotide tags attached to the same solid support are the same, and the common barcode domains comprised in the oligonucleotide tags attached to different solid supports are different from each other. In certain embodiments, the oligonucleotide tags released from the same solid support are linked to the target nucleic acids from one cell, and the cell source can be recognized by the common barcode domains.

In this application, the term "variable domain" usually refers to a nucleotide sequence outside the common barcode domain, which is set according to different needs. For example, linker sequence, amplification primer recognition sequence, sequencing primer recognition sequence, etc.

In this application, the term "transposase-nucleic acid complex" generally refers to a complex formed by a transposase and a sequence comprising the oligonucleotide adapter. Transposase usually refers to an enzyme that can bind to the end of transposon and catalyze its movement to other parts of the genome through shearing, pasting or replicative transposition mechanism. Transposon usually refers to a nucleotide fragment that can jump freely in the genome. It was put forward by Barbara McClintock when she studied the genetic mechanism of maize in the late 1940s. After that, other research groups described the molecular basis of transposition. For example, McClintock found that chromosome fragments can change positions and jump from one chromosome to another. The relocation of these transposons can change the expression of other genes. For example, transposition can cause color change in maize, and in other organisms such as bacteria, it can cause antibiotic resistance in the process of human evolution. The transposase-nucleic acid complex can comprise a dimer formed by two transposases which are respectively bound to oligonucleotide adapters, wherein the two transposases can be the same transposase or different, and the oligonucleotide adapters which are respectively bound to them can be the same or different.

In this application, the term "Tn5" usually refers to Tn5 transposase, which is a member of the ribonuclease (RNase) superfamily. Tn5 can be found in *Shewanella* and *Escherichia coli.* Tn5 can include naturally occurring Tn5 transposase and its various active mutant forms; and like most other transposases, Tn5 comprises a DDE motif, which is the active site for transposon transfer catalysis. It is reported that a DDE motif can coordinate with divalent metal ions (e.g., magnesium and manganese) and play an important role in catalytic reactions. Transposase Tn5 may increase transposition activity through mutation in the DDE region, and catalyze transposon movement. For example, wherein glutamic acid at position 326 is converted into aspartic acid, while two aspartic acids at positions 97 and 188 are converted into glutamic acid (amino acid number based on the amino acid sequence of GenBank accession number YP_001446289), and so on.

In this application, the term "microfluidic device" generally refers to an equipment or system capable of achieving microfluidics. Wherein, microfluidic usually refers to a technology of accurately controlling and manipulating microscale fluids, especially submicron structures. "Micro" usually refers to tiny capacity or volume (e.g., nanoliter, picoliter, and femtoliter level). Microfluidic technologies have been widely used in many fields, for example, the biomedicine field, e.g., enzyme analysis (such as glucose and lactic acid analysis), DNA analysis (such as the polymerase chain reaction and high-throughput sequencing) and protein omics analysis in molecular biology methods. The main structure of a microfluidic device can include a simple reservoir connected to it, a fluid pipeline for delivering fluids from sources outside the device, manifolds, fluid flow units (e.g., actuators, pumps, compressors), etc., a fluid conduit for distributing and delivering micro-fluids to subsequent treatment operations, instruments or parts, and the like.

In this application, the term "hybridization", "hybridizable" or "complementary" usually means that under the conditions of suitable temperature and ionic strength of solution in vitro and/or in vivo, the nucleotide sequence comprised in nucleic acid (e.g., RNA, and DNA) can be allowed to be specifically non-covalently bound (i.e., form Watson-Crick base pairs and/or G/U base pairs) to another nucleic acid sequence. Watson-Crick base pairing includes: adenine/adenosine (A) paired with thymine (T), A paired with uracil/uridine (U), guanine/guanine (G) paired with cytosine/cytidine (C). In certain embodiments, the hybridization between two RNA molecules (e.g., dsRNA), or the hybridization between a DNA molecule and an RNA molecule (e.g., when a DNA target nucleic acid base is paired with a guide RNA, etc.) is included, and G can also be paired with a U base. Hybridization requires that two nucleic acids comprise complementary sequences, but the possible mismatch between bases cannot be eliminated. Conditions suitable for hybridization between two nucleic acids depend on the length and degree of complementarity of nucleic acids, which are well known in the art. The greater the degree of complementarity between two nucleotide sequences, the greater the melting temperature (Tm) of the hybrid of the nucleic acids with these complementary sequences.

In this application, the term "read length", namely reads, generally refers to the sequencing sequence obtained by a reaction in nucleotide sequencing. Reads can be a short sequencing fragment, which is the base sequence data obtained by a single sequencing by a sequencer. The length of reads can be different for different sequencing instruments.

### Detailed description of the invention

In one aspect, the present application provides a method for analyzing a target nucleic acid from a cell, including:
a) providing the discrete partition comprising the following:
   i. a target nucleic acid derived from a single cell, wherein at least a part of the target nucleic acids is added with oligonucleotide adapter sequences to become target nucleic acids attached; and
   ii. a solid support with at least one oligonucleotide tag attached, wherein each of the oligonucleotide tags comprises a first chain and a second chain, where the first chain comprises a barcode sequence and a hybridization sequence located at the 3' end of the barcode sequence, the second chain comprises a first portion complementary to the hybridization sequence of the first chain and a second portion complementary to the oligonucleotide adapter sequence attached to the target nucleic acid, and the first chain and the second chain form a partially double-stranded structure;
      or ii. a solid support with at least one oligonucleotide tag attached, wherein each of the oligonucleotide tags comprises a first chain and a second chain, where the first chain comprises a barcode sequence and a hybridization sequence located at the 3' end of the barcode sequence, the second chain comprises a first portion complementary to the hybridization sequence of the first chain and a second portion complementary to the oligonucleotide adapter sequence attached to the target nucleic acid, and the second chain and the target nucleic acid attached form a partially double-stranded structure; and
b) in the discrete partition, the oligonucleotide tag is linked to the target nucleic acid attached, thereby producing a barcoded target nucleic acid.

For example, it further includes:
c) obtaining the characterization result of the barcoded target nucleic acid; and
d) identifying the sequence of the target nucleic acid as deriving from the single cell based at least in part on the presence of the same cell barcode sequence in the characterization result obtained in c).

In another aspect, the present application further provides a method for amplifying a target nucleic acid from a cell, including:
a) providing a discrete partition comprising: i. a target nucleic acid derived from a single cell, wherein at least a part of the target nucleic acids is added with an oligonucleotide adapter sequence to become a target nucleic acid attached; and ii. a solid support with at least one oligonucleotide tag attached, wherein each of the oligonucleotide tags comprises a first chain and a second chain, where the first chain comprises a barcode sequence and a hybridization sequence located at the 3' end of the barcode sequence, the second chain comprises a first portion complementary to the hybridization sequence of the first chain and a second portion complementary to the oligonucleotide adapter sequence attached to the target nucleic acid, and the first chain and the second chain form a partially double-stranded structure; or the step of ii. can be a solid support with at least one oligonucleotide tag attached, wherein each of the oligonucleotide tags comprises a first chain and a second chain, where the first chain comprises a barcode sequence and a hybridization sequence located at the 3' end of the barcode sequence, the second chain comprises a first portion complementary to the hybridization sequence of the first chain and a second portion complementary to the oligonucleotide adapter sequence attached to the target nucleic acid, and the second chain and the target nucleic acid attached form a partially double-stranded structure;
b) in the discrete partition, the oligonucleotide tag is linked to the target nucleic acid attached, thereby producing a barcoded target nucleic acid; and
c) amplifying the barcoded target nucleic acid.

In another aspect, the present application further provides a method for sequencing a target nucleic acid from a cell, including:
a) providing a discrete partition comprising: i. a target nucleic acid derived from a single cell, wherein at least a part of the target nucleic acids is added with an oligonucleotide adapter sequence to become a target nucleic acid attached; and ii. a solid support with at least one oligonucleotide tag attached, wherein each of the oligonucleotide tags comprises a first chain and a second chain, where the first chain comprises a barcode sequence and a hybridization sequence located at the 3' end of the barcode sequence, the second chain comprises a first portion complementary to the hybridization sequence of the first chain and a second portion complementary to the oligonucleotide adapter sequence attached to the target nucleic acid, and the first chain and the second chain form a partially double-stranded structure; or the step of ii. can be a solid support with at least one oligonucleotide tag attached, wherein each of the oligonucleotide tags comprises a first chain and a second chain, where the first chain comprises a barcode sequence and a hybridization sequence located at the 3' end of the barcode sequence, the second chain comprises a first portion complementary to the hybridization sequence of the first chain and a second portion complementary to the oligonucleotide adapter sequence attached to the target nucleic acid, and the second chain and the target nucleic acid attached form a partially double-stranded structure;
b) in the discrete partition, the oligonucleotide tag is linked to the target nucleic acid attached, thereby producing a barcoded target nucleic acid; and
c) sequencing the barcoded target nucleic acid.

For example, the oligonucleotide tag in this application can comprise a first chain and a second chain, wherein the first chain and the second chain can be provided simultaneously or separately. In this application, when the first chain and the second chain are provided simultaneously, the first chain and the second chain can form a partially double-stranded structure; and when the first chain and the second chain are provided separately, the second chain can form a partially double-stranded structure with the target nucleic acid attached.

### Barcoded target nucleic acid

In this application, the barcoded target nucleic acid is generated by linking the oligonucleotide tag with the target nucleic acid attached. For example, the hybridization sequence of the first chain in the oligonucleotide tag is linked to the oligonucleotide adapter attached to the target nucleic acid, thereby producing the barcoded target nucleic acid. For example, the second part of the second chain in the oligonucleotide tag is hybridized with the oligonucleotide adapter attached to the target nucleic acid, and the hybridization sequence of the first chain in the oligonucleotide tag is linked to the oligonucleotide adapter attached to the target nucleic acid, thereby producing the barcoded target nucleic acid. With regards to the hybridization, conditions suitable for hybridization between two nucleic acids depend on the length and degree of complementarity of nucleic acids, which are well known in the art. The greater the degree of complementarity between two nucleotide sequences, the greater the melting temperature (Tm) of the hybrid of the nucleic acids with these complementary sequences.

For example, the length of the second portion of the second chain of the oligonucleotide tag is sufficient to form a double-stranded structure with its complementary sequence (the oligonucleotide adapter sequence attached to the target nucleic acid or a part thereof).

For example, the length of the second portion of the second chain can be 1 nucleotide or more, 2 nucleotides or more, 3 nucleotides or more, 5 nucleotides or more, 8 nucleotides or more, 10 nucleotides or more, 12 nucleotides or more, 15 nucleotides or more, 20 nucleotides or more, 22 nucleotides or more, 25 nucleotides or more, or 30 nucleotides or more.

For example, the possible mismatch between bases is not eliminated in the hybridization. For example, the sequence of the first portion of the second chain or the second portion of the second chain need not be 100% complementary to the sequence of its hybridization sequence. For example, it can be 60% or more, 65% or more, 70% or more, 75% or more, 80% or more, 85% or more, 90% or more, 95% or more, 98% or more, 99% or more, 99.5% or more complementary. The remaining non-complementary nucleotides can be clustered or scattered with complementary nucleotides, and do not need to be adjacent to each other or complementary nucleotides. For example, polynucleotides can be hybridized on one or more segments, so that intermediate or adjacent segments are not involved in the hybridization event (e.g., a hairpin structure, "bump", etc., are formed).

For example, the oligonucleotide tag is linked to the target nucleic acid attached by a ligation reaction. The linking can include joining two nucleic acid segments, e.g., the hybridization sequence of the first chain of the oligonucleotide tag and the oligonucleotide adapter attached to the target nucleic acid, together by catalyzing the formation of phosphodiester bonds. The ligation reaction can include DNA ligase, such as *E. coli* DNA ligase, T4 DNA ligase, T7 DNA ligase, mammalian ligase (e.g., DNA ligase I, DNA ligase III, DNA ligase IV), thermostable ligase, etc. T4 DNA ligase can link segments containing DNA, oligonucleotides, RNA and RNA-DNA hybrids. The ligation reaction may not include DNA ligase, but use a substitute such as topoisomerase. Employing high concentration of DNA ligase and comprising PEG can achieve rapid linking. In order to select the favorable temperature for the ligation reaction, the optimum temperature of DNA ligase (for example, it can be 37°C) and the melting temperature of the DNA to be linked can be considered. The target nucleic acid and the barcoded solid support are suspended in a suitable buffer to minimize the ionic effect that may affect the ligation.

For example, it includes releasing at least a part of the target nucleic acids from the single cell in the discrete partition to the outside of the cell, and linking the released target nucleic acids to the oligonucleotide tags in b), thereby producing the barcoded target nucleic acids. For example, the step of releasing at least a part of the target nucleic acid from the single cell in the discrete partition to the outside of the cell can include contacting the cell with a lytic agent to release the contents of the cell in the discrete partition. The lytic agent can include bioactive reagents, for example, lytic enzymes for lysing different cell types (e.g., gram positive or negative bacteria, plants, yeasts, mammals, etc.), e.g., lysozyme, colorless peptidase, lysostaphin, kitalase, lyticase, and other commercially available lytic enzymes. For example, a surfactant-based lytic solution can also be used to lyse cells. For example, the lytic solution can include nonionic surfactants such as TritonX-100 and Tween 20. For example, the lytic solution can include ionic surfactants, such as sodium dodecyl sarcosinate and sodium dodecyl sulfate (SDS). For example, other lytic methods available (such as electroporation, heat, sound or mechanical cell destruction) can also be used.

For example, the step of releasing at least a part of the target nucleic acid from the single cell in the discrete partition to the outside of the cell can include releasing at least 5%, at least 10%, at least 15%, at least 20%, at least 25%, at least 30%, at least 35%, at least 40% and at least 45% of the target nucleic acids from the single cell in the discrete partition to the outside of the cell.

For example, it includes introducing at least a part of the oligonucleotide tags released from the solid support into the single cell, and linking them to the target nucleic acids in b), thereby producing barcoded target nucleic acids.

For example, the step of introducing at least a part of the oligonucleotide tags released from the solid support into the single cell can include introducing at least 25%, at least 30%, at least 35%, at least 40%, at least 50%, at least 55%, at least 60%, at least 70%, at least 75% and at least 75% of the oligonucleotide tags into the single cell.

For example, the oligonucleotide tag is releasably attached to the solid support. For example, the oligonucleotide tag releasably, cleavable or reversibly attached to the solid support includes the oligonucleotide tag that is released or releasable by cutting/breaking the linkage between the oligonucleotide tag molecule and the solid support, or the oligonucleotide tag that is released by degradation of the solid support itself, so that the oligonucleotide tag can be accessed or accessible by other reagents, or both.

For example, the acrydite moiety attached to the solid support precursor, another substance attached to the solid support precursor, or the precursor itself comprises unstable bonds, for example, chemically, thermally or light-sensitive bonds, e.g., disulfide bonds, UV-sensitive bonds, etc. The unstable bond can reversibly link (covalently link) a substance (e.g., an oligonucleotide tag) to a solid support. For example, thermally unstable bonds can include nucleic acid hybridization-based attachment (e.g., when an oligonucleotide is hybridized with a complementary sequence attached to a solid support), so that the pyrolysis chain of the hybrid releases the oligonucleotide from the solid support (or a bead), for example, a sequence containing an oligonucleotide tag. In addition, adding various types of unstable bonds to a gel solid support can lead to the generation of solid supports that can respond to different stimuli. Each type of unstable bonds can be sensitive to related stimuli (e.g., chemical stimuli, light, temperature, etc.), so that the release of a substance attached to a solid support through each type of unstable bonds can be controlled by applying suitable stimuli. For example, by an activating functional group of a gel bead, another substance comprising an unstable bond can be linked to the gel solid support after the gel solid support is formed. A reagent (with associated activatable groups) that is releasably attached to a solid support or otherwise arranged in a discrete partition can be provided, so that once delivered to a desired set of reagents (e.g., by co-distribution), the activatable groups can react with the desired reagent. Such activatable groups include caging groups, removable blocking or protecting groups, e.g., light unstable groups, thermally unstable groups, or chemically removable groups. In addition to thermally cleavable bonds, disulfide bonds and UV-sensitive bonds, other non-limiting examples of unstable bonds that can be coupled with precursors or solid supports include ester linkages (e.g., cleavable with acid, alkali or hydroxylamine), o-diol linkages (e.g., cleavable with sodium periodate), Diels-Alder linkages (e.g., thermally cleavable), sulfone linkages (e.g., cleavable by alkali), silyl ether linkages (e.g., cleavable by acid), glycoside linkages (e.g., cleavable by amylase), peptide linkages (e.g., cleavable by protease) or phosphodiester linkages (e.g., cleavable by nuclease (DNase)).

For example, the oligonucleotide tag is directly or indirectly attached to the solid support through the 5' end of its first chain. For example, it includes releasing the at least one oligonucleotide tag from the solid support, and linking the released oligonucleotide tag to the target nucleic acid attached in b), thereby producing a barcoded target nucleic acid.

For example, in the barcoded target nucleic acid, the target nucleic acid sequence is located at the 3' end of the barcode sequence. For example, the target nucleic acid can be directly linked to the 3' end of the barcode sequence; and for example, the target nucleic acid is not directly linked to the 3' end of the barcode sequence, and any other nucleotide sequence can exist between the target nucleic acid and the barcode sequence.

For example, the barcoded target nucleic acid is amplified after b) and before c). For example, the barcoded target nucleic acid is released from the discrete partition after b) and before c), and the amplification is performed after the barcoded target nucleic acid is released from the discrete partition. For example, after the barcoded target nucleic acid is released from the discrete partition, further chemical or enzymatic modification, for example, which can include bisulfite conversion, 5hmc conversion, etc., can be performed, and then amplification can be performed.

For example, amplification primers are used in the amplification. For example, the amplification can also include further modification of the barcoded target nucleic acid, so that it also has a fixing sequence on the other side that can be used for PCR amplification. For example, the modification can include reverse transcription chain conversion, and second chain synthesis, and can be a terminal transferase reaction, as well as linking to a second adaptor.

For example, the amplification primer can also comprise universal primers.

For example, an amplification primer is used in the amplification, and the amplification primer can comprise a random guide sequence. The random guide sequence includes a random primer that can exhibit quadruple degeneracy at each position. For example, a random primer includes any nucleic acid primer known in the art with various random sequence lengths. For example, a random primer can include random sequences of 3, 4, 5, 6, 7, 8, 10, 11, 12, 13, 14, 15, 16, 17, 18, 19, 20 or more nucleotides in length. For example, the plurality of random primers can include random primers with different lengths. For example, the plurality of random primers can include random primers with equal lengths. For example, the plurality of random primers can include random sequences of about 5 to about 18 nucleotides in length. For example, the plurality of random primers include random hexamers. The random hexamer is commercially available and widely used in amplification reactions, for example, the multiple displacement amplification (MDA), e.g., the REPLI-g whole genome amplification kit (QIAGEN, Valencia, CA). Random primers of any suitable length can be used in the methods and compositions described herein.

For example, the amplification includes at least partially hybridizing the random guide sequence with the barcoded target nucleic acid and extending the random guide sequence in a template-oriented manner.

### Oligonucleotide tag

In the present application, the oligonucleotide tag comprises a first chain and a second chain, where the first chain comprises a barcode sequence and a hybridization sequence located at the 3' end of the barcode sequence, the second chain comprises a first portion complementary to the hybridization sequence of the first chain and a second portion complementary to the oligonucleotide adapter sequence attached to the target nucleic acid, and the first chain and the second chain form a partially double-stranded structure. Or, in the present application, the oligonucleotide tag comprises a first chain and a second chain, where the first chain comprises a barcode sequence and a hybridization sequence located at the 3' end of the barcode sequence, the second chain comprises a first portion complementary to the hybridization sequence of the first chain and a second portion complementary to the oligonucleotide adapter sequence attached to the target nucleic acid, and the second chain and the target nucleic acid attached form a partially double-stranded structure.

With regards to the hybridization sequence, conditions suitable for hybridization between two nucleic acids depend on the length and degree of complementarity of nucleic acids, which are well known in the art. The greater the degree of complementarity between two nucleotide sequences, the greater the melting temperature (Tm) of the hybrid of the nucleic acids with these complementary sequences.

For example, the length of the first portion of the second chain or the second portion of the second chain is sufficient to form a double-stranded structure with its complementary sequence (for example, the hybridization sequence located at the 3' end of the barcode sequence in the first chain, e.g., the oligonucleotide adapter sequence attached to the target nucleic acid or a part thereof).

For example, the length of the first portion of the second chain or the second portion of the second chain can be 1 nucleotide or more, 2 nucleotides or more, 3 nucleotides or more, 5 nucleotides or more, 8 nucleotides or more, 10 nucleotides or more, 12 nucleotides or more, 15 nucleotides or more, 20 nucleotides or more, 22 nucleotides or more, 25 nucleotides or more, or 30 nucleotides or more.

For example, the lengths of the sequences of the first portion of the second chain and the second portion of the second chain can be the same or different.

For example, the possible mismatch between bases is not eliminated in the double-stranded structure. For example, the sequence of the first portion of the second chain or the second portion of the second chain need not be 100% complementary to the sequence of its hybridization sequence. For example, it can be 60% or more, 65% or more, 70% or more, 75% or more, 80% or more, 85% or more, 90% or more, 95% or more, 98% or more, 99% or more, 99.5% or more complementary. The remaining non-complementary nucleotides can be clustered or scattered with complementary nucleotides, and do not need to be adjacent to each other or complementary nucleotides. For example, polynucleotides can be hybridized on one or more segments, so that intermediate or adjacent segments are not involved in the hybridization event (e.g., a hairpin structure, "bump", etc., are formed).

For example, the second portions of the oligonucleotide tags attached to the same solid support can be the same.

For example, the second portions of the oligonucleotide tags attached to the same solid support can be different. For example, the second portion of each oligonucleotide tag attached to the same solid support can include 1 or more nucleotide sequences, for example, the sequence of the second portion can be 2 or more, for example, 3 or more, for example, 4 or more, for example, 5 or more, for example, 6 or more, for example, 7 or more, for example, 8 or more, for example, 9 or more, for example, 10 or more, for example 11 or more, for example, 12 or more, for example, 13 or more, for example, 14 or more, and for example, 15 or more nucleotide sequences, so that the oligonucleotide tags attached to the same solid support can be linked to the corresponding 1 or more of the target nucleic acids attached.

For example, the number of the oligonucleotide tags attached to the same solid support and containing the same second portion can be 1 or more, for example, 50 or more, 100 or more, 500 or more, 1000 or more, 1500 or more, 2000 or more, 3000 or more, 5000 or more, 8000 or more, 10000 or more, 12000 or more, 15000 or more, 18000 or more, 20000 or more, 22000 or more, 25000 or more, 28000 or more, 30000 or more, 35000 or more, 40000 or more, 45000 or more, and 50000 or more.

For example, the number of the oligonucleotide tags containing different second portions attached to the same solid support can be set to different proportions as required, so as to be linked to the corresponding target nucleic acids attached.

For example, the barcode sequence comprises a cell barcode sequence, and each oligonucleotide tag attached to the same solid support comprises the same cell barcode sequence.

For example, the oligonucleotide tags attached to the same solid support can include 1 or more oligonucleotide tags, for example, 50 or more, 100 or more, 500 or more, 1000 or more, 1500 or more, 2000 or more, 3000 or more, 5000 or more, 8000 or more, 10000 or more, 12000 or more, 15000 or more, 18000 or more, 20000 or more, 22000 or more, 25000 or more, 28000 or more, 30000 or more, 35000 or more, 40000 or more, 45000 or more, 50000 or more, 55000 or more, 60000 or more, 65000 or more, 70000 or more, 75000 or more, 80000 or more, 85000 or more, 90000 or more, 95000 or more, 100000 or more, 110000 or more, and 120000 or more. The cell barcode sequences of these oligonucleotide tags are the same, and the sequences of the second portions of the second chains thereof can be 1 or more, for example, the sequences of the second portions are 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, and 20 or more.

For example, the cell barcode sequences comprised in an oligonucleotide tag group attached to different solid supports are different from each other, where the oligonucleotide tag group can be a combination of all oligonucleotide tags attached to the same solid support.

For example, the cell barcode sequence comprises at least 2 cell barcode segments. For example, the cell barcode segment is 4 or more nucleotides (nt), for example, 5 or more, for example, 10 or more, 12 or more, 15 or more, 18 or more, 20 or more, 21 or more, 22 or more, 23 or more, 24 or more, 25 or more, 26 or more, 27 or more, 28 or more, 29 or more, 30 or more, 31 or more, 32 or more, 33 or more, 34 or more, or 35 or more.

For example, the cell barcode sequence comprises at least 2 cell barcode segments, at least 3 cell barcode segments, at least 4 cell barcode segments, at least 5 cell barcode segments, at least 6 cell barcode segments, at least 7 cell barcode segments, and at least 8 cell barcode segments. The cell barcode segments are coded as cell barcode segment 1, cell barcode segment 2, cell barcode segment 3, cell barcode segment 4, cell barcode segment 5 ... and cell barcode segment n according to the sequence from 5' end to 3' end in the oligonucleotide tag. For example, the at least 2 cell barcode segments can form the cell barcode sequence by PCR or DNA ligase.

For example, the cell barcode sequence can be generated by the following method:
1) dividing the at least 1 of the solid supports into at least 2 primary aliquots, for example, at least 8 aliquots, at least 16 aliquots, at least 24 aliquots, at least 32 aliquots, at least 40 aliquots, at least 48 aliquots, at least 56 aliquots, at least 64 aliquots, at least 72 aliquots, at least 80 aliquots, at least 88 aliquots and at least 96 aliquots;
2) providing to each of the primary aliquots at least 1 cell barcode section 1, for example, at least 1000 cell barcode sections 1, for example, at least 10000 cell barcode sections 1, for example, at least 100000 cell barcode sections 1, for example, at least 1000000 cell barcode sections 1, and for example, at least 10000000 cell barcode sections 1, wherein the sequences and/or lengths of the cell barcode sections 1 in each aliquot are different from that in any other aliquot;
3) linking the at least 1 solid support in each of the primary aliquots is directly or indirectly to the cell barcode sections 1, wherein each solid support is linked to at least one cell barcode section 1;
4) combining the at least 2 primary aliquots, and dividing the combined primary aliquots into at least 2 secondary aliquots, for example, at least 8 aliquots, at least 16 aliquots, at least 24 aliquots, at least 32 aliquots, at least 40 aliquots, at least 48 aliquots, at least 56 aliquots, at least 64 aliquots, at least 72 aliquots, at least 80 aliquots, at least 88 aliquots and at least 96 aliquots;
5) providing to each of the secondary aliquots at least 1 cell barcode section 2 or the complementary sequence thereof, for example, at least 1000 cell barcode sections 2 or the complementary sequences thereof, for example, at least 10000 cell barcode sections 2 or the complementary sequences thereof, for example, at least 100000 cell barcode sections 2 or the complementary sequences thereof, for example, at least 1000000 cell barcode sections 2 or the complementary sequences thereof, and for example, at least 10000000 cell barcode sections 2 or the complementary sequences thereof, wherein the sequences and/or lengths of the cell barcode sections 2 or the complementary sequences thereof in each aliquot are different from that in any other aliquot;
6) linking the at least 1 cell barcode section 1 linked to the solid support to the cell barcode sections 2 in each of the secondary aliquots directly or indirectly.

For example, steps 4)-6) can be repeated for n times, where n can be 1, 2, 3, 4, 5, 6, 7, 8, 9, 10 or more, to link cell barcode sections 3, cell barcode sections 4, cell barcode sections 5..., and cell barcode sections n, so that the target nucleic acid in the first cell can have a first cell barcode with a unique sequence, the target nucleic acid in the second cell can have a second cell barcode with a unique sequence, and so on.

For example, the barcoded target nucleic acid is released from the discrete partition after b) and before c).

For example, c) is further carried out: sequencing the barcoded target nucleic acid, thereby obtaining the characterization result.

For example, the characterization result can include the nucleotide sequence information of the barcode target nucleic acid, for example, the nucleotide sequence information of the cell barcode, the nucleotide sequence information of the target nucleic acid, and UMI sequence information.

For example, continuous nucleic acid sequences of at least a part of the genome of the single cell are assembled from the sequences of the barcoded target nucleic acids.

For example, the single cell is characterized based on the nucleic acid sequences of at least a part of the genome of the single cell.

For example, the oligonucleotide tag further comprises a linker sequence 1, by which the 5' end of the cell barcode segment 1 can be linked to the solid support. The linker sequence 1 can comprise sequences such as the acrydite modification, photo-cleavage modification, S-S modification, and dU base modification, and can be broken by various methods to release the oligonucleotide tag.

For example, the oligonucleotide tag further comprises other functional sequences, which can be located between the cell barcode segment 1 and the linker sequence 1, for example, full or partial functional sequences (e.g., primer sequence (for example, universal primer sequence, targeted primer sequence, and random primer sequence) recognition regions, primer annealing sequences, attachment sequences, sequencing primer recognition regions, amplification primer recognition regions (for example, universal amplification primer recognition regions), and the like), for subsequent treatment.

For example, the subsequent treatment includes amplification. For example, the amplification can include PCR amplification (for example, Taq DNA polymerase amplification, Super Taq DNA polymerase amplification, LA Taq DNA polymerase amplification, Pfu DNA polymerase amplification, Phusion DNA polymerase amplification, KOD DNA polymerase amplification, etc.), isothermal amplification (for example, it can include loop-mediated isothermal amplification (LAMP), helicase-dependent amplification (HDA), recombinase polymerase amplification (RPA), strand displacement amplification (SDA), nucleic acid sequence-based amplification (NASBA), transcription-mediated amplification (TMA),etc.), T7 promoter linear amplification, degenerate oligonucleotide primer PCR amplification (DOP-PCR), multiple displacement amplification (MDA), multiple annealing and looping based amplification cycles (MALBAC), etc.

For example, the cell barcode may also not comprise a linker, and the cell barcode can be a single nucleic acid sequence synthesized by other methods.

For example, the universal primer sequence can include P5 or other suitable primers. The universal primer (e.g., P5) can also be compatible with the sequencing device, for example, it can be attached to the flow cell in the sequencing device. For example, such universal primer sequences can provide complementary sequences of oligonucleotides constrained on the surface of a flow cell in a sequencing device, so that the barcoded target nucleic acid sequence can be fixed on the surface for sequencing.

For example, amplification primer sequences, which are primer sequences for amplification or replication processes (e.g., extending primers along the target nucleic acid sequence), so as to produce amplified barcoded target nucleic acid sequences.

For example, sequencing primer sequences; the resulting amplification target sequences will comprise such primers and be easily transferred to the sequencing system. For example, when the amplified target is sequenced by the Illumina sequencing system, the sequencing primer sequence can comprise the R1 primer sequence and the R2 primer sequence.

For example, the oligonucleotide tag can comprise the T7 promoter sequence. For example, the T7 promoter sequence comprises a nucleotide sequence as shown in SEQ ID NO: 1 (TAATACGACTCACTATAG).

For example, the oligonucleotide tag can comprise a region with at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity with any one of SEQ ID NO: 6-9.

For example, the nucleotide adapter sequence can comprise the P5 sequence. For example, the nucleotide adapter sequence comprises the P7 sequence.

For example, any of the above functional sequences or combination thereof can be comprised between the cell barcode segment 1 and the linker sequence 1. For example, these oligonucleotides can include any one or more of the following: the P5, R1 and R2 sequences, non-cleavable 5' acrydite-P5, cleavable 5' acrydite-SS-P5, R1c, a sequencing primer, a reading primer, a universal primer, P5_U, a universal reading primer and/or binding sites of any of these primers.

For example, the cell barcode sequence comprises at least 2 cell barcode segments separated by a linker sequence.

For example, the linker sequence 2 is at the 3' end of the cell barcode segment 1, the linker sequences 3 and 4 are each at the 5' end and 3' end of the cell barcode segment 2, the linker sequences 5 and 6 are each at the 5' end and 3' end of the cell barcode segment 3, the linker sequences 7 and 8 are each at the 5' end and 3' end of the cell barcode segment 4, and so on, and the linker sequences 2n-1 and 2n are each at the 5' end and 3' end of the cell barcode segment n; and the linker sequence 2 is at least partially complementarily paired with the linker sequence 3 to form a double-stranded structure, the linker sequence 4 is at least partially complementarily paired with the linker sequence 5 to form a double-stranded structure, and the linker sequence 6 is at least partially complementarily paired with the linker sequence 7 to form a double-stranded structure, and so on, so as to start the linking of the cell barcode section 1, the cell barcode section 2, the cell barcode section 3, the cell barcode section 4 ..., and the cell barcode section n.

For example, a ligation reaction is used to link each of the cell barcode segments to form an oligonucleotide tag. The linking can include joining two nucleic acid segments together by catalyzing the formation of phosphodiester bonds, for example, the cell barcode segment 1 and the aforementioned functional sequence, for example, the linker sequence 2 and the cell barcode segment 2, the linker sequence 3 and the cell barcode segment 3, the linker sequence 4 and the cell barcode segment 4, the linker sequence 5 and the cell barcode segment 5, the linker sequence 6 and the cell barcode segment 6, and so on. The ligation reaction can include DNA ligase, such as *E. coli* DNA ligase, T4 DNA ligase, mammalian ligase (e.g., DNA ligase I, DNA ligase III, DNA ligase IV), thermostable ligase, etc. T4 DNA ligase can link segments containing DNA, oligonucleotides, RNA and RNA-DNA hybrids. The ligation reaction may not include DNA ligase, but use a substitute such as topoisomerase. Employing high concentration of DNA ligase and comprising PEG can achieve rapid linking. In order to select the favorable temperature for the ligation reaction, the optimum temperature of DNA ligase (for example, it can be 37°C) and the melting temperature of the DNA to be linked can be considered. The sample and the barcoded solid support are suspended in a buffer to minimize the ionic effect that may affect the ligation.

For example, under the condition at which the ligase generates an oligonucleotide tag, the cell bar code segment provided in each round can comprise the following structure: the cell barcode segment and the linker sequence located at the 3' end of the cell barcode segment which is a double-stranded structure, and the linker sequence located at the 5' end of the cell barcode segment which is a prominent single-stranded structure, and which is at least partially complementarily paired with the linker sequence at the 5' end of the cell barcode segment in the previous round to form a double-stranded structure.

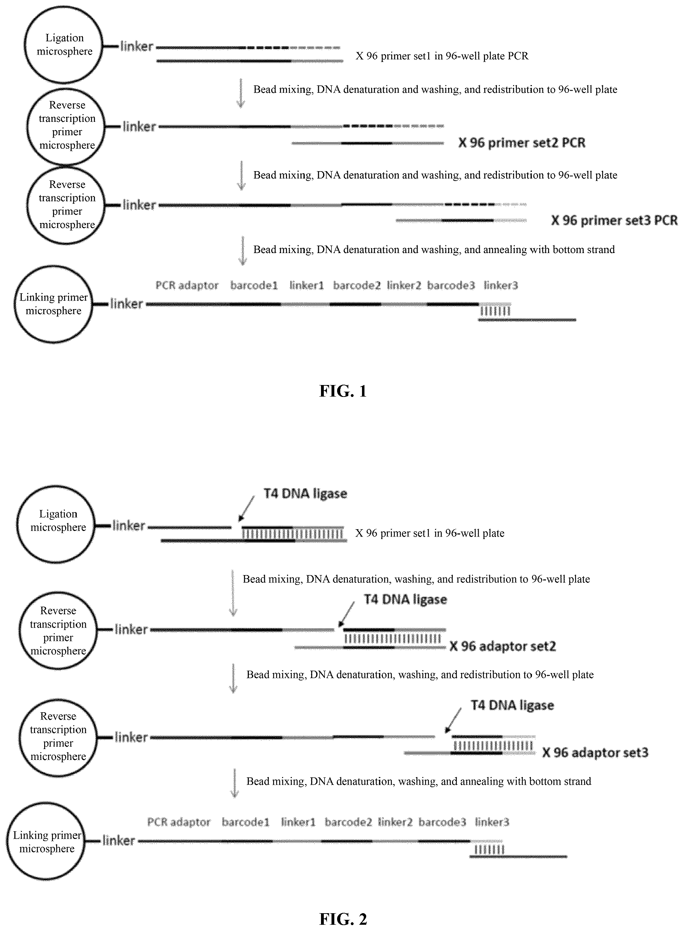

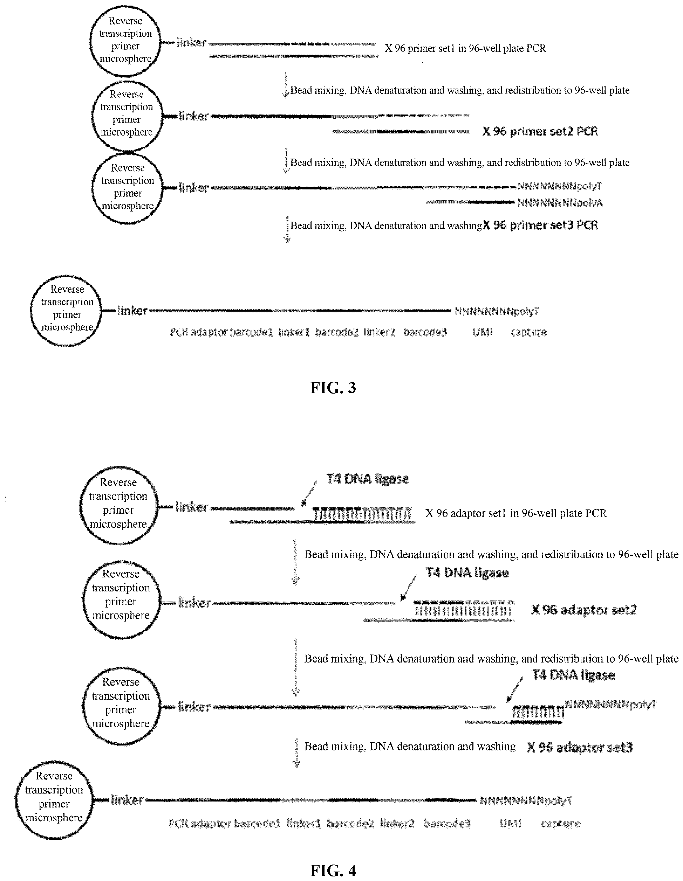

For example, an example of using a ligation reaction to link each of the cell barcode segments to form an oligonucleotide tag can be shown in Fig. 2 or Fig. 4.

For example, each cell barcode segment is linked by the polymerase chain reaction (PCR) to form an oligonucleotide tag. For example, the polymerase chain reaction can be carried out by any one or more of the following polymerases: Taq DNA polymerase, Super Taq DNA polymerase, LA Taq DNA polymerase, UlltraPF DNA polymerase, Tth DNA polymerase, Pfu DNA polymerase, VentR DNA polymerase, Phusion DNA polymerase, KOD DNA polymerase and Iproof DNA polymerase. For example, buffers and metal ions can be further included in the polymerase chain reaction to keep the above-mentioned polymerases active; and for example, dNTPs and/or the modified derivatives thereof can be further included in the polymerase chain reaction.

For example, under the condition at which the polymerase chain reaction (PCR) generates an oligonucleotide tag, the complementary sequence of the cell barcode segment is provided in each round, which is a single-stranded structure, and the 5' end and 3' end each has a linker sequence with a single-stranded structure, wherein the linker sequence at the 5' end can be at least partially complementarily paired with the linker sequence at the 3' end of the cell barcode segment linked in the previous round to form a double-stranded structure, and the linker sequence at the 3' end can be at least partially complementarily paired with the linker sequence at the 5' end of the cell barcode segment linked in the next round to form a double-stranded structure.

For example, an example of using a polymerase chain reaction (PCR) to link each of the cell barcode segments to form an oligonucleotide tag can be shown in Fig. 1 or Fig. 3.

### Target nucleic acid attached

In the present application, the target nucleic acid includes one or more selected from the group consisting of DNA, RNA and cDNA. For example, the target nucleic acid includes cDNA derived from RNA in the single cell. For example, the RNA includes mRNA.

In the present application, the target nucleic acid is added with an oligonucleotide adapter sequence to become a target nucleic acid attached. For example, the oligonucleotide adapter sequence is located at the 5' end of the target nucleic acid.

For example, the oligonucleotide adapter sequence can comprise a nucleotide sequence L complementary to the second portion of the second chain in the oligonucleotide tag. The length of the nucleotide sequence L can be the same as or different from the length of the second portion of the second chain in the oligonucleotide tag; and for example, the length of the nucleotide sequence L can be 1 nucleotide or more, 2 nucleotides or more, 3 nucleotides or more, 5 nucleotides or more, 8 nucleotides or more, 10 nucleotides or more, 12 nucleotides or more, 15 nucleotides or more, 20 nucleotides or more, 22 nucleotides or more, 25 nucleotides or more, or 30 nucleotides or more.

For example, the nucleotide sequence L can be complementarily paired with the second portion of the second chain in the oligonucleotide tag to form a double-stranded structure. For example, the possible mismatch between bases may not be eliminated in the double-stranded structure. For example, the sequence of the nucleotide sequence L need not be 100% complementary to the sequence of the second portion of the second chain in the oligonucleotide tag. For example, it can be 60% or more, 65% or more, 70% or more, 75% or more, 80% or more, 85% or more, 90% or more, 95% or more, 98% or more, 99% or more, 99.5% or more complementary. The remaining non-complementary nucleotides can be clustered or scattered with complementary nucleotides, and do not need to be adjacent to each other or complementary nucleotides. For example, polynucleotides can be hybridized on one or more segments, so that intermediate or adjacent segments are not involved in the hybridization event (e.g., a hairpin structure, "bump", etc., are formed).

For example, the nucleotide adapter sequence comprises a transposon terminal sequence. For example, the transposon terminal sequence is a Tn5 or a modified Tn5 transposon terminal sequence. For example, the transposon terminal sequence is a Mu transposon terminal sequence. For example, the Tn5 or the modified Tn5 transposon terminal sequence or the Mu transposon terminal sequence can comprise 15 to 25 nucleotides, for example, 16 nucleotides, 17 nucleotides, 18 nucleotides, 19 nucleotides, 20 nucleotides, 21 nucleotides, 22 nucleotides, 23 nucleotides, and 24 nucleotides.

For example, the Tn5 chimeric end sequence A14 (Tn5MEA) and/or the Tn5 chimeric end sequence B15 (Tn5MEB) (including complementary non-transferred sequences (NTS) described below) can be used as the transposon terminal sequences.
Tn5MEA: 5'-TCGTCGGCAGCGTCAGATGTGTATAAGAGACAG-3'; (SEQ ID NO: 2)
Tn5MEB: 5'-GTCTCGTGGGCTCGGAGATGTGTATAAGAGACAG-3'; (SEQ ID NO: 3)
Tn5NTS: 5'-CTGTCTCTTATACACATCT-3'. (SEQ ID NO: 4)

For example, before step a) in the method described in this application, the RNA is reverse transcribed, and the target nucleic acid attached is generated. For example, in the reverse transcription, the first chain of cDNA is synthesized from the mRNA in each mRNA sample using a first chain synthesis primer. For example, the first chain synthesis primer includes an oligomeric dT primer. For example, the first chain synthesis primer used in the reverse transcription can be a reverse transcription primer, and the reverse transcription primer comprises the oligonucleotide adapter sequence and a polyT sequence in a 5' to 3' direction. For example, the reverse transcription includes hybridizing the polyT sequence with the RNA and extending the polyT sequence in a template-oriented manner. For example, the first chain synthesis primer is a random primer. For example, the first chain synthesis primer is a mixture of the oligomeric dT primer and the random primer. For example, the method further includes incorporating a template conversion oligonucleotide primer (TSO primers) together with the mixture of the oligomeric dT primer and the random primer. For example, the second chain of the cDNA is synthesized by the TSO primer. For example, the second chain of the cDNA is synthesized by using a second amplification primer complementary to the first chain of the cDNA, wherein the first chain extends beyond the mRNA template to include the complementary TSO chain.

For example, the target nucleic acid includes DNA derived from the single cell. For example, the DNA includes genomic DNA,

For example, the DNA includes genomic DNA, accessible chromatin DNA, protein-bound DNA regions and/or an exogenous nucleic acid that linked to proteins, lipids and/or small molecule compounds which can bind to target molecules in the cell. For example, the protein can include antibodies and antigens. For example, the target molecule can include the target nucleic acid sequence to be analyzed in the cell. For example, before step a) in the method described in this application, the DNA derived from the single cell is fragmented. For example, for example, DNA fragmentation can include the separation or destruction of DNA chains into small pieces or segments. For example, various methods can be employed to perform DNA fragmentation, for example, further attaching the sequence of the oligonucleotide adapter after DNA fragmentation (the sequence of the oligonucleotide adapter attached under this condition does not comprise the transposon terminal sequence), including various methods of restriction digestion or shearing force generation. For example, restriction digestion can use restriction endonuclease to make cuts in DNA sequences by cutting the flat ends of two chains or by uneven cutting to produce cohesive ends. For example, DNA chain disruption mediated by a shearing force can include ultrasonic treatment, acoustic shearing, needle shearing, pipetting or atomization. Ultrasonic treatment is a type of hydrodynamic shearing, which exposes DNA sequences to a short-term shearing force, and can produce a fragment size of about 700 bp. Acoustic shearing applies high-frequency acoustic energy to the DNA sample in a bowl-shaped transducer. Needle shearing produces a shearing force by passing DNA through a needle with a small diameter to physically tear the DNA into smaller segments. Atomizing force can be generated by passing DNA through the pores of the sprayer unit, wherein the resulting DNA fragments are collected from the fine mist leaving the unit. Generally, these fragments can be any length between about 200 and about 100000 bases. For example, the fragment will be about 200 bp to about 500 bp, about 500 bp to about 1 kb, about 1 kb to about 10 kb, or about 5 kb to about 50 kb, or about 10 kb to about 30 kb, for example, about 15 kb to about 25 kb. For example, the fragmentation of larger genetic components can be carried out by any convenient method available, including, for example, commercially available shearing-based fragmentation systems (e.g., the Covaris fragmentation system), size-targeted fragmentation systems (e.g., Blue Pippin (Sage Sciences)), enzyme fragmentation methods (e.g., DNA endonuclease, and DNA exonuclease), etc. For example, the fragmentation includes breaking with ultrasound, and then adding a sequence comprising the oligonucleotide adapter to the broken DNA, thereby obtaining the target nucleic acid attached.

For example, the target nucleic acid attached is generated after or during the fragmentation. For example, the fragmentation includes integrating the sequence comprising the oligonucleotide adapter into the DNA with a transposase-nucleic acid complex, and releasing the transposase to obtain the target nucleic acid attached.

For example, the transposase includes *Staphylococcus aureus* Tn5 (Colegio et al., J. BacterioL, 183:2384-8, 200, 1; Kirby C et al., Mol. Microbiol., 43:173-86, 200, 2.), Tylosin (Tyl) (Devine and Boeke, Nucleic Acids Res., 22:3765-72, 199, 1994, and International Publication WO 95/23875), Transposon Tn7 (Craig, N L, Science. 271: 1512, 1996; Craig, N L, a review in Curr Top Microbiol Immunol., 204:27-48, 199, 1996), Tn/O and IS10 (KlecknerN et al., Curr Top Microbiol Immunol., 204:49-82, 199, 1996), Mariner transposase (Lampe D J et al., EMBO J., 15:5470-9, 1996), Tel (Plasterk R H, Curr. Topics Microbiol. Immunol., 204:125-43, 199, 1996), P factor (Gloor, G B, Methods Mol. Biol., 260: 97-114, 200, 4), Tn3 (Ichikawa and Ohtsubo, J Biol. Chem. 265:18829-32, 199, 0), a bacterial insertion sequence (Ohtsubo and Sekine, Curr. Top. Microbiol. Immunol. 204:1-26, 199, 1996), retrovirus (Brown et al., Proc Natl Acad Sci USA, 86:2525-9, 9) and yeast retrotransposon (Boeke and Corces, Annu Rev Microbiol., 43:403-34, 198, 9), as well as IS5, Tnl0, Tn903, IS911, and engineering forms of transposase family enzymes (Zhang et al., (2009) PLoS Genet. 5:el000689., published electronically on October 16th, 2009.; Wilson C. et al., (2007) J. Microbiol. Methods 71:332-5).

For example, the transposase-nucleic acid complex comprises transposase and a transposon terminal nucleic acid molecule, wherein the transposon terminal nucleic acid molecule comprises the oligonucleotide adapter sequence.

For example, the transposase is Mu transposase. For example, the transposase is Tn5 transposase or Tn10 transposase. The Tn5 transposase is selected from full-length Tn5 transposase, partial functional domains of Tn5 transposase, and Tn5 transposase mutation. The Tn10 transposase is selected from full-length Tn10 transposase, partial functional domains of Tn10 transposase and Tn10 transposase mutants. For example, the Tn5 transposase mutant can be selected from R30Q, K40Q, Y41H, T47P, E54K/V, M56A, R62Q, D97A, E110K, D188A, Y319A, R322A/K/Q, E326A, K330A/R, K333A, R342A, E344A, E345K, N348A, L372P, S438A, K439A, S445A, G462D, and A466D.

For example, the two transposase molecules can bind the same or different double-stranded DNA transposons, so that the insertion site is labeled by one or two kinds of DNA. For example, the two transposase molecules (e.g., Tn5 (comprising point mutation hyperactivity) or other types of transposases) can be assembled into a hybrid transposition complex with one of the oligonucleotide adapter sequences and another standard transposon DNA sequence, or only the above double-stranded structure 2 can be used to form a single Tn5 transposition complex. The standard transposon DNA sequence can comprise an amplification primer sequence and/or a sequencing primer sequence.

For example, the DNA can include a protein-bound DNA region, and the transposase-nucleic acid complex further comprises a moiety that directly or indirectly recognizes the protein. For example, the moiety that directly or indirectly recognizes the protein can include *Staphylococcus aureus* Protein A (ProteinA), *Streptococcus* Protein G (ProteinG), *Streptococcus* Protein L (ProteinL) or other protein analogues with antibody binding function. For example, the moiety that directly or indirectly recognizes the protein can further include an antibody that specifically binds to the protein. For example, each of the *Staphylococcus aureus* Protein A (ProteinA), *Streptococcus* Protein G (ProteinG), *Streptococcus* Protein L (ProteinL) or other protein analogues with antibody binding function can bind to the antibody that specifically binds to the protein.

For example, the transposase forms a fusion protein with the *Staphylococcus aureus* Protein A (ProteinA), *Streptococcus* Protein G (ProteinG), *Streptococcus* Protein L (ProteinL) or other protein analogues with antibody binding function.

For example, the fusion protein binds to the antibody that specifically binds to the protein to form a complex, and then targets the protein.

For example, the antibody that specifically binds to the protein binds to the protein, and then the fusion protein binds to the antibody to target the protein.

For example, the oligonucleotide adapter sequence can also comprise an antibody recognition sequence for recognizing/mapping tracking the different antibodies. The antibody recognition sequence can be generated in a manner similar to random primers.

For example, the target nucleic acid attached comprises a unique molecular identification region. The unique molecular identification region (UMI) refers to a unique nucleic acid sequence attached to each of a plurality of nucleic acid molecules. For example, when incorporated into nucleic acid molecules, UMI can be used to correct the subsequent amplification bias by directly counting the unique molecular identification region (UMI) sequenced after the amplification. For example, it includes identifying the single nucleic acid sequence in the barcoded target nucleic acid as deriving from a given nucleic acid in the target nucleic acid based at least in part on the presence of the unique molecular identification region. For example, it includes determining the amount of a given nucleic acid in the target nucleic acid based on the presence of the unique molecular identification region. The design, incorporation and application of UMI can be carried out according to methods known in the art, for example as publicly demonstrated by WO 2012/142213, Islam et al. Nat. Methods (2014) 11:163-166, and Kivioja, T. et al. Nat. Methods (2012) 9:72-74, each of which is incorporated herein by reference in its entirety. For example, the unique molecular identification region is located between the oligonucleotide adapter sequence and the target nucleic acid sequence.

For example, the target nucleic acid can further include an exogenous nucleic acid including that linked to proteins, lipids and/or small molecule compounds which can bind to target molecules in the cell. For example, the protein can include antibodies and antigens. For example, the target molecule can include the target nucleic acid sequence to be analyzed in the cell.

For example, the transposition reactions and methods described herein are carried out in batch, and then biological particles (e.g., nuclei/cells/chromatin from a single cell) are distributed, so that a plurality of discrete partitions are occupied by biological particles (e.g., cells, nuclei, chromatin or cell beads) alone. For example, a plurality of biological particles can be distributed into a plurality of discrete partitions, so that the discrete partition among the plurality of discrete partitions includes a single biological particle.

### Solid support

In the present application, the solid support can include a bead. For example, the bead can be porous, non-porous and/or combinations thereof. For example, the bead can be solid, semi-solid, semi-fluid, fluid and/or combinations thereof. For example, the bead can be soluble, destructible and/or degradable. For example, the bead can be non-degradable. For example, the bead can be a gel bead. The gel bead can be a hydrogel bead. The gel bead can be formed from molecular precursors, for example, polymers or monomer substances. The semi-solid bead can be a liposome bead. The solid bead can comprise metals, including iron oxide, gold and silver. For example, the bead can be a silica bead. For example, the bead is a magnetic bead. For example, the bead can be rigid. For example, the bead can be flexible and/or compressible.

For example, the bead can be of any suitable shape. For example, the shape of the bead can include, but not limited to, spherical, non-spherical, elliptical, oblong, amorphous, circular, cylindrical shapes and their variants.

For example, the bead can have a uniform size or non-uniform size. For example, the diameter of the bead can be at least about 10 nm, 100 nm, 500 nm, 1 µm, 5 µm, 10 µm, 20 µm, 30 µm, 40 µm, 50 µm, 60 µm, 70 µm, 80 µm, 90 µm, 100 µm, 250 µm, 500 µm, 1mm or more. For example, the diameter of the bead can be smaller than 10 nm, 100 nm, 500 nm, 1 µm, 5 µm, 10 µm, 20 µm, 30 µm, 40 µm, 50 µm, 60 µm, 70 µm, 80 µm, 90 µm, 100 µm, 250 µm, 500 µm, 1 mm or less. For example, the diameter of the bead can be in the range of about 40-75 µm, 30-75 µm, 20-75 µm, 40-85 µm, 40-95 µm, 20-100 µm, 10-100 µm, 1-100 µm, 20-250 µm or 20-500 µm.

For example, the bead can be provided in the form of a bead group or a plurality of beads with a relatively monodisperse size distribution. In the case that it is necessary to provide a relatively consistent number of reagents in a discrete partition, maintaining a relatively consistent bead characteristic (e.g., size) can contribute to the overall consistency. Particularly, the beads described herein can have a size distribution with a coefficient of variation of their cross-sectional dimensions of less than 50%, less than 40%, less than 30%, less than 20%, and for example, less than 15%, less than 10%, less than 5% or less.

For example, the bead can comprise natural and/or synthetic materials. For example, the bead can comprise natural polymers, synthetic polymers, or natural and synthetic polymers. Natural polymers can include proteins and carbohydrates, for example deoxyribonucleic acid, rubber, cellulose, starch (e.g., amylose and amylopectin), proteins, enzymes, polysaccharide, silk, polyhydroxyalkanoate, chitosan, dextran, collagen, carrageenan, egg-leaf plantain, acacia, agar, gelatin, shellac, buttonwood gum, xanthan gum, corn gum, guar gum, karaya gum, agarose, alginic acid, alginate or their natural polymers. Synthetic polymers can include acrylics, nylon, silicone, spandex, viscose rayon, polycarboxylic acid, polyvinyl acetate, polyacrylamide, polyacrylate, polyethylene glycol, polyurethane, polylactic acid, silicon dioxide, polystyrene, polyacrylonitrile, polybutadiene, polycarbonate, polyethylene, polyethylene terephthalate, polytrifluorochloroethylene, polyethylene oxide, polyethylene terephthalate, polyisobutylene, polymethyl methacrylate, polyformaldehyde, polypropylene, polystyrene, polytetrafluoroethylene, polyvinyl alcohol, polyvinyl chloride, polyvinylidene chloride, polyvinylidene fluoride, polyvinyl fluoride and/or combinations thereof (e.g., copolymers). The bead can also be formed of materials other than polymers, for example lipids, micelles, ceramics, glass ceramics, material composites, metals, other inorganic materials, etc.

For example, the bead can contain molecular precursors (e.g., monomers or polymers), which can form a polymer network by polymerization of the molecular precursors. For example, the precursor can be a substance that has already been polymerized, which can be further polymerized by, for example, chemical cross-linking. For example, the precursor can comprise one or more of acrylamide or methacrylamide monomers, oligomers or polymers. For example, the bead can comprise prepolymers, which are oligomera capable of further polymerization. For example, prepolymers can be used to prepare polyurethane beads. For example, the bead can contain individual polymer that can be further polymerized together. For example, the bead can be produced by polymerization of different precursors so that they contain mixed polymers, copolymers and/or block copolymers. For example, the bead can comprise covalent or ionic bonds between polymer precursors (e.g., monomers, oligomers, and linear polymers), nucleic acid molecules (e.g., oligonucleotides), primers, and other entities. For example, the covalent bond can be a carbon-carbon bond, a thioether bond or a carbon-heteroatom bond.

For example, the cross-linking can be permanent or reversible, depending on the specific cross-linking agent used. The reversible cross-linking can allow polymers to be linearized or dissociated under appropriate conditions. For example, the reversible cross-linking can also allow binding substances to reversibly attach to the surface of beads. For example, the cross-linking agent can form disulfide bonds. For example, the chemical cross-linking agent that forms disulfide bonds can be cystamine or modified cystamine.

For example, disulfide bonds can be formed between molecular precursor units (e.g., monomers, oligomers or linear polymers) incorporated into beads or between precursors and nucleic acid molecules (e.g., oligonucleotides). For example, cysteamine (including modified cysteamine) is an organic reagent comprising disulfide bonds, which can be used as a cross-linking agent between individual monomers or polymer precursors of beads. Polyacrylamide can be polymerized in the presence of cystamine or a substance comprising cystamine (e.g., modified cystamine) to produce polyacrylamide gel beads comprising disulfide bonds (e.g., chemically degradable beads comprising chemically reducible cross-linking agents). The disulfide bonds can allow beads to degrade or dissolve when exposed to reducing agents.

For example, chitosan (a linear polysaccharide polymer) can be cross-linked with glutaraldehyde through hydrophilic chains to form beads. The cross-linking of the chitosan polymer can be achieved by chemical reactions initiated by heat, pressure, pH change and/or radiation.

For example, the bead can be a macromolecule of single or mixed monomers polymerized by various monomers such as agarose, polyalkenamide, and PEG, or macromolecular gel such as chitin, hyaluronic acid, and dextran, which is polymerized into gel beads with uniform size in drops by using a microfluidic drop platform.

For example, the bead can comprise an acrydite moiety, which in some aspects can be used to attach one or more nucleic acid molecules (e.g., barcode sequences, barcoded nucleic acid molecules, barcoded oligonucleotides, primers or other oligonucleotides) to the bead. For example, the acrydite moiety can refer to the acrydite analog produced by the reaction of acrydite with one or more substances, such as the reaction of acrydite with other monomers and cross-linking agents during the polymerization reaction. The acrydite moiety can be modified to form chemical bonds with substances to be attached, for example, nucleic acid molecules (e.g., barcode sequences, barcoded nucleic acid molecules, barcoded oligonucleotides, primers or other oligonucleotides). The acrydite moiety can be modified with thiol groups capable of forming disulfide bonds, or can be modified with groups that already comprise disulfide bonds. Thiol or disulfide (by disulfide exchange) can be used as the anchor point of the substance to be attached, or another part of the acrydite moiety can be used for attachment. For example, the attachment can be reversible, so that when the disulfide bond is broken (for example, in the presence of a reducing agent), the substance attached is released from the bead. In other cases, the acrydite moiety can comprise reactive hydroxyl groups that can be used for attachment. In addition to disulfide bonds, other release modes can also be included, for example, UV-induced release, or an enzyme can be used for the release

### Discrete partition and microfluidic device

The present application provides a device for co-distributing solid supports (e.g., beads) with samples, for example, for co-distributing sample components and beads to the same discrete partition. For example, the target nucleic acid derived from the single cell and the solid support attached with at least one oligonucleotide tag are co-distributed into the discrete partition.

For example, the device can be formed of any suitable material. For example, the device can be formed of a material selected from the group consisting of fused silica, soda-lime glass, borosilicate glass, poly(methylmethacrylate) PMMA, PDMS, sapphire, silicon, germanium, cycloolefin copolymer, polyethylene, polypropylene, polyacrylate, polycarbonate, plastic, thermosetting plastic, hydrogel, thermoplastic plastic, paper, elastomer and combinations thereof

For example, the discrete partition can include a hole or droplet. For example, the target nucleic acid derived from the single cell and the solid support attached with at least one oligonucleotide tag are co-distributed into the hole or droplet. For example, the holes can include the loading holes of a cell culture plate or any other container hole that can fit with the device and are suitable for co-distribution. For example, the discrete partition is a droplet. For example, wherein each of the discrete partitions includes at most the target nucleic acids derived from the single cell. For example, the target nucleic acid is located in a single cell or single nucleus. For example, the target nucleic acid derived from the single cell and the solid support attached with at least one oligonucleotide tag are co-distributed into the discrete partition using a microfluidic device.

For example, the discrete partitions (e.g., drops or holes) comprise single cells and are treated according to the methods described in this application. For example, the discrete partitions comprise single cells and/or single nuclei. The single cells and/or single nuclei can be distributed and treated according to the methods described in this application. For example, the single nucleus can be a building block of a cell. For example, the discrete partition comprises chromatin (e.g., a single chromosome or other parts of genome) from the single cell or single nucleus, and are distributed and treated according to the methods described in this application.

For example, a ligase is further comprised in the discrete partition, and the ligase links the oligonucleotide tag to the target nucleic acid attached. The discrete partition comprises, but not limited to, a ligase, and can further comprise other required enzymes. For example, DNA polymerase, DNA endonuclease, DNA exonuclease, terminal transferase, and light-sensitive enzymes capable of releasing the oligonucleotide tag from the solid support. The ligase includes, but not limited to, T4 ligase, for example, it can further include *E. coli* DNA ligase, T4 DNA ligase, T7 DNA ligase, mammalian ligase (e.g., DNA ligase I, DNA ligase III, DNA ligase IV), thermostable ligase, etc.

For example, the device is formed in such a way as to comprise a fluid flow channel. Any suitable channel can be used. For example, the device comprises one or more fluid input channels (e.g., inlet channels) and one or more fluid outlet channels. For example, the inner diameter of the fluid channel can be about 10 µm, 20 µm, 30 µm, 40 µm, 50 µm, 60 µm, 65 µm, 70 µm, 75 µm, 80 µm, 85 µm, 90 µm, 100 µm, 125 µm or 150 µm. For example, the inner diameter of the fluid channel can be greater than 10 µm, 20 µm, 30 µm, 40 µm, 50 µm, 60 µm, 65 µm, 70 µm, 75 µm, 80 µm, 85 µm, 90 µm, 100 µm, 125 µm, 150 µm or more. For example, the inner diameter of the fluid channel can be less than about 10 µm, 20 µm, 30 µm, 40 µm, 50 µm, 60 µm, 65 µm, 70 µm, 75 µm, 80 µm, 85 µm, 90 µm, 100 µm, 125 µm or 150 µm. The volumetric flow rate in the fluid channel can be any flow rate known in the art.

For example, the microfluidic device is a droplet generator. For example, a microfluidic device can be used to combine a solid support attached with at least one oligonucleotide tag with a sample (for example, a sample comprising a target nucleic acid) by forming an aqueous droplet comprising both the solid support attached with at least one oligonucleotide tag and the sample. The aqueous droplet is used as a discrete partition. The aqueous droplet can be an aqueous core surrounded by an oil phase, for example, an aqueous droplet in a water-in-oil emulsion. The aqueous droplet can contain one or more of solid supports attached with at least one oligonucleotide tag, samples, amplification reagents and reducing agents. For example, the aqueous droplets can comprise one or more of: water, nuclease-free water, solid supports attached with at least one oligonucleotide tag, acetonitrile, solid supports, gel solid supports, polymer precursors, polymer monomers, polyacrylamide monomers, acrylamide monomers, degradable cross-linking agents, non-degradable cross-linking agent, disulfide bonds, acrydite moieties, PCR reagents, cells, nuclei, chloroplast, mitochondria, ribosome, primers, polymerases, barcodes, polynucleotides, oligonucleotides, DNA, RNA, peptide polynucleotides, complementary DNA (cDNA), double-stranded DNA (dsDNA), single-stranded DNA (ssDNA), plasmid DNA, cosmid DNA, chromosomal DNA, genomic DNA, chloroplast DNA, mitochondrial DNA, ribosome RNA, viral DNA, bacterial DNA, mtDNA (mitochondrial DNA), mRNA, rRNA, tRNA, nRNA, siRNA, snRNA, snoRNA, scaRNA, microRNA, dsRNA, probes, dyes, organics, emulsifiers, surfactants, stabilizers, polymers, aptamers, reducing agents, initiators, biotin labels, fluorophores, buffers, acidic solutions, alkaline solutions, light-sensitive enzymes, pH-sensitive enzymes, aqueous buffers, oils, salts, detergents, ionic detergents, nonionic detergents, etc. In a word, the composition of the aqueous droplet will vary depending on the specific treatment requirements.

The aqueous droplets can have uniform size or non-uniform size. For example, the diameter of the aqueous droplet can be about 1 µm, 5 µm, 10 µm, 20 µm, 30 µm, 40 µm, 45 µm, 50 µm, 60 µm, 65 µm, 70 µm, 75 µm, 80 µm, 90 µm, 100 µm, 250 µm, 500 µm or 1 mm. For example, the fluid droplet can have a diameter of at least about 1 µm, 5 µm, 10 µm, 20 µm, 30 µm, 40 µm, 45 µm, 50 µm, 60 µm, 65 µm, 70 µm, 75 µm, 80 µm, 90 µm, 100 µm, 250 µm, 500 µm, 1 mm or more. For example, the fluid droplet can have a diameter of less than about 1 µm, 5 µm, 10 µm, 20 µm, 30 µm, 40 µm, 45 µm, 50 µm, 60 µm, 65 µm, 70 µm, 75 µm, 80 µm, 90 µm, 100 µm, 250 µm, 500 µm or 1 mm. For example, the fluid droplet can have diameters in the range of about 40-75 µm, 30-75 µm, 20-75 µm, 40-85 µm, 40-95 µm, 20-100 µm, 10-100 µm, 1-100 µm, 20-250 µm or 20-500 µm.

As described above, the microfluidic device (e.g., a droplet generator) can be used to combine a sample with a solid support (e.g., a library of barcoded solid supports attached with at least one oligonucleotide tag) and (if necessary) a reagent capable of degrading the solid support (e.g., a reducing agent if the solid support is linked by a disulfide bond). For example, a sample (e.g., a nucleic acid sample) can be provided to a first fluid input channel in fluid communication with a first fluid intersection (e.g., a first fluid junction). A preformed solid support (e.g., a solid support attached with at least one oligonucleotide tag, for example a degradable solid support) can be provided to a second fluid input channel also in fluid communication with the first fluid intersection, wherein the first fluid input channel and the second fluid input channel meet at the first fluid intersection. The sample and the solid support attached with at least one oligonucleotide tag can be mixed at the first fluid intersection to form a mixture (e.g., an aqueous mixture). For example, a reducing agent (or other reagents required, such as surfactants, stabilizers, polymers, aptamers, initiators, biotin labels, fluorophores, buffers, acidic solutions, alkaline solutions, light-sensitive enzymes, pH-sensitive enzymes, aqueous buffers, etc.) can be provided to a fourth fluid input channel, which is also in fluid communication with the first fluid intersection and meets the first and second fluid input channels at the first fluid intersection. Then, the reducing agent can be mixed with the solid support attached with at least one oligonucleotide tag and the sample at the first fluid intersection. For example, the reducing agent (or other reagents required, such as surfactants, stabilizers, polymers, aptamers, initiators, biotin labels, fluorophores, buffers, acidic solutions, alkaline solutions, light-sensitive enzymes, pH-sensitive enzymes, aqueous buffers, etc.) can also be premixed with the sample and/or the solid support attached with at least one oligonucleotide tag before entering the microfluidic device, so that the sample is provided to the microfluidic device through the first fluid input channel and/or the solid support attached with at least one oligonucleotide tag is provided to the microfluidic device through the second fluid input channel.

For example, the mixture of the sample comprising the target nucleic acid and the solid support attached with at least one oligonucleotide tag can leave the first fluid intersection through a first outlet channel in fluid communication with the first fluid intersection (and with any fluid channel constituting the first fluid intersection). The mixture can be provided to a second fluid intersection (e.g., a second fluid junction) in fluid communication with the first outlet channel. For example, an oil (or other suitable immiscible) fluid can enter the second fluid intersection from one or more separate fluid input channels that are in fluid communication with the second fluid intersection (and any fluid channel constituting the intersection) and meet the first outlet channel at the second fluid intersection. For example, the oil (or other suitable immiscible fluids) can be provided in one or two separate fluid input channels that are in fluid communication with the second fluid intersection (and with the first outlet channel) and meet the first outlet channel and each other at the second fluid intersection. the mixture of the oil, the sample and the solid support attached with at least one oligonucleotide tag can be mixed at the second fluid intersection. The resulting aqueous droplets can be transported in the oil through the second fluid outlet channel leaving from the second fluid intersection. For example, the resulting aqueous droplets can also leave the second outlet channel from the first fluid intersection, and the fluid droplets can be distributed into the holes for further treatment.

For example, the occupancy rate of the sample comprising the target nucleic acid relative to the solid support attached with at least one oligonucleotide tag can also be controlled. This control is described as in U.S. Patent Application Publication No.20150292988, the entire disclosure of which is incorporated herein by reference in its entirety for all purposes. Generally, the sample comprising the target nucleic acid is formed into droplets, so that at least 50%, 60%, 70%, 80%, 90% or more of the droplets contain no more than one solid support attached with at least one oligonucleotide tag. In addition, at least 50%, 60%, 70%, 80%, 90% or more of the samples comprising the target nucleic acid are formed into droplets which comprise exactly one solid support attached with at least one oligonucleotide tag.

For example, a sample can be premixed with a solid support (e.g., a degradable solid support) attached with at least one oligonucleotide label which comprises any other reagent (e.g., amplification agent, reduction agent, etc., which are required for sample amplification) before the mixture enters the microfluidic device, to produce an aqueous reaction mixture. When the aqueous mixture enters the fluid device, the mixture can flow from a first fluid input channel and enter a fluid intersection. For example, an oil phase can enter the fluid intersection from a second fluid input channel (e.g., a fluid channel perpendicular or substantially perpendicular to the first fluid input channel) which is also in fluid communication with the fluid intersection. The aqueous mixture and oil can be mixed at the fluid intersection, so that a water-in-oil emulsion (e.g., a solid support-water-oil emulsion) is formed. The emulsion can comprise a plurality of aqueous droplets (for example, droplets comprising the aqueous reaction mixture) in a continuous oil phase. For example, each aqueous droplet can comprise a single solid support (e.g., a gel solid support attached to a set of the same barcodes), an aliquot of the sample (e.g., a target nucleic acid from one cell), and an aliquot of any other reagent (e.g., reducing agent, reagent required for sample amplification, etc.). For example, the fluid droplets can comprise a plurality of solid supports attached with at least one oligonucleotide tag. When the droplets are formed, the droplets can be transported through a fluid outlet channel leaving the fluid intersection via the continuous oil phase. The fluid droplets leaving the outlet channel can be distributed into the holes for further treatment.

In case that the reducing agent can be added to the sample before entering the microfluidic device or the reducing agent can be added at the first fluid intersection, the fluid droplets formed at the second fluid intersection can contain the reducing agent. In this case, when the droplets travel through the outlet channel leaving the second fluid intersection, the reducing agent can degrade or dissolve the solid supports contained in the fluid droplets.

For example, the microfluidic device can contain three discrete fluid intersections in parallel. Fluid droplets can be formed at any of these three fluid intersections. A sample and a solid support attached with at least one oligonucleotide tag can be mixed at any of these three fluid intersections. Reducing agents (or any other required agent, e.g., permeabilization agents, amplification agents, and cutting agent to release oligonucleotide tags from solid supports) can be added at any of these three fluid intersections. Oil can be added at any of these three fluid intersections.

For example, the microfluidic device includes a first input channel and a second input channel, which meet at a junction in fluid communication with an output channel. For example, the outlet channel can be in fluid communication with a third input channel at a junction.

For example, the method further includes introducing a sample comprising the target nucleic acid into the first input channel, and introducing the solid support attached with at least one oligonucleotide tag into the second input channel, thereby generating a mixture of the sample and the solid support attached with at least one oligonucleotide tag in the output channel.

For example, a fourth input channel can also be comprised and it can intersect with the third input channel and the outlet channel at the joint. For example, the microfluidic device can comprise a first, second and third input channels, wherein the third input channel intersects with the first input channel, the second input channel or the junction of the first input channel and the second input channel. For example, the output channel is in fluid communication with a third input channel at a junction. For example, the first input channel and the second input channel form a substantially perpendicular angle with each other.

For example, it further includes introducing oil into the third input channel, so that aqueous droplets in the water-in-oil emulsion are formed as the discrete partitions. For example, each of the discrete partitions comprises at most the target nucleic acids from the single cell.

The methods, compositions, devices and kits of this application can be used with any suitable oil. For example, oil can be used to produce droplets. For example, the oil can include fluorinated oil, silicone oil, mineral oil, vegetable oil and combinations thereof.

For example, the aqueous fluid in the microfluidic device can also contain alcohol. For example, the alcohol can be glycerol, ethanol, methanol, isopropanol, pentanol, ethane, propane, butane, pentane, hexane and combinations thereof. The alcohol can be present in the aqueous fluid at about 5%, 6%, 7%, 8%, 9%, 10%, 11%, 12%, 13%, 14%, 15%, 16%, 17%, 18%, 19% or 20% (v/v). For example, the alcohol can be present in the aqueous fluid at a concentration of at least about 5%, 6%, 7%, 8%, 9%, 10%, 11%, 12%, 13%, 14%, 15%, 16%, 17%, 18%, 19%, 20% or higher (v/v). For example, the alcohol can be present in the aqueous fluid at less than about 5%, 6%, 7%, 8%, 9%, 10%, 11%, 12%, 13%, 14%, 15%, 16%, 17%, 18%, 19% or 20% (v/v).

For example, the oil can also contain surfactants to stabilize the emulsion. For example, the surfactant can be fluorine-containing surfactant, Krytox lubricant, Krytox FSH, an engineered fluid, HFE-7500, silicone compound, a silicon compound containing PEG, such as bis krytoxpeg (BKP). The surfactant can be present at about 0.1%, 0.5%, 1%, 1.1%, 1.2%, 1.3%, 1.4%, 1.5%, 1.6%, 1.7%, 1.8%, 1.9%, 2%, 5% or 10% (w/w). For example, the surfactant can be present at a concentration of at least about 0.1%, 0.5%, 1%, 1.1%, 1.2%, 1.3%, 1.4%, 1.5%, 1.6%, 1.7%, 1.8%, 1.9%, 2%, 5%, 10% (w/w) or higher. For example, the surfactant can be present at less than about 0.1%, 0.5%, 1%, 1.1%, 1.2%, 1.3%, 1.4%, 1.5%, 1.6%, 1.7%, 1.8%, 1.9%, 2%, 5% or 10% (w/w).

For example, an accelerator and/or an initiator can be added to the oil. For example, the accelerator can be tetramethylethylene diamine (TMEDA or TEMED). For example, the initiator can be ammonium persulfate or calcium ion. The accelerator can be present at about 0.1%, 0.2%, 0.3%, 0.4%, 0.5%, 0.6%, 0.7%, 0.8%, 0.9%, 1%, 1.1%, 1.2%, 1.3%, 1.4%, 1.5%, 1.6%, 1.7%, 1.8%, 1.9% or 2% (v/v). For example, the accelerator can be present at a concentration of about 0.1%, 0.2%, 0.3%, 0.4%, 0.5%, 0.6%, 0.7%, 0.8%, 0.9%, 1%, 1.1%, 1.2%, 1.3%, 1.4%, 1.5%, 1.6%, 1.7%, 1.8%, 1.9% or 2% (v/v) or higher. For example, the accelerator can be present at less than about 0.1%, 0.2%, 0.3%, 0.4%, 0.5%, 0.6%, 0.7%, 0.8%, 0.9%, 1%, 1.1%, 1.2%, 1.3%, 1.4%, 1.5%, 1.6%, 1.7%, 1.8%, 1.9% or 2% (v/v).

### Cell and sample

In this application, the cells are cells of any organism. The cells of organisms can be in vitro cells (for example, established cultured cell lines) or ex vivo cells (cultured cells from individuals, i.e., primary cells). Cells can be cells in vivo (cells in biological individuals), for example, cells from various tissues.

For example, the cells of organisms can include animal cells, plant cells and microbial cells. For example, the plant cells can include Arabidopsis thaliana cells, and can also include cells of agricultural crops, for example, somatic cells of plants such as wheat, corn, rice, sorghum, millet, and soybean; and the plant cells can also include cells of fruits and nut plants, for example, plants that produce apricots, oranges, lemons, apples, plums, pears, almonds, walnuts, etc. For example, the plant cells can be cells from any part of plants, for example, root cells, leaf cells, xylem cells, phloem cells, cambium cells, apical meristem cells and parenchyma cells.

For example, the microbial cells can include bacterial (e.g., *E. coli* and *archaea*), fungal (e.g., yeast), actinomycetal, rickettsial, mycoplasmal, chlamydial, spirochetal cells, etc.

For example, the animal cells can include invertebrate (e.g., Drosophila melanogaster, nematode, planarian, etc.) cells and vertebrate (e.g., zebrafish, chicken, mammal) cells.

For example, the mammal cells can include mice, rats, rabbits, pigs, dogs, cats, monkeys, humans, etc.

For example, the animal cells can include cells from any tissue of an organism, for example, stem cells, induced pluripotent stem cells (iPS), germ cells (e.g., oocyte, ootid, sperm cells, etc.), adult stem cells, somatic cells (e.g., fibroblast cells, hematopoietic cells, myocardial cells, neurons, muscle cells, bone cells, liver cells, pancreatic cells, epithelial cells, immune cells, and any cell derived from organs or tissues such as lung, spleen, kidney, stomach, large intestine and small intestine) as well as cells of embryos at any stage in vitro or in vivo.

For example, the cells can be cells from body fluids of an organism. For example, the body fluids of the organism can include cerebrospinal fluids, aqueous humor, lymph, digestive fluids (e.g., saliva, gastric fluids, intestinal fluids, bile, etc.), milk, blood, urine, sweats, tears, feces, respiratory secretions, genital secretions (e.g., semen and cervical mucus), etc.

The sample includes the cell and/or the nucleus obtained therefrom.

For example, the sample can include nucleic acid molecules of the organism. The nucleic acid molecules can be isolated and extracted from any organism by the technical means of isolating nucleic acid molecules known to those skilled in the art, including DNA and RNA. For example, the nucleic acid molecules are extracted from the above-mentioned organism cells or body fluids of an organism.

For example, the target nucleic acid can include the nucleic acid from any cell above. For example, the nucleic acid in a single cell.

For example, the target nucleic acid can be a polynucleotide from a single cell, for example, double-stranded DNA. For example, the double-stranded DNA can include genomic DNA, e.g., coding DNA and non-coding DNA; and for example, accessible chromatin region DNA, protein binding site DNA, mitochondrial DNA and chloroplast DNA. For example, the polynucleotide can include RNA, e.g., ribosomal RNA, and mRNA.

For example, the target nucleic acid can also be a Formalin-Fixed and Paraffin-Embedded (FFPE) sample containing cells.

For example, the target nucleic acid can further include sequences containing SNP sites in the genome of the organism, and nucleotide sequences modified by methylation and hydroxymethylation.

For example, the cells can further be pretreated. For example, the pretreatment further includes exposing the nucleus of the cell. For example, the nucleus can be exposed by treating with a lysis buffer and a concentrated sucrose solution.

For example, the cell and/or the nucleus exposed (obtained) therefrom can be wrapped in a suitable matrix to form a microsphere, which is reacted as a sample.

For example, the pretreatment includes fixing the cell and/or the nucleus exposed (obtained) therefrom. For example, the cell is immobilized using a fixing agent selected from one or more of the group consisting of formaldehyde, paraformaldehyde, methanol, ethanol, acetone, glutaraldehyde, osmic acid and potassium dichromate.

Wherein, the pretreatment includes treating the cell or nucleus with a detergent including Triton, Tween, SDS, NP-40 and/or digitonin.

For example, the pretreatment can further include removing organelles such as mitochondria, chloroplasts, and ribosomes.

For example, cells can be distributed together with a lytic agent to release the contents of cells within discrete partitions. For example, the lytic agent is brought into contact with the cell suspension when the cells are introduced into the droplet generation region through an additional channel, or just before the cells are introduced into the droplet generation region. The lytic agent can include bioactive reagents, for example, lytic enzymes for lysing different cell types (e.g., gram positive or negative bacteria, plants, yeasts, mammals, etc.), e.g., lysozyme, colorless peptidase, lysostaphin, kitalase, lyticase, and other commercially available lytic enzymes. For example, other lytic agents can also be co-distributed with the cells to release the contents of the cells into discrete partitions. For example, a surfactant-based lytic solution can be used to lyse cells. For example, the lytic solution can include nonionic surfactants such as TritonX-100 and Tween 20. For example, the lytic solution can include ionic surfactants, such as sodium dodecyl sarcosinate and sodium dodecyl sulfate (SDS). For example, other lytic methods available (such as electroporation, heat, sound or mechanical cell destruction) can also be used.

### Composition and kit

The present application further provides a composition, which comprises a plurality of solid supports each attached with at least one oligonucleotide tag, wherein each of the oligonucleotide tags comprises a first chain and a second chain, where the first chain comprises a barcode sequence and a hybridization sequence located at the 3' end of the barcode sequence, the second chain comprises a first portion complementary to the hybridization sequence of the first chain and a second portion complementary to the sequence in the nucleic acid to be tested, and the first chain and the second chain form a partially double-stranded structure, or the second chain and the target nucleic acid attached form a partially double-stranded structure; and the barcode sequence of the oligonucleotide tag comprises a common barcode domain and a variable domain, where the common barcode domains are the same in the oligonucleotide tags attached to the same solid support, and the common barcode domains are different between two or more solid supports in the plurality of solid supports. The present application further provides a kit for analyzing a target nucleic acid from a cell, which comprises the composition described in this application. For example, the kit can further include a transposase. For example, the kit further comprises at least one of a nucleic acid amplification agent, a reverse transcription agent, a fixing agent, a permeabilization agent, a ligation agent and a lysis agent.

Not to be limited by any theory, the following examples are only used to illustrate the methods and use in this application, and are not intended to limit the scope of the invention of this application.

### Examples

### Example 1 Detection of accessible chromatin regions (ATAC)

(1) Preparation of a nucleotide tag comprising a barcode sequence, which was fixed on a solid support.

The nucleotide tag had two chains, thus forming a partial double-stranded structure 1, as shown below:
chain I: solid support~attachment sequence-barcode-hybridization sequence (a fixing sequence hybridizing with the complementary portion of chain II), where the barcode was (barcode-linker)n with n greater than or equal to 1.

A specific example: Bead-acrydite-S-S-ACACTCTTTCCCTACACGACGCTCTTCCGATCT (read1, SEQ ID NO: 6)-barcode-ATCCACGTGCTTGAG (SEQ ID NO: 12)
chain II: hybridization sequence (a fixing sequence hybridizing with the fixing DNA sequence of chain I)-the sequence complementary to the 5' end of the transposon complex chain I

A specific example: CGAATGCTCTGGCCTCTCAAGCACGTGGAT (SEQ ID NO:9)

The solid support was a polyacrylamide microsphere, which was prepared by a microfluidic equipment as follows: the acrylamide: Bis mixture, acrydite-DNA primer and the APS inducer were mixed into droplets in the microfluidic device, in which a TEMED catalyst was contained, and the droplets would spontaneously polymerize into gel microspheres, and then the microspheres were labeled according to the synthesis method of barcodes.

In the ligation reaction, the solution contained 10 mM DTT, and the S-S bond could be reduced to release the primer.

(2) Preparation of a transposon complex and assembly of Tn5 transposon containing a DNA partial double-stranded sequence.

One of the DNA sequences contained was a double-stranded structure 2 formed by annealing the A chain and the B chain.
chain A: phosphate group-the sequence that is at least partially complementary to the fixing DNA sequence in chain I or chain II of the nucleic acid molecule in chain II-(UMI)-Tn5 transposase binding sequence

A specific example: AGGCCAGAGCATTCGNNNNNNNAGATGTGTATAAGAGACAG (SEQ ID NO: 5)
chain B: Tn5 transposase binding sequence (the sequence complementary to the sequence binding to the transposon protein (Tn5) in chain A)-phosphate group

A specific example: p-CTGTCTCTTATACACATCT (SEQ ID NO: 4)
where, the UMI in chain A was not necessary; and the sequences in (1) and (2) may contain modifying bases, such as 5mC.

The Tn5 transposition complex was a dimer, and two Tn5 proteins could bind to the same or different partial double-stranded DNA transposons, so that the insertion site was labeled by one or two kinds of DNA; and the Tn5 proteins (which could comprise point mutation hyperactivity, or other types of transposases) could be assembled into a hybrid transposition complex with the above double-stranded structure 2 and another standard transposon DNA, or only the above double-stranded structure 2 could be used to form a single Tn5 transposition complex.

(3) Preparation of samples. The samples could be non-fixed cells or nuclei, cells or nuclei fixed by formaldehyde (or other fixing agents), non-fixed or fixed tissue slices, etc. Wherein, the fixed or non-fixed sample was treated with a buffer solution comprising a detergent (Triton, NP-40, or Digitonin, etc.), which could also include the intermediate step of lysing the cells (the non-fixed samples) to obtain the nuclei. The cells and nuclei were lysed and permeabilized by the detergent, so that Tn5 enzyme could enter the nuclei for action. Typical permeabilization agent solutions can include Tris, sucrose, sodium chloride and a detergent.

(4) Transposition reaction. A Tn5 enzyme buffer comprising a divalent metal ion (e.g., a magnesium ion) was added to the above treated samples, and the assembled Tn5 transposition complex was added to carry out the ATAC transposition reaction (37°C, 30 minutes-2 hours). That is, the reaction system included: the cells or nuclei or tissues; the Tn5 transposition complex; and the buffer. After the reaction, the samples were washed with the buffer to remove the unreacted Tn5 enzyme.

(5) Ligation reaction. A T4 DNA ligase reaction buffer, the nucleotide tags in ligation step (1), T4 DNA ligase, and the nucleotide tags were added to carry out a ligation reaction at a appropriate temperature (4°C-37°C) for more than 20 minutes.

The reaction system included: the cells or nuclei or tissues (after the transposition reaction); T4 DNA ligase, and the nucleotide tags. After the reaction, excessive amounts of the nucleotide tags in free form and sequences complementary to the nucleotide tags were added into the ligation reaction system to block the excess unreacted nucleotide tags.

(6) DNA extraction from cells. For non-fixed samples, a lysis solution was directly added, then the purification was carried out by a method such as a DNA extraction kit, and magnetic beads; and for the fixed samples, a protease K reaction buffer and protease K were added, and then the DNA was purified after de-crosslinking at 55-65°C.

For the purified DNA, 1) if hybrid Tn5 was used, the product had PCR amplification sequences on both sides, which could be directly amplified to obtain a sequencing library.

2) If a single Tn5 was used, only one side of the DNA product had PCR primers. We needed to interrupt and link this DNA, and add amplification primers on the other side. This could be done by using a single Tn5 enzyme, or by using ultrasound or enzyme to interrupt, and then adding A and an adaptor at the end to obtain the sequencing library.

According to the above steps, taking human 293T cells as an example, fresh cells were taken to prepare nuclei. An ATAC reaction was carried out with a hybrid Tn5, and then an adaptor of the amplification sequences on the P5 end (read1) side of a Illumina sequencing library was linked to construct a library. The products were amplified with read1 primers, and read2 primers of another DNA fragment in the hybrid Tn5, and finally analyzed. The specific steps were as follows:

### A. Tn5 transposome

The following sequences were annealed to form a double strand:
10 µM Top1 5'p-AGGCCAGAGCATTCGNNNNNNNAGATGTGTATAAGAGACAG (SEQ ID NO: 5) (chain A)
10 µM Top2 GTCTCGTGGGCTCGGAGATGTGTATAAGAGACAG (SEQ ID NO: 3) (chain A)
20 µM Bottom 5'p-CTGTCTCTTATACACATCT (SEQ ID NO: 4) (chain B)

Then, it was incubated with 10 µm of the Tn5 enzyme (purchased from the Epicenter Company) at room temperature to assemble into 10 µm of Tn5 transposon. The transposon formed by the Top1/Bottom double strand and Tn5 was p-Tn5, and the transposon formed by the Top2/Bottom double strand and Tn5 was TN5-B.

### B. Preparation of beads

1) the sequence attached to the bead was as follows: Bead-S-S-PCR adapter-barcode1-linker1-barcode2-linker2-barcode3 -ligation linker
   where, the PCR adaptor sequence was ACACTCTTTCCCTACACGACGCTCTTCCGATCT (SEQ ID NO: 6), Linker1 was CGACTCACTACAGGG (SEQ ID NO: 7), the sequence of Linker2 was TCGGTGACACGATCG (SEQ ID NO: 8), the sequence of Ligation linker was ATCCACGTGCTTGAG (SEQ ID NO: 12). Barcode1=96 kinds of 5bp base sequences, Barcode2=96 kinds of 5bp base sequences, and Barcode1=96 kinds of 5bp base sequences.
2) Synthesis of 3x96 sequences
   1. PCR handle-96x barcode1-linker1, synthesizing 96 reverse complementary sequences of this sequence;
   2. linker1-96x barcode2-linker2, synthesizing 96 reverse complementary sequences of this sequence;
   3. linker2-96x barcode3-ligation linker, synthesizing 96 reverse complementary sequences of this sequence.
3) Synthesis of microspheres:
   The following amino sequences were synthesized: 5' amine-S-S-ACACTCTTTCCCTACACGACGCTCTTCCGATCT (SEQ ID NO: 6) and 30 µm of carboxyl modified microspheres (Zhiyi, KBsphere^{®}, www.kbspheres.com/productshow.asp?id=903).

Coupling reaction: microsphere +50 mM EDC+100 µM amino sequence (SEQ ID NO: 6), the amino sequence and the carboxyl microspheres were coupled to obtain the following structure: bead-S-S-ACACTCTTTCCCTACACGACGCTCTTCCGATCT (SEQ ID NO: 6)

### 4) Attachment of tags

The synthesized microspheres were evenly divided into a 96-well plate, and PCR handle-96xbarcode1-linker1 was added respectively for the first round of barcoding reactions. The reaction system and process were as follows: 10 µl microsphere + 2 µl BstI buffer + 1 µl 10 µm dNTP + 1 µl 100 µm PCR handle-96xbarcode1-linker1. Then they were kept at 95°C for 5 min and 60°C for 20 min; and then, 1 µl BstI+5 µl H₂O was added and kept at 60°C for 60 min.

After the first round of the barcoding reactions, all microspheres were collected, mixed, and reacted at 95c for 5 min to remove complementary chains, and washed to obtain the first round of microspheres barcoded (96xbarcode1-linker1). Then the microspheres were evenly divided into a 96-well plate, and linker1-96xbarcode2-linker2 (the second round) and linker2-96x barcode 3-ligation linker (the third round) were added. The second and third rounds of barcoding reactions were carried out according to the system method of the first round, and single-stranded microspheres with triple barcodes were finally obtained. After being washed, the microspheres were annealed with the complementary sequence CGAATGCTCTGGCCTCTCAAGCACGTGGAT (SEQ ID NO: 9) to form a partial double-stranded structure, and the microspheres attached with the partial double-stranded structure as follows were finally obtained:
Bead-S-S-ACACTCTTTCCCTACACGACGCTCTTCCGATCT (SEQ ID NO: 6)-barcode1-CGACTCACTACAGGG-barcode2-TCGGTGACACGATCG (SEQ ID NO: 8)-barcode3-ATCCACGTGCTTGAG (SEQ ID NO: 12)
3'-TAGGTGCACGAACTCTCCGGTCTCGTAAGC-5' (reverse arrangement of SEQ ID NO: 9)

### C. ATAC experiment

Human 293T cell lines were resuspended in the lysis solution (10 mM Tris-Cl, pH 7.4; 10 mM NaCl; 3 mM MgCl₂; and 0.01% NP-40) for lysing the cells, to obtain nuclei.

100,000 of the nuclei were taken and reacted with p-Tn5 and Tn5-B obtained in step (1). The reaction system was as follows:
25 µl 2xTD Buffer (Illumina) + 2.5 µl 10 µM p-Tn5 + 2.5 µl 10 µM Tn5-B + 20 µl nucleus (100,000). They were reacted at 37°C for 30 min, and the nuclei were washed with PBS.

### D. High-throughput labeling

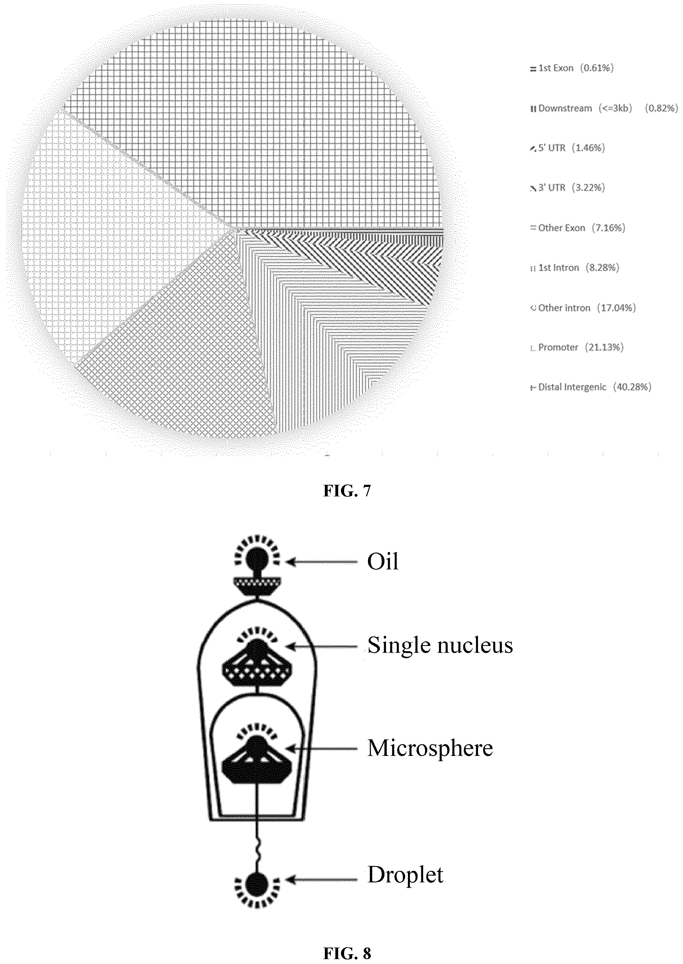

The microfluidic chip as shown in Fig. 8 was used for cell labeling, with Bead channel: 100 µm and Nuclei Channel: 50 µm.

The following solutions were prepared:
1 ml of a nucleus solution (100 nuclei/µl concentration), including: 200 µl 10xT4 DNA ligase Buffer, 10 µl T4 DNA ligase, 10 µl 1M DTT, and 780 µl nucleus/water.

A bead solution (100 beads/µl concentration): Beads in PBS.

Drops with a diameter of 120 µm were collected from the nucleus solution, bead solution and oil (FC40 fluorocarbon oil, containing 1% of the surfactant FluoroSurfactant, Ran Biotech) on the microfluidic chip, and linked at 37°C for 1 hour.

### E. Construction of library

Equal volume of perfluorooctanol fragmenting drops were added to the drops in step D, and centrifuged. The aqueous phase was pipetted, and the DNA in the aqueous phase was purified with the Qiagen DNA purification kit, and amplified with the following reaction system to obtain the final sequencing library: 36 µl DNA template, 10 µl 5xPCR Buffer, 1 µl 10 mM dNTP, 1 µl 10 µm primer TrueseqD501, 1 µl 10 µM primer N701, 1 µl Taq, 94°C for 2 min, 94°C for 30 sec, 55°C for 30 sec and 72c for 30 sec, 18 cycles.
Sequence of primer TrueseqD501: AATGATACGGCGACCACCGAGATCTACACTCTTTCCCTACACGACGCTCTTCCGATCT (SEQ ID NO: 10)
Sequence of primer N701: CAAGCAGAAGACGGCATACGAGATATCGGCTAGTCTCGTGGGCTCGG (SEQ ID NO: 11)
100,000 PE150 reads were measured by Illumina Novaseq in each cell.

The above library comprised about 500 cells, and 100,000 PE150 reads were sequenced in each cell, with a total data volume of 15G.

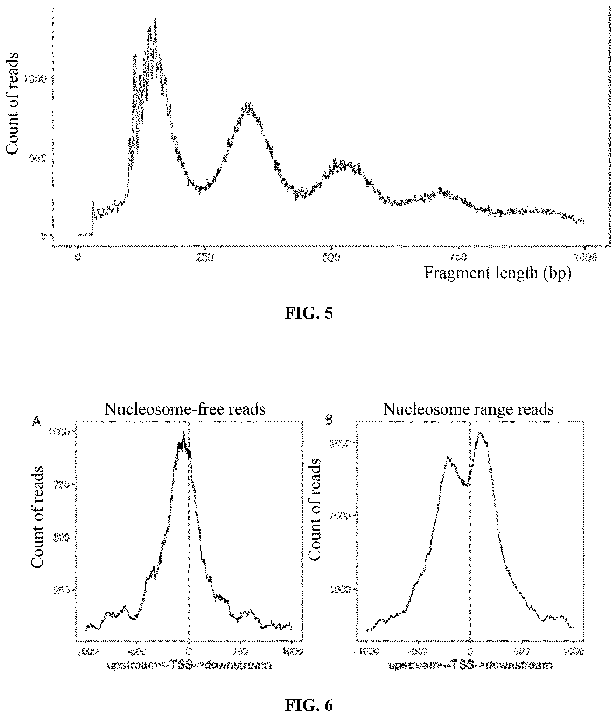

The esATAC software was used for analysis and quality control of the data, and all of the sequencing data were combined for analysis. As shown in Figs. 5-7, the size of the sequenced fragments represents a typical nucleosome gradient of ATAC (see Fig. 5), and the signal-enriched transcription start sites (TSS) represents a typical ATAC signal (see Fig. 6A and 6B). The peaks are highly coincident with the known accessible regions (see Fig. 7), wherein the total peak value is 11898. The Peaks overlapped with union DHS ratio is 74.0%, the Peaks overlapped with blacklist ratio is 0.5%, and FRiP (Fraction of reads in peaks) is 99.8%. These results show that this method can precisely detect ATAC insertion products mediated by Tn5 in cells.

### F. Single cell data splitting and analysis

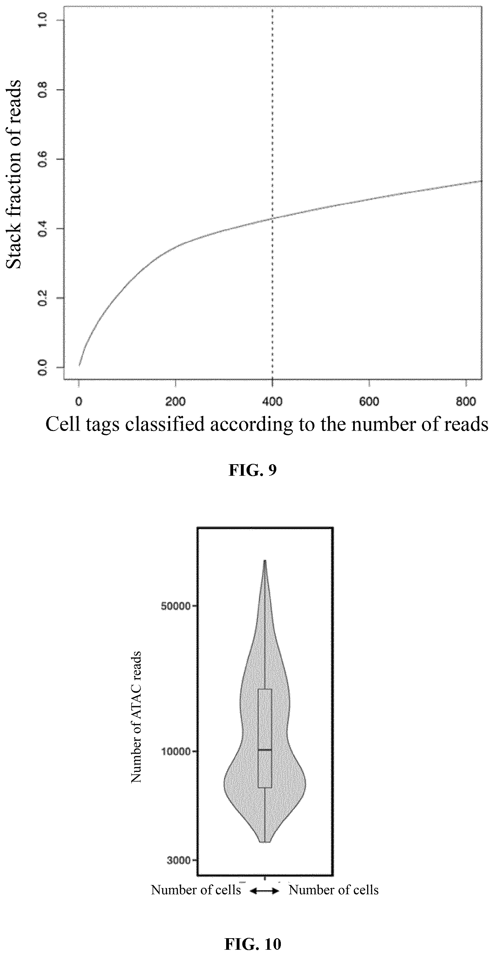

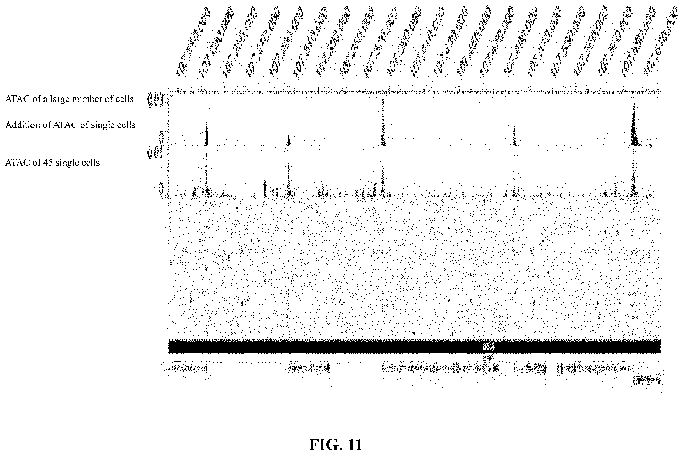

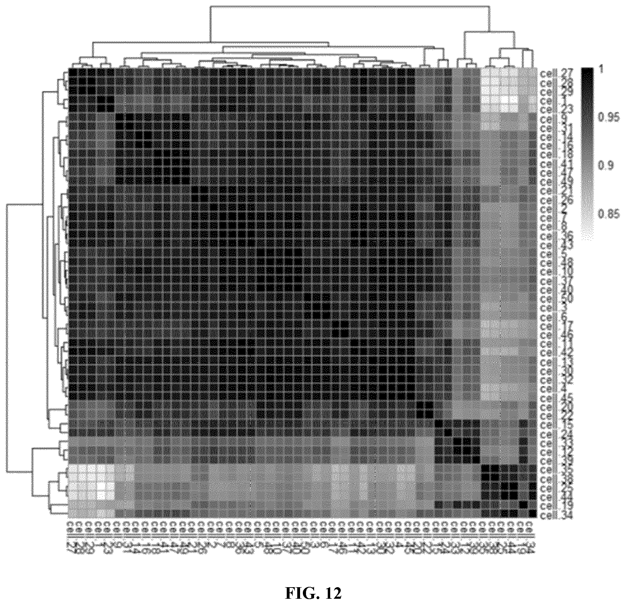

For the above sequencing data, firstly, cell nucleic acid tags (cell barcodes) were identified by using the Dropseq pipeline. In read1, 1-45bp was barcode positions. According to the combination possibility of 96x96x96 barcodes, the number of reads in each barcode was counted, a stackplot was drawn, and the number of effective cells in the library was determined to be about 400, as shown in Fig. 9.
ii. The number of unique mapped reads in each cell was obtained. Its distribution is shown in Fig. 10, that is, averagely, the median number of ATAC reads obtained for each cell is about 10000, which is better than the average value of about 2-3000 in Bing Ren's.
iii. Results of the single-cell ATAC. The information of the position of each read aligned to the genome (obtained by the dropseq program flow) was loaded into the IGV genome browser for visualization, to get the result as shown in Fig. 11. The bottom part of the figure shows the distribution of ATAC data of 45 single cells in the gene region. The middle part of the picture is the result presentation of addition of the ATAC data of 45 single cells together. It is highly similar to the ATAC pattern of a large number of cells (about 10,000 cells) in the upper part of the figure, and the cells were at the gene transcription start sites.
iv. Single cell correlation analysis. By calculating the Pearson Correlation function in R language package, the result shown in Fig. 12 was obtained. The darker the color in the figure, the higher the correlation between cells. It can be seen from the figure that the ATAC signal of the single cell is highly correlated, which indicates the true accuracy of the single cell data obtained by this method.

### Example 2 Detection of interaction between DNA and proteins

(1) Instead of the traditional ChIP-seq method, CUT&Tag is the latest method to study the interaction between DNA and a protein. Its principle is that a Protein A (a protein of cell origin that can bind to the conserved region of an antibody heavy chain from different species) forms a fused protein with Tn5. Through the binding of protein A and an antibody, the Tn5 enzyme is targeted to the target protein bound by the antibody. DNA fragments are directly inserted into the DNA region bound by the target protein through the transposition activity of the Tn5 enzyme. This product is amplified and sequenced, thereby obtaining the binding position information of the protein directly. Therefore, the molecular product of CUT&Tag is the same as ATAC, except that the Tn5 enzyme insertion site in ATAC is in the accessible chromatin region, and the Tn5 insertion site in CUT&Tag is around the target protein. Therefore, this product can be labeled by a method similar to ATAC in Example 1, the DNA transposon used is similar to ATAC, and one or a hybrid Tn5 transposition complex can also be assembled. The different step is: assembling a Tn5 transposition complex with a Protein A or G-Tn5 fusion protein; and in order to distinguish various antibodies, DNA transposon can comprise antibody identification codes in different positions besides the sequence of ATAC Tn5, to distinguish various antibodies.
(2) Sample preparation: samples could be non-fixed cells or nuclei, cells or nuclei fixed by formaldehyde (or other fixing agents), non-fixed or fixed tissue slices, etc. Wherein, the fixed or non-fixed sample was treated with a buffer solution comprising a detergent (Triton, NP-40, or Digitonin, etc.), which could also include the intermediate step of lysing the cells (the non-fixed samples) to obtain the nuclei. The cells and nuclei were lysed and permeabilized by the detergent, so that Tn5 enzyme could enter the nuclei for action.
(3) Antibody binding. The sample was blocked with serum BSA and the like, then an primary antibody was added to bind the target protein, and wash to remove the redundant primary antibody. Further, the secondary antibody against the primary antibody can be used to continuously bind the sample (this step was not necessary), to increase the binding sites of Protein A/G, and amplify the signal. To detect the interaction between two kinds of proteins simultaneously, the primary antibody could be bound to the Protein A/G-Tn5 fusion protein first to form a complex. The DNA on each antibody-bound Protein A/G-Tn5 fusion protein had different antibody identification code. At the same time, two or more primary antibody-Protein A/G-Tn5 fusion protein complexes were directly bound to cells/tissues to bring Tn5 around the target protein in one step.
(4) Transposition reaction. The Protein A-Tn5 fusion protein (primary antibody-Protein A-Tn5 fusion protein complex) was bound to the sample, the excess enzyme was washed, and then a Tn5 reaction solution containing a divalent ion was added into the sample for transposition reaction (37°C, 30 minutes-2 hours).
(5) The ligation reaction and subsequent treatment were carried out as in Example 1, a library was constructed and sequenced.

The specific steps were as follows:
(1) Preparation of a nucleotide tag comprising a barcode sequence, which was fixed on a solid support. The nucleotide tag had two chains, thus forming a partial double-stranded structure 1, as shown below:
   chain I: solid support-attachment sequence-barcode-hybridization sequence (a fixing sequence hybridizing with the complementary portion of chain II), where the barcode was (barcode-linker)n with n greater than or equal to 1.

A specific example: Bead-acrydite-S-S-ACACTCTTTCCCTACACGACGCTCTTCCGATCT (read1, SEQ ID NO: 6)-barcode-ATCCACGTGCTTGAG (SEQ ID NO: 12)
chain II: hybridization sequence (a fixing sequence hybridizing with the fixing DNA sequence of chain I)-the sequence complementary to the 5' end of the transposon complex chain I

A specific example: CGAATGCTCTGGCCTCTCAAGCACGTGGAT (SEQ ID NO:9)

The solid support was a polyacrylamide microsphere, which was prepared by a microfluidic equipment as follows: the acrylamide: Bis mixture, acrydite-DNA primer and the APS inducer were mixed into droplets in the microfluidic device, in which a TEMED catalyst was contained, and the droplets would spontaneously polymerize into gel microspheres, and then the microspheres were labeled according to the synthesis method of barcodes.

In the ligation reaction, the solution contained 10 mM DTT, and the S-S bond could be reduced to release the primer.

(2) Preparation of a transposon complex and assembly of pA-Tn5 transposon containing a DNA partial double-stranded sequence.

One of the DNA sequences contained was a double-stranded structure 2 formed by annealing the A chain and the B chain.
chain A: phosphate group-the sequence that is at least partially complementary to the fixing DNA sequence in chain I or chain II of the nucleic acid molecule in chain II-(UMI)-Tn5 transposase binding sequence

A specific example: AGGCCAGAGCATTCGNNNNNNNAGATGTGTATAAGAGACAG (SEQ ID NO: 5)
chain B: Tn5 transposase binding sequence (the sequence complementary to the sequence binding to the transposon protein (Tn5) in chain A)-phosphate group

A specific example: p-CTGTCTCTTATACACATCT (SEQ ID NO: 4)
where, the UMI in chain A was not necessary; and the sequences in (1) and (2) could contain modifying bases, such as 5mC.

The Tn5 transposition complex was a dimer, and two pA-Tn5 proteins could bind to the same or different partial double-stranded DNA transposons, so that the insertion site was labeled by one or two kinds of DNA; the pA-Tn5 proteins (which could comprise point mutation hyperactivity, or other types of transposases) could be assembled into a hybrid transposition complex with the above double-stranded structure 2 and another standard transposon DNA, or only the above double-stranded structure 2 could be used to form a single Tn5 transposition complex.

### Specific operation:

Equimolar concentration of the pA-Tn5 protein and the annealed double-stranded primer were mixed, and then placed at room temperature for more than 1 hour to form a functional transposon complex.

(3) Preparation of sample. The sample could be non-fixed cells or nuclei, cells or nuclei fixed by formaldehyde (or other fixing agents), non-fixed or fixed tissue slices, etc. Wherein, the fixed or non-fixed sample was treated with a buffer solution comprising a detergent (Triton, NP-40, or Digitonin, etc.), which could also include the intermediate step of lysing the cells (the non-fixed samples) to obtain the nuclei. The cells and nuclei were lysed and permeabilized by the detergent, so that the antibody and the pA-Tn5 enzyme could enter the nuclei for action. Typical permeabilization agent solutions can include Tris, sucrose, sodium chloride and a detergent.

The antibody against the target protein was incubated with the sample to specifically bind the antibody to the target protein, and washed to remove the unbound antibody. Then, the pA-Tn5 transposon was incubated with the sample to bind the pA-Tn5 protein to the antibody, and thus locating it around the target protein.

(4) Transposition reaction. A Tn5 enzyme buffer comprising a divalent metal ion (e.g., a magnesium ion) was added to the above treated samples, to carry out the transposition reaction (37°C, 30 minutes-2 hours). That is, the reaction system included: the cells or nuclei or tissues; and the buffer. After the reaction, the samples were washed with the buffer to remove the unreacted reagent.

(5) Ligation reaction. A T4 DNA ligase reaction buffer, the nucleotide tags in ligation step (1), T4 DNA ligase, and the nucleotide tags were added to carry out a ligation reaction at an appropriate temperature (4°C-37°C) for more than 20 minutes.

The reaction system included: the cells or nuclei or tissues (after the transposition reaction); T4 DNA ligase, and the nucleotide tags. After the reaction, excessive amounts of the nucleotide tags in free form and sequences complementary to the nucleotide tags were added into the ligation reaction system to block the excess unreacted nucleotide tags.

(6) DNA extraction from cells. For non-fixed samples, a lysis solution was directly added, then the purification was carried out by a method such as a DNA extraction kit, and magnetic beads; and for the fixed samples, a protease K reaction buffer and protease K were added, and then the DNA was purified after de-crosslinking at 55-65°C.

For the purified DNA, 1) if hybrid Tn5 was used, the product had PCR amplification sequences on both sides, which could be directly amplified to obtain a sequencing library.

2) If a single Tn5 was used, only one side of the DNA product had PCR primers. We needed to interrupt and link this DNA, and add amplification primers on the other side. This could be done by using a single Tn5 enzyme, or by using ultrasound or enzyme to interrupt, and then adding A and an adaptor at the end to obtain the sequencing library.

According to the above steps, taking human 293T cells as an example, fresh cells were taken to prepare nuclei. A CUT Tag reaction was carried with a hybrid pA-Tn5, and then a linker of the amplification sequences on the P5 end (read1) side of a Illumina sequencing library was linked to construct a library. The products were amplified with read1 primers, and read2 primers of another DNA fragment in the hybrid pA-Tn5, and finally analyzed. The specific steps were as follows:

### A. pA-Tn5 transposome

The following sequences were annealed to form a double strand:
10 µM Top1 5'p-AGGCCAGAGCATTCGNNNNNNNAGATGTGTATAAGAGACAG (SEQ ID NO: 5) (chain A)
10 µM Top2 GTCTCGTGGGCTCGGAGATGTGTATAAGAGACAG (SEQ ID NO: 3) (chain A)
20 µM Bottom 5'p-CTGTCTCTTATACACATCT (SEQ ID NO: 4) (chain B)

Then, it was incubated with 10 µm of the pA-Tn5 enzyme (purchased from azyme) at room temperature to assemble into 10 µm of pA-Tn5 transposon. The transposon formed by the Top1/Bottom double strand and Tn5 was p-pA-Tn5, and the transposon formed by the Top2/Bottom double strand and Tn5 was pA-TN5-B.

### B. Preparation of cell-labeled microspheres

1) the sequence attached to the bead was as follows: Bead-S-S-PCR adapter-barcode1-linker1-barcode2-linker2-barcode3 -ligation linker
   where, the PCR adaptor sequence was ACACTCTTTCCCTACACGACGCTCTTCCGATCT (SEQ ID NO: 6), Linker1 was CGACTCACTACAGGG (SEQ ID NO: 7), the sequence of Linker2 was TCGGTGACACGATCG (SEQ ID NO: 8), the sequence of Ligation linker was ATCCACGTGCTTGAG (SEQ ID NO: 12). Barcode1=96 kinds of 5bp base sequences, Barcode2=96 kinds of 5bp base sequences, and Barcode1=96 kinds of 5bp base sequences.
2) Synthesis of 3x96 sequences
   1. PCR handle-96x barcode1-linker1, synthesizing 96 reverse complementary sequences of this sequence;
   2. linker1-96xbarcode2-linker2, synthesizing 96 reverse complementary sequences of this sequence;
   3. linker2-96xbarcode3-ligation linker, synthesizing 96 reverse complementary sequences of this sequence.
3) Synthesis of microspheres:
   The following amino sequences were synthesized: 5' amine-S-S-ACACTCTTTCCCTACACGACGCTCTTCCGATCT (SEQ ID NO: 6) and 30 µm of carboxyl modified microspheres (Zhiyi, KBsphere^{®}, www.kbspheres.com/productshow.asp?id=903).

Coupling reaction: microsphere +50 mM EDC+100 µM amino sequence (SEQ ID NO: 6), the amino sequence and the carboxyl microspheres were coupled to obtain the following structure: bead-S-S-ACACTCTTTCCCTACACGACGCTCTTCCGATCT (SEQ ID NO: 6)

### 4) Attachment of tags

The synthesized microspheres were evenly divided into a 96-well plate, and PCR handle-96xbarcode1-linker1 was added respectively for the first round of barcoding reactions. The reaction system and process were as follows: 10 µl microsphere + 2 µl BstI buffer + 1 µl 10 µm dNTP + 1 µl 100 µm PCR handle-96xbarcode1-linker1. Then they were kept at 95°C for 5 min and 60°C for 20 min; and then, 1 µl BstI+5 µl H₂O was added and kept at 60°C for 60 min.

After the first round of the barcoding reactions, all microspheres were collected, mixed, and reacted at 95c for 5 min to remove complementary chains, and washed to obtain the first round of microspheres barcoded (96xbarcode1-linker1). Then the microspheres were evenly divided into a 96-well plate, and linker 1-96xbarcode2-linker2 (the second round) and linker2-96x barcode 3-ligation linker (the third round) were added. The second and third rounds of barcoding reactions were carried out according to the system method of the first round, and single-stranded microspheres with triple barcodes were finally obtained. After being washed, the microspheres were annealed with the complementary sequence CGAATGCTCTGGCCTCTCAAGCACGTGGAT (SEQ ID NO: 9) to form a partial double-stranded structure, and the microspheres attached with the partial double-stranded structure as follows were finally obtained:

Bead-S-S-ACACTCTTTCCCTACACGACGCTCTTCCGATCT-barcode1-
CGACTCACTACAGGG-barcode2-TCGGTGACACGATCG-barcode3-ATCCACGTGCTTGAG
3'-
TAGGTGCACGAACTCTCCGGTCTCGTAAGC-5' (reverse arrangement of SEQ ID NO: 9)

### C. ATAC experiment

Human 293T cell lines were resuspended in the lysis solution (10 mM Tris-Cl, pH 7.4; 10 mM NaCl; 3 mM MgCl₂; and 0.01% NP-40) for lysing the cells, to obtain nuclei.

100,000 cell nuclei were taken and incubated with the target protein antibody, for example the antibody against histone H3K4me3 (the Abcam Company). The binding condition was as follows: 0.05% Digitonin, 20 mM HEPES, pH 7.5, 300 mM NaCl, 0.5 mM Spermidine, 1X Protease inhibitor (Roche) buffer, the antibody concentration 1 µg/100 µl, binding for 1 h at room temperature, or overnight at 4°C.

The sample was washed three times with 0.05% Digitonin, 20 mM HEPES, pH 7.5, 300 mM NaCl, 0.5 mM Spermidine, 1X Protease inhibitor (Roche) buffer.

1 µg/100 µl of the pA-Tn5 transposon complex was added to the sample with the buffer condition as above, and incubated at room temperature for 1 hr, and the sample was washed three times with this buffer.

MgCl₂ was added to the buffer until the magnesium ion concentration was 20 mM. The transposition reaction was carried out at 37c for 1 hr, during which pA-Tn5 would cut off the DNA adjacent to its binding position and insert the DNA sequence on it.

After the reaction, the nuclei were washed with PBS.

### D. High-throughput labeling

The microfluidic chip as shown in Fig. 8 was used for cell labeling, with Bead channel: 100 µm and Nuclei Channel: 50 µm.

The following solutions were prepared:
1 ml of a nucleus solution (100 nuclei/µl concentration), including: 200 µl 10xT4 DNA ligase Buffer, 10 µl T4 DNA ligase, 10 µl 1M DTT, and 780 µl nucleus/water.

A bead solution (100 beads/µl concentration): Beads in PBS.

Drops with a diameter of 120 µm were collected from the nucleus solution, bead solution and oil (FC40 fluorocarbon oil, containing 1% of the surfactant FluoroSurfactant, Ran Biotech) on the microfluidic chip, and linked at 37°C for 1 hour.

### E. Construction of library

Equal volume of perfluorooctanol fragmenting drops were added to the drops in step D, and centrifuged. The aqueous phase was pipetted, and the DNA in the aqueous phase was purified with the Qiagen DNA purification kit, and amplified with the following reaction system to obtain the final sequencing library: 36 µl DNA template, 10 µl 5xPCR Buffer, 1 µl 10 mM dNTP, 1 µl 10 µm primer TrueseqD501, 1 µl 10 µM primer N701, 1 µl Taq, 94°C for 2 min, 94°C for 30 sec, 55°C for 30 sec and 72°C for 30 sec, 18 cycles.
Sequence of primer TrueseqD501: AATGATACGGCGACCACCGAGATCTACACTCTTTCCCTACACGACGCTCTTCCGATCT (SEQ ID NO: 10)
Sequence of primer N701: CAAGCAGAAGACGGCATACGAGATATCGGCTAGTCTCGTGGGCTCGG (SEQ ID NO: 11)
100,000 PE150 reads were measured by Illumina Novaseq in each cell.

The above library comprised about 500 cells, and 100,000 PE150 reads were sequenced in each cell, with a total data volume of 15G.

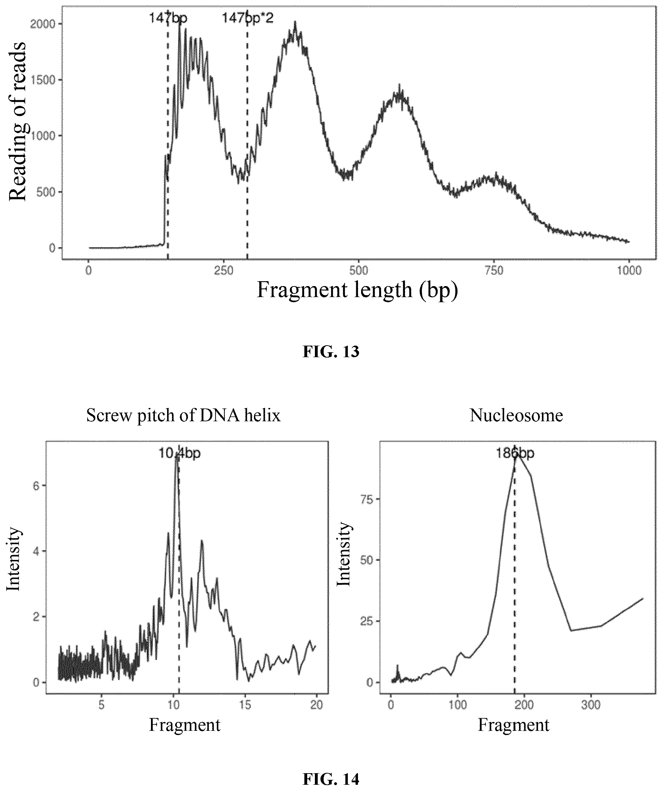

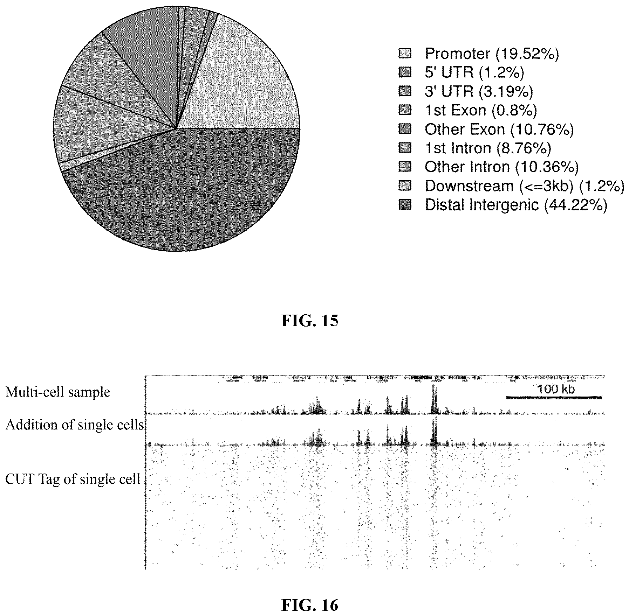

The antibody used was rabbit-anti-H3K4me3 of the Abcam company. Fig. 13 shows the fragment distribution results of the Cut tag library. Fig. 14 shows the diagram of distribution position results of the Cut tag fragments at transcription start sites. Fig. 15 shows the distribution ratio of the Cut tag fragments in the genome. Fig. 16 shows the distribution results of the Cut tag results of single cells. After superimposing the single-cell data, it presents a typical H3K4me3 histone modification distribution profile, which is highly similar to the experimental results of multi-cell samples, indicating the authenticity and accuracy of the single-cell data obtained by this method.

### Example 3 Detection of transcriptomes in cells or nuclei

(1) Preparation of reverse transcription primers. 5'-phosphorylated sequence complementary to nucleotide tag-UMI molecular counting sequence-polyT sequence. The nucleotide tag was prepared in the same way as in Example 1. RT primer AGGCCAGAGCATTCGNNNNNNNTTTTTTTTTTTTTTTTTTTTTTTTTTTTTT (SEQ ID NO: 13);
(2) Preparation of sample. Samples were non-fixed cells or nuclei, cells or nuclei fixed by formaldehyde (or other fixing agents), non-fixed or fixed tissue slices, etc.
   The fixed or non-fixed sample was treated with a buffer solution comprising a detergent (Triton, NP-40 Digitonin, etc.), which could include the intermediate step of lysing the cells (the non-fixed samples) to obtain the nuclei. The cells and nuclei were lysed and permeabilized by the detergent, so that molecular biological reagents such as enzymes could enter the cells and nuclei.
(3) Reverse transcription. A reverse transcriptase reaction system was provided by the reverse transcription primer in step (1) and a chain transformation template was added, to carry out intracellular reverse transcription reaction on the sample. After the reaction, the cells/nuclei still had an individual and intact morphology. The reaction system and condition were as follows: cells/tissues, a reverse transcriptase buffer, a RNase inhibitor, dNTP, a TSO chain conversion primer, a reverse transcription primer; 50-55°C, 5 minutes, 4°C + a reverse transcriptase, 42°C. Washing was carried out to remove the primer and enzyme system, and nucleotide tag ligation reactions on cells or tissues were carried out. After completion, primers were added to neutralize excess primers.
(4) Subsequent treatment of samples. Purification of mRNA/cDNA: For non-fixed tissues, mRNA/cDNA was purified directly, while for fixed tissues, mRNA/cDNA was purified after de-crosslinking; and PCR amplification of cDNA was carried out on mRNA/cDNA, to obtain a cDNA library, and the cDNA library was constructed into a sequencing library by Tn5 or other DNA interruption methods.

The specific steps were as follows:
Preparation of a nucleotide tag comprising a barcode sequence, which was fixed on a solid support. The step was the same as those in the above examples.

Preparation of a transposon complex, the step of which was the same as those in the above examples.

Preparation of samples. Nucleus: in 10 mM Tris-Cl, pH 7.4; 10 mM NaCl; 3 mM MgCl₂; and 0.01% NP-40 buffer, the tissues were homogenate, the cells were lysed, centrifuged at 500g for 5 min, resuspended once with a buffer, centrifuged at 500g for 5 min, and resuspended in the above buffer.

### Reverse transcription

The following reaction was set, and the final concentration of each component was as follows: 1000/µl nucleus, 1x RT Buffer, 1 µM dNTP, 1 µM reverse transcription primer above, 1 u/µl RNase inhibitor, 1 µM TSO primer with the primer sequence of (5'-AAGCAGTGGTATCAACGCAGAGTACATrGrGrG (SEQ ID NO: 14)-3', where the G on 3 end could be rG, and the rG represents riboguanine, and 1 unit/µl RT enzyme (Superscript II reverse transcriptase); reaction condition: 50°C for 5 min, 4°C for 5 min, 42°C for 60 min, the nuclei were washed with PBS, centrifuged and washed at 500g for 5 min twice to remove the unreacted enzymes and primers.

### High-throughput labeling

The microfluidic chip as shown in Fig. 8 was used for cell labeling, with Bead channel: 100 µm and Nuclei Channel: 50 µm.

The following solutions were prepared:
1 ml of a nucleus solution (100 nuclei/µl concentration), including: 200 µl 10xT4 DNA ligase Buffer, 10 µl T4 DNA ligase, 10 µl 1M DTT, and 780 µl nucleus/water.

A bead solution (100 beads/µl concentration): Beads in PBS.

Drops with a diameter of 120 µm were collected from the nucleus solution, bead solution and oil (FC40 fluorocarbon oil, containing 1% of the surfactant FluoroSurfactant, Ran Biotech) on the microfluidic chip, and linked at 37°C for 1 hour.

### Construction of library

Equal volume of perfluorooctanol fragmenting drops were added to the high-throughput labeled drops, and centrifuged. The aqueous phase was pipetted, and the cDNA/mRNA complex in the aqueous phase was purified with the Qiagen DNA purification kit.

DNA was amplified with the following reaction system to obtain the final sequencing library: 36 µl DNA template, 10 µl 5xPCR Buffer, 1 µl 10 mM dNTP, 1 µl 10 µm primer TrueseqD501, 1 µl 10 µM primer ISPCR (AAGCAGTGGTATCAACGCAGAGT (SEQ ID NO: 15)), 1 µl Taq, 94°C for 2 min, 94°C for 30 sec, 60°C for 30 sec and 72°C for 3 min, 18 cycles. The amplified cDNA was purified by AMPure XP magnetic beads at a volume of 1:1, and quantified by QuBit.

### Interruption of sequencing library

1 ng cDNA, 10 µl 2xTD Buffer (the Illumina Nextera kit), and 1 µl Nextera enzyme (Illumina Nextera), 20 µl reaction system, 55°C for 7 min. 5 µl Tn5 stop buffer (the Nextera kit) was added.

### Amplification of library

25 µl reaction system above, 1 µl 10 µM primer TrueseqD501, 1 µl 10 µM primer Nextera N701 primer, and 1 µl Taq enzyme. 72°C for 5 min, 94°C for 2 min, 94°C for 30 sec, 60°C for 30 sec and 72°C for 3 sec, 18 cycles. The library was purified by AMPure XP magnetic beads at a volume of 1:1.

100,000 PE150 reads were measured by Illumina Novaseq in each cell.

The above library comprised about 500 cells, and 100,000 PE150 reads were sequenced in each cell, with a total data volume of 15G.

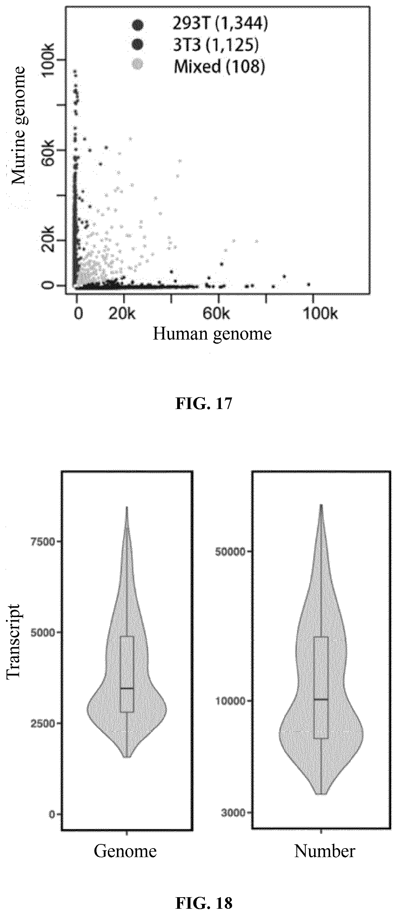

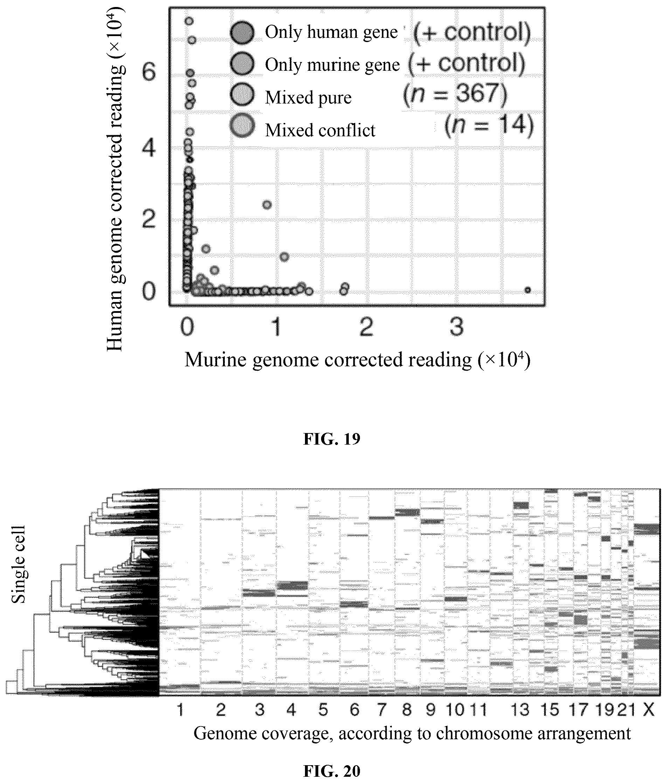

After two kinds of mixed cells, i.e., 293T (human) and 3T3 (murine), a single cell transcriptome experiment was carried out, and posted according to cell barcodes. Fig. 17 shows that the single cell results clearly distinguish the single cells of the two kinds of cells. Fig. 18 shows the distribution results of transcripts and gene numbers detected in each cell. The method of this application can be used for transcriptome detection of single cells. Similarly, the single cell genome experiment could be carried out by the method of this application after two kinds of mixed cells, i.e., 293T (human) and 3T3 (murine). According to the ratio of the measured sequences aligned to the human or mouse genome, Fig. 19 shows that the single cell results clearly distinguish the single cells of the two kinds of cells. Mixed pure means that the pure human or mouse source can be separated from the mixed cells, and only a few cells are in conflict with each other. Fig. 20 shows the genome coverages of single human cells according to chromosome arrangement, showing that single cell sequencing has different coverage in each cell and each genome site. The method of this application can be used for genome detection of single cells. The method of this application can also be used for distinguishing single cells of various cells in mixed cells by genome and transcriptome detection.

### Example 4 Detection of DNA sequence and number in cells

(1) The nucleotide tag was prepared in the same way as in Example 1. The only difference was that in 5' amine-S-S-ACACTCTTTCCCTACACGACGCTCTTCCGATCT (SEQ ID NO: 6), a sequence coupled with microspheres, all C bases were replaced by 5mC modifying bases.
(2) Preparation of sample. Sample: fixed single cells or nuclei. Sample treatment method: cells or nuclei were treated with a certain concentration of SDS and/or other detergents for a certain period of time under the heating condition, to remove the proteins bound to DNA, but not de-crosslink, so the DNA was still fixed in the cell structure.
(3) Transposition reaction. A Tn5 enzyme buffer comprising a divalent metal ion (e.g., a magnesium ion) was added to the above treated samples, and the assembled Tn5 enzyme was added to carry out the transposition reaction on genomes (37°C, 30 minutes-2 hours). System: cells/nuclei, a Tn5 buffer, and a Tn5 transposition complex, 37°C. After that, the samples were washed with the buffer to remove the unreacted Tn5 enzyme.
(4) Ligation reaction. A T4 DNA ligase reaction buffer, the nucleotide tags in ligation step (1), T4 DNA ligase, and the nucleotide tags were added to carry out a ligation reaction at a appropriate temperature (4°C-37°C) for more than 20 minutes.
   The reaction system included: the cells or nuclei or tissues (after the transposition reaction); T4 DNA ligase, the nucleotide tags and T4 DNA ligase. After the reaction, excessive amounts of the nucleotide tags in free form and sequences complementary to the nucleotide tags were added into the ligation reaction system to block the excess unreacted nucleotide tags.
(5) Obtaining DNA. A Proteinase K reaction buffer and Proteinase K were added, and DNA was purified after de-crosslinking at 55-65°C, thus obtaining labeled whole genome DNA. Subsequent treatments were as follows:
   1) DNA was directly sequenced to obtain whole genome sequence information, including copy number information (CNV) or point mutation information (SNV) of different regions of the genome.
   2) DNA was detected for 5mC, for example, by method such as Bisulfite conversion or NEB enzymatic conversion (NEB), or reduced bisulfite sequence based on MspI digestion, to detect 5mC information on the genome. When modifying C was transformed, the linking primer was designed to resist the transformed base or modifying base, thus ensuring amplification.
   3) DNA was detected for 5hmC by modifying the 5hmC sites with beta-galactose transferase, and 5hmC was detected by the downstream methods.
   4) Detection of other DNA modifying bases.

The specific steps were as follows:

### Sample treatment

The cells were fixed with 4% formaldehyde in 1xPBS at room temperature for 10 min, then a Glysine solution was added until the final concentration of 0.1M to terminate at room temperature for 5 min. The cells were washed with PBS twice, and centrifuged for 5 min at 500g. The fixed cells could be stored at-80°C or-20°C; the cells were thawed at room temperature, and 10 mM Tris 0.2% SDS solution was added to treat at 42°C for 10 min; PBS solution was used to wash 3 times; and 100,000 nuclei were taken and reacted with the obtained p-Tn5 and Tn5-B prepared in the ATAC experiment of the above examples. The reaction system was as follows:
25 µl 2xTD Buffer (Illumina) + 2.5 µl 10 µM p-Tn5 + 2.5 µl 10 µM Tn5-B + 20 µl nucleus (100,000). They were reacted at 37°C for 30 min, and the nuclei were washed with PBS.

### High-throughput labeling

The microfluidic chip as shown in Fig. 8 was used for cell labeling, with Bead channel: 100 µm and Nuclei Channel: 50 µm.

The following solutions were prepared:
1 ml of a nucleus solution (100 nuclei/µl concentration), including: 200 µl 10xT4 DNA ligase Buffer, 10 µl T4 DNA ligase, 10 µl 1M DTT, and 780 µl nucleus/water.

A bead solution (100 beads/µl concentration): Beads in PBS.

Drops with a diameter of 120 µm were collected from the nucleus solution, bead solution and oil (FC40 fluorocarbon oil, containing 1% of the surfactant FluoroSurfactant, Ran Biotech) on the microfluidic chip, and linked at 37°C for 1 hour.

### Construction of library for genome

Equal volume of perfluorooctanol fragmenting drops were added to the high-throughput labeled drops, and centrifuged. The aqueous phase was pipetted, the following reagents were added into the aqueous phase to the final concentration: 1% SDS, and Proteinease K 20 µg/ml, and reacted at 55°C for 2 hr; and the DNA in the aqueous phase was purified with the Qiagen DNA purification kit, and amplified with the following reaction system to obtain the final sequencing library: 36 µl DNA template, 10 µl 5xPCR Buffer, 1 µl 10 mM dNTP, 1 µl 10 µm primer TrueseqD501, 1 µl 10 µM primer N701, 1 µl Taq, 94°C for 2 min, 94°C for 30 sec, 55°C for 30 sec and 72°C for 30 sec, 18 cycles.

Sequence of primer TrueseqD501: AATGATACGGCGACCACCGAGATCTACACTCTTTCCCTACACGACGCTCTTCCGATCT (SEQ ID NO: 10)

Sequence of primer N701: CAAGCAGAAGACGGCATACGAGATATCGGCTAGTCTCGTGGGCTCGG (SEQ ID NO: 11)

100,000 PE150 reads were measured by Illumina Novaseq in each cell.

### Analysis of single cell genome

### 5mC methylation sequencing

With regards to the DNA obtained above, firstly, the genomic DNA obtained above was transformed with a transformation kit such as the EpiTect Fast Bisulfite Conversion Kit or NEB Enzymatic Methylation conversion kit. For example, taking the Qiagen kit as an example, the bisulfite conversion reagent was prepared according to the instructions.

The DNA above, 85 µl Bisulfite solution, 35 µl DNA protection Buffer, H₂O, with a total volume of 140 µl.

95°C for 5 min, 60°C for 10 min, 95°C for 5 min, 60°C for 10min and holding at 20°C. According to the steps in the instructions, the transformed DNA was purified by column.

### Amplification of DNA

The DNA was amplified with the following reaction system to obtain the final sequencing library: 36 µl DNA template, 10 µl 5xPCRBuffer, 1 µl 10 mM dNTP, 1 µl 10 µm primer TrueseqD501, 1 µl 10 µM primer N701, 1 µl Taq, 94°C for 2 min, 94°C for 30 sec, 55°C for 30 sec and 72°C for 30 sec, 18 cycles.

Sequence of primer TrueseqD501: AATGATACGGCGACCACCGAGATCTACACTCTTTCCCTACACGACGCTCTTCCGATCT (SEQ ID NO: 10)

Sequence of primer N701: CAAGCAGAAGACGGCATACGAGATATCGGCTAGTCTCGTGGGCTCGG (SEQ ID NO: 11)

100,000 PE150 reads were measured by Illumina Novaseq in each cell.

### Analysis of single cell methylation

### 5hmC methylation sequencing

The Thermo Fisher EpiJET 5-hmC Enrichment Kit was used to enrich the recovered DNA for 5hmc, and then the library was constructed for sequencing.

The DNA was recovered by 12.5 µL 4X Enzyme Reaction Buffer, and 10 µl 5-hmC Modifying Enzyme, water was added to 50 µl, and reacted at 30°C for 1 hr. DNA was purified using magnetic beads at 1:1 volume.

40 µL elution sample, 10 µl 10xbiotin conjugation buffer, and 50 µl biotin reagent, 50°C for 5min, 100 µl elution buffer was added to stop the reaction, and then the DNA was purified by column with a kit.

### Amplification of DNA

The DNA was amplified with the following reaction system to obtain the final sequencing library: 36 µl DNA template, 10 µl 5xPCRBuffer, 1 µl 10 mM dNTP, 1 µl 10 µm primer TrueseqD501, 1 µl 10 µM primer N701, 1 µl Taq, 94°C for 2 min, 94°C for 30 sec, 55°C for 30 sec and 72°C for 30 sec, 18 cycles.

Sequence of primer TrueseqD501: AATGATACGGCGACCACCGAGATCTACACTCTTTCCCTACACGACGCTCTTCCGATCT (SEQ ID NO: 10)

Sequence of primer N701: CAAGCAGAAGACGGCATACGAGATATCGGCTAGTCTCGTGGGCTCGG (SEQ ID NO: 11)

100,000 PE150 reads were measured by Illumina Novaseq in each cell.

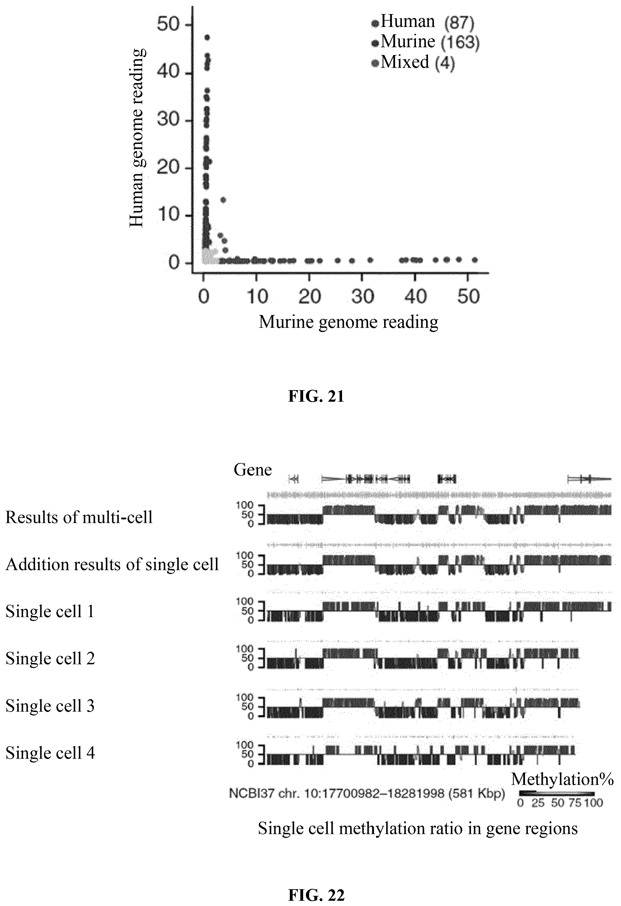

The single cell methylation experiment was carried out after two kinds of mixed cells, i.e., 293T (human) and 3T3 (murine). Fig. 21 shows that, according to the ratio of the measured sequences aligned to the human or mouse genome, the single cell results clearly distinguish the single cells of the two kinds of cells. Fig. 22 shows the distribution results of single cell methylation. After superimposing the single-cell data, it is highly similar to the experimental results of multi-cell samples, indicating the authenticity and accuracy of the single-cell methylation data obtained by this method.

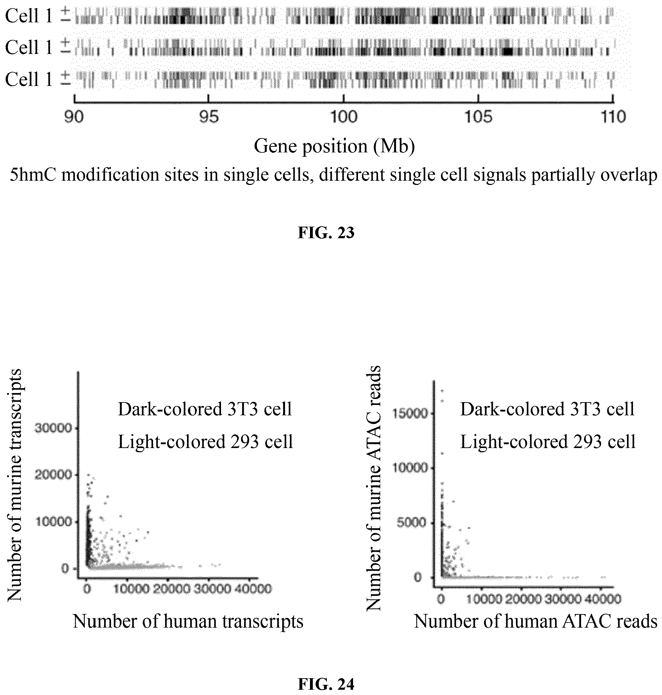

The method of this application can also be used for 5hmC sequencing of single cells. Fig. 23 shows the distribution results of 5hmC modification sites of single cells. The single cell 5hmC modification data obtained by this application are of authenticity and accuracy.

### Example 5 Simultaneous detection of transcriptomes and ATAC from the same cell

A dT primer and Tn5 enzyme containing the same 5' end linker were used. The nuclei were prepared, and then the cells were subjected to a RT (reverse transcription) reaction. After washing to remove the RT reaction system, a Tn5 ATAC reaction was carried out, and then mRNA and ATAC in the cells were labeled simultaneously. And then linking to the primers released on the microspheres was carried out. The ATAC DNA and RT mRNA/cDNA mixture were recovered.

For this mixture, a Tn5 library and cDNA library were amplified by general primers on linking adaptors and Tn5 and cDNA specific primers respectively, and the libraries were constructed for sequencing.

The specific steps were as follows:
Human 293T cell lines were resuspended in the lysis solution (10 mM Tris-Cl, pH 7.4; 10 mM NaCl; 3 mM MgCl₂; and 0.01% NP-40) for lysing the cells, to obtain nuclei.

100,000 of the nuclei were taken and reacted with p-Tn5 and Tn5-B obtained in examples of this application. The reaction system was as follows:
25 µl 2xTD Buffer (Illumina), 2.5 µl 10 µM p-Tn5, 2.5 µl 10 µM Tn5-B, and 20 µl nucleus (100,000). They were reacted at 37°C for 30 min, and the nuclei were washed with PBS.

The above obtained nuclei were subjected to the following RT reaction:
1000/µl nucleus, 1x RT Buffer, 1 µM dNTP, 1 µM reverse transcription primer above, 1 u/µl RNase inhibitor, 1 µM TSO primer with the primer sequence of (5'-AAGCAGTGGTATCAACGCAGAGTACATrGrGrG (SEQ ID NO: 14)-3', where the G on 3 end could be rG, and the rG represents riboguanine, and 1 unit/µl RT enzyme (Superscript II reverse transcriptase); reaction condition: 50°C for 5 min, 4°C for 5 min, 42°C for 60 min, the nuclei were washed with PBS, centrifuged and washed at 500g for 5 min twice to remove the unreacted enzymes and primers.

### High-throughput labeling

The microfluidic chip as shown in Fig. 8 was used for cell labeling, with Bead channel: 100 µm and Nuclei Channel: 50 µm.

The following solutions were prepared:
1 ml of a nucleus solution (100 nuclei/µl concentration), including: 200 µl 10xT4 DNA ligase Buffer, 10 µl T4 DNA ligase, 10 µl 1M DTT, and 780 µl nucleus/water.

A bead solution (100 beads/µl concentration): Beads in PBS.

Drops with a diameter of 120 µm were collected from the nucleus solution, bead solution and oil (FC40 fluorocarbon oil, containing 1% of the surfactant FluoroSurfactant, Ran Biotech) on the microfluidic chip, and linked at 37°C for 1 hour.

### Construction of library

Equal volume of perfluorooctanol fragmenting drops were added to the high-throughput labeled drops, and centrifuged. The aqueous phase was pipetted, and the ATAC DNA and mRNA/cDNA in the aqueous phase were purified with the Qiagen DNA purification kit.

The library was amplified, and ATAC DNA and mRNA/cDNA were amplified simultaneously

The DNA was amplified with the following reaction system to obtain the final sequencing library: 36 µl DNA template, 10 µl 5xPCRBuffer, 1 µl 10 mM dNTP, 1 µl 10 µm primer TrueseqD501, 1 µl 10 µM primer N701, 1 µl 10 µM ISPCR primer, 1 µl Taq, 72°C for 5 min, 94°C for 2 min, 94°C for 30 sec, 55°C for 30 sec, and 72°C 3 min, 12 cycles.

Sequence of primer TrueseqD501: AATGATACGGCGACCACCGAGATCTACACTCTTTCCCTACACGACGCTCTTCCGATCT (SEQ ID NO: 10)

Sequence of primer N701: CAAGCAGAAGACGGCATACGAGATATCGGCTAGTCTCGTGGGCTCGG (SEQ ID NO: 11)

ISPCR primer: AAGCAGTGGTATCAACGCAGAGT (SEQ ID NO: 15)

The above mixed libraries were purified by AMPure beads at 1:1, and quantified.

### Amplification of ATAC library

1 ng the above DNA, a DNA template, 10 µl 5xPCR Buffer, 1 µl 10 mM dNTP, 1 µl 10 µm primer TrueseqD501, 1 µl 10 µM primer N701, 1 µl Taq, 94°C for 2 min, 94°C for 30 sec, 55°C for 30 sec and 72°C for 30 sec, 18 cycles. The library was purified by AMPure at 1:1, quantified and sequenced.

### Amplification of cDNA and Construction of sequencing library

1 ng the above DNA, a DNA template, 10 µl 5xPCR Buffer, 1 µl 10 mM dNTP, 1 µl 10 µm primer TrueseqD501, 1 µl 10 µM ISPCR primer, and 1 µl Taq, 94°C for 2 min, 94°C for 30 sec, 55°C for 30 sec and 72°C for 30 sec, 18 cycles. The library was purified by AMPure at 1:1, quantified and sequenced.

### Interruption of sequencing library

1 ng cDNA, 10 µl 2xTD Buffer (the Illumina Nextera kit), and 1 µl Nextera enzyme (Illumina Nextera), 20 µl reaction system, 55°C for 7 min. 5 µl Tn5 stop buffer (the Nextera kit) was added.

### Amplification of library

25 µl reaction system above, 1 µl 10 µM primer TrueseqD501, 1 µl 10 µM primer Nextera N701 primer, 1 µl Taq enzyme, 72°C for 5 min, 94°C for 2 min, 94°C for 30 sec, 60°C for 30 sec, and 72°C for 3 sec, 18 cycles.

The library was purified by AMPure XP magnetic beads at a volume of 1: 1.

100,000 PE150 reads were measured by Illumina Novaseq in each cell.

Transcriptomes and ATAC were analyzed simultaneously.

The present application is used to detect the transcriptomes and ATAC of the same cell simultaneously. Fig. 24 shows that, according to both transcriptomes and ATAC genomes, single cells in two kinds of cells can be well distinguished. The method of this application can be used to accurately detecting transcriptomes and ATAC of the same cell simultaneously.

### Example 6 Simultaneous detection of transcriptomes and CUT&Tag from the same cell

The specific steps were as follows:
Human 293T cell lines were resuspended in the lysis solution (10 mM Tris-Cl, pH 7.4; 10 mM NaCl; 3 mM MgCl₂; and 0.01% NP-40) for lysing the cells, to obtain nuclei.

100,000 cell nuclei were taken and incubated with the target protein antibody, for example the antibody against histone H3K4me3 (the Abcam Company). The binding condition was as follows: 0.05% Digitonin, 20 mM HEPES, pH 7.5, 300 mM NaCl, 0.5 mM Spermidine, 1X Protease inhibitor (Roche) buffer, the antibody concentration 1 µg/100 µl, binding for 1 h at room temperature, or overnight at 4°C.

The sample was washed three times with 0.05% Digitonin, 20 mM HEPES, pH 7.5, 300 mM NaCl, 0.5 mM Spermidine, 1X Protease inhibitor (Roche) buffer.

1 µg/100 µl of the pA-Tn5 transposon complex was added to the sample with the buffer condition as above, and incubated at room temperature for 1 hr, and the sample was washed three times with this buffer.

MgCl₂ was added to the buffer until the magnesium ion concentration was 20 mM. The transposition reaction was carried out at 37°C for 1 hr, during which pA-Tn5 would cut off the DNA adjacent to its binding position and insert the DNA sequence on it.

After the reaction, the nuclei were washed with PBS.

The above obtained nuclei were subjected to the following RT reaction, and the final concentration of each component was as follows: 1000/µl nucleus, 1x RT Buffer, 1 µM dNTP, 1 µM reverse transcription primer above, 1 u/µl RNase inhibitor, 1 µM TSO primer with the primer sequence of (5'-AAGCAGTGGTATCAACGCAGAGTACATrGrGrG (SEQ ID NO: 14)-3', where the G on 3 end could be rG, and the rG represents riboguanine, and 1 unit/µl RT enzyme (Superscript II reverse transcriptase); reaction condition: 50°C for 5 min, 4°C for 5 min, 42°C for 60 min, the nuclei were washed with PBS, centrifuged and washed at 500g for 5 min twice to remove the unreacted enzymes and primers.

### High-throughput labeling

The microfluidic chip as shown in Fig. 8 was used for cell labeling, with Bead channel: 100 µm and Nuclei Channel: 50 µm.

The following solutions were prepared:
1 ml of a nucleus solution (100 nuclei/µl concentration), including: 200 µl 10xT4 DNA ligase Buffer, 10 µl T4 DNA ligase, 10 µl 1M DTT, and 780 µl nucleus/water.

A bead solution (100 beads/µl concentration): Beads in PBS.

Drops with a diameter of 120 µm were collected from the nucleus solution, bead solution and oil (FC40 fluorocarbon oil, containing 1% of the surfactant FluoroSurfactant, Ran Biotech) on the microfluidic chip, and linked at 37°C for 1 hour.

### Construction of library

Equal volume of perfluorooctanol fragmenting drops were added to the high-throughput labeled drops, and centrifuged. The aqueous phase was pipetted, and the ATAC DNA and mRNA/cDNA in the aqueous phase were purified with the Qiagen DNA purification kit

The library was amplified, and ATAC DNA and mRNA/cDNA were amplified simultaneously

The DNA was amplified with the following reaction system to obtain the final sequencing library: 36 µl DNA template, 10 µl 5xPCRBuffer, 1 µl 10 mM dNTP, 1 µl 10 µm primer TrueseqD501, 1 µl 10 µM primer N701, 1 µl 10 µM ISPCR primer, 1 µl Taq, 72°C for 5 min, 94°C for 2 min, 94°C for 30 sec, 55°C for 30 sec, and 72°C 3 min, 12 cycles.

Sequence of primer TrueseqD501: AATGATACGGCGACCACCGAGATCTACACTCTTTCCCTACACGACGCTCTTCCGATCT (SEQ ID NO: 10)

Sequence of primer N701: CAAGCAGAAGACGGCATACGAGATATCGGCTAGTCTCGTGGGCTCGG (SEQ ID NO: 11)

ISPCR primer: AAGCAGTGGTATCAACGCAGAGT (SEQ ID NO: 15)

The above mixed libraries were purified by AMPure beads at 1:1, and quantified.

### Amplification of CUT Tag library

### 1 ng the above DNA

A DNA template, 10 µl 5xPCR Buffer, 1 µl 10 mM dNTP, 1 µl 10 µm primer TrueseqD501, 1 µl 10 µM primer N701, 1 µl Taq, 94°C for 2 min, 94°C for 30 sec, 55°C for 30 sec and 72°C for 30 sec, 18 cycles

The library was purified by AMPure at 1:1, quantified and sequenced.

### Amplification of cDNA and Construction of sequencing library

1 ng the above DNA, a DNA template, 10 µl 5xPCR Buffer, 1 µl 10 mM dNTP, 1 µl 10 µm primer TrueseqD501, 1 µl 10 µM ISPCR primer, and 1 µl Taq, 94°C for 2 min, 94°C for 30 sec, 55°C for 30 sec and 72°C for 30 sec, 18 cycles.

The library was purified by AMPure at 1:1, quantified and sequenced.

### Interruption of sequencing library

1 ng cDNA, 10 µl 2xTD Buffer (the Illumina Nextera kit), and 1 µl Nextera enzyme (Illumina Nextera), 20 µl reaction system, 55°C for 7 min. 5 µl Tn5 stop buffer (the Nextera kit) was added.

### Amplification of library

25 µl reaction system above, 1 µl 10 µM primer TrueseqD501, 1 µl 10 µM primer Nextera N701 primer, 1 µl Taq enzyme, 72°C for 5 min, 94°C for 2 min, 94°C for 30 sec, 60°C for 30 sec, and 72°C for 3 sec, 18 cycles.

The library was purified by AMPure XP magnetic beads at a volume of 1: 1.

100,000 PE150 reads were measured by Illumina Novaseq in each cell.

Transcriptomes and cut tags were analyzed simultaneously.

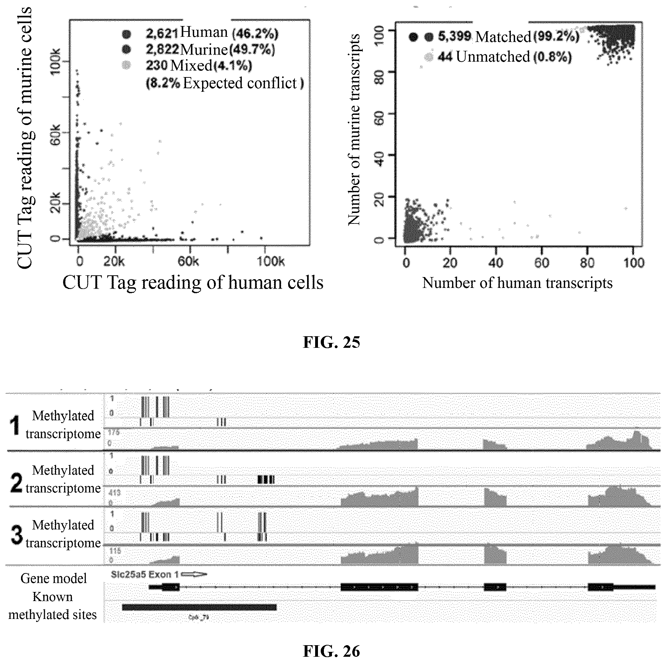

The present application is used to detect the transcriptomes and cut tags of the same cell simultaneously. Fig. 25 shows that, according to both transcriptomes and cut tag sets, single cells in two kinds of cells can be well distinguished. The method of this application can be used to accurately detecting transcriptomes and cut tags of the same cell simultaneously.

### Example 7 Simultaneous detection of transcriptomes and genomes from the same cell

The samples were treated the same as detecting the genomic DNA. Firstly, the nuclei were stripped, and then subjected to a Tn5 transposition reaction, followed by a RT (reverse transcription) reaction, and then treated as in Example 5.

The specific steps were as follows:

### Sample treatment

The cells were fixed with 4% formaldehyde in 1xPBS at room temperature for 10 min, then a Glysine solution was added until the final concentration of 0.1M to terminate at room temperature for 5 min. The cells were washed with PBS twice, and centrifuged for 5 min at 500g. The fixed cells could be stored at-80°C or-20°C; the cells were thawed at room temperature, and 10 mM Tris 0.2% SDS solution was added to treat at 42°C for 10 min; PBS solution was used to wash 3 times; and 100,000 nuclei were taken and reacted with the obtained p-Tn5 and Tn5-B prepared in the ATAC experiment of the above examples. The reaction system was as follows:

25 µl 2xTD Buffer (Illumina) + 2.5 µl 10 µM p-Tn5 + 2.5 µl 10 µM Tn5-B + 20 µl nucleus (100,000). They were reacted at 37°C for 30 min, and the nuclei were washed with PBS.

The above obtained nuclei were subjected to the following RT reaction:
1000/µl nucleus, 1x RT Buffer, 1 µM dNTP, 1 µM reverse transcription primer above, 1 u/µl RNase inhibitor, 1 µM TSO primer with the primer sequence of (5'-AAGCAGTGGTATCAACGCAGAGTACATrGrGrG (SEQ ID NO: 14)-3', where the G on 3 end could be rG, and the rG represents riboguanine, and 1 unit/µl RT enzyme (Superscript II reverse transcriptase); reaction condition: 50°C for 5 min, 4°C for 5 min, 42°C for 60 min, the nuclei were washed with PBS, centrifuged and washed at 500g for 5 min twice to remove the unreacted enzymes and primers.

### High-throughput labeling

The microfluidic chip as shown in Fig. 8 was used for cell labeling, with Bead channel: 100 µm and Nuclei Channel: 50 µm.

The following solutions were prepared:
1 ml of a nucleus solution (100 nuclei/µl concentration), including: 200 µl 10xT4 DNA ligase Buffer, 10 µl T4 DNA ligase, 10 µl 1M DTT, and 780 µl nucleus/water.

A bead solution (100 beads/µl concentration): Beads in PBS.

Drops with a diameter of 120 µm were collected from the nucleus solution, bead solution and oil (FC40 fluorocarbon oil, containing 1% of the surfactant FluoroSurfactant, Ran Biotech) on the microfluidic chip, and linked at 37°C for 1 hour.

### Construction of library

Equal volume of perfluorooctanol fragmenting drops were added to the high-throughput labeled drops, and centrifuged. The aqueous phase was pipetted, and the DNA was obtained. A Proteinase K reaction buffer and Proteinase K were added, and DNA was purified after de-crosslinking at 55-65°C, thus obtaining labeled whole genome DNA. Subsequent treatments were as follows:

The Genomic DNA and mRNA/cDNA in the aqueous phase were purified with the Qiagen DNA purification kit.

The library was amplified, and ATAC DNA and mRNA/cDNA were amplified simultaneously

The DNA was amplified with the following reaction system to obtain the final sequencing library: 36 µl DNA template, 10 µl 5xPCRBuffer, 1 µl 10 mM dNTP, 1 µl 10 µm primer TrueseqD501, 1 µl 10 µM primer N701, 1 µl 10 µM ISPCR primer, 1 µl Taq, 72°C for 5 min, 94°C for 2 min, 94°C for 30 sec, 55°C for 30 sec, and 72°C 3 min, 12 cycles.

Sequence of primer TrueseqD501: AATGATACGGCGACCACCGAGATCTACACTCTTTCCCTACACGACGCTCTTCCGATCT (SEQ ID NO: 10)

Sequence of primer N701: CAAGCAGAAGACGGCATACGAGATATCGGCTAGTCTCGTGGGCTCGG (SEQ ID NO: 11)

ISPCR primer: AAGCAGTGGTATCAACGCAGAGT (SEQ ID NO: 15)

The above mixed libraries were purified by AMPure beads at 1:1, and quantified.

### Amplification of genome library

1 ng the above DNA, a DNA template, 10 µl 5xPCR Buffer, 1 µl 10 mM dNTP, 1 µl 10 µm primer TrueseqD501, 1 µl 10 µM primer N701, 1 µl Taq, 94°C for 2 min, 94°C for 30 sec, 55°C for 30 sec and 72°C for 30 sec, 18 cycles. The library was purified by AMPure at 1:1, quantified and sequenced.

### Amplification of cDNA and Construction of sequencing library

1 ng the above DNA, a DNA template, 10 µl 5xPCR Buffer, 1 µl 10 mM dNTP, 1 µl 10 µm primer TrueseqD501, 1 µl 10 µM ISPCR primer, and 1 µl Taq, 94°C for 2 min, 94°C for 30 sec, 55°C for 30 sec and 72°C for 30 sec, 18 cycles. The library was purified by AMPure at 1:1, quantified and sequenced.

### Interruption of sequencing library

1 ng cDNA, 10 µl 2xTD Buffer (the Illumina Nextera kit), and 1 µl Nextera enzyme (Illumina Nextera), 20 µl reaction system, 55°C for 7 min. 5 µl Tn5 stop buffer (the Nextera kit) was added.

### Amplification of library

25 µl reaction system above, 1 µl 10 µM primer TrueseqD501, 1 µl 10 µM primer Nextera N701 primer, 1 µl Taq enzyme, 72°C for 5 min, 94°C for 2 min, 94°C for 30 sec, 60°C for 30 sec, and 72°C for 3 sec, 18 cycles.

The library was purified by AMPure XP magnetic beads at a volume of 1:1.

100,000 PE150 reads were measured by Illumina Novaseq in each cell.

Transcriptomes and genomes were analyzed simultaneously

The present application is used to detect the transcriptomes and genomes of the same cell simultaneously, and single cells in two kinds of cells can be well distinguished. It can accurately detect transcriptomes and cut tags of the same cell simultaneously.

### Example 8 Simultaneous detection of transcriptomes and DNA modifications from the same cell

The specific steps were as follows:

### Sample treatment

The cells were fixed with 4% formaldehyde in 1xPBS at room temperature for 10 min, then a Glysine solution was added until the final concentration of 0.1M to terminate at room temperature for 5 min. The cells were washed with PBS twice, and centrifuged for 5 min at 500g. The fixed cells could be stored at-80°C or-20°C; the cells were thawed at room temperature, and 10 mM Tris 0.2% SDS solution was added to treat at 42°C for 10 min; PBS solution was used to wash 3 times; and 100,000 nuclei were taken and reacted with the obtained p-Tn5 and Tn5-B prepared in the ATAC experiment of the above examples. The reaction system was as follows:
25 µl 2xTD Buffer (Illumina) + 2.5 µl 10 µM p-Tn5 + 2.5 µl 10 µM Tn5-B + 20 µl nucleus (100,000). They were reacted at 37°C for 30 min, and the nuclei were washed with PBS.

The above obtained nuclei were subjected to the following RT reaction:
1000/µl nucleus, 1x RT Buffer, 1 µM dNTP, 1 µM reverse transcription primer above, 1 u/µl RNase inhibitor, 1 µM TSO primer with the primer sequence of (5'-AAGCAGTGGTATCAACGCAGAGTACATrGrGrG (SEQ ID NO: 14)-3', where the G on 3 end could be rG, and the rG represents riboguanine, and 1 unit/µl RT enzyme (Superscript II reverse transcriptase); reaction condition: 50°C for 5 min, 4°C for 5 min, 42°C for 60 min, the nuclei were washed with PBS, centrifuged and washed at 500g for 5 min twice to remove the unreacted enzymes and primers.

### High-throughput labeling

The microfluidic chip as shown in Fig. 8 was used for cell labeling, with Bead channel: 100 µm and Nuclei Channel: 50 µm.

The following solutions were prepared:
1 ml of a nucleus solution (100 nuclei/µl concentration), including: 200 µl 10xT4 DNA ligase Buffer, 10 µl T4 DNA ligase, 10 µl 1M DTT, and 780 µl nucleus/water.

A bead solution (100 beads/µl concentration): Beads in PBS.

Drops with a diameter of 120 µm were collected from the nucleus solution, bead solution and oil (FC40 fluorocarbon oil, containing 1% of the surfactant FluoroSurfactant, Ran Biotech) on the microfluidic chip, and linked at 37°C for 1 hour.

### Construction of library

Equal volume of perfluorooctanol fragmenting drops were added to the high-throughput labeled drops, and centrifuged. The aqueous phase was pipetted, and the DNA was obtained. A Proteinase K reaction buffer and Proteinase K were added, the de-crosslinking was carried out at 55-65°C.
The DNA and mRNA/cDNA in the aqueous phase were purified with the Qiagen DNA purification kit
The above library was divided into two parts, which were used for methylation sequencing and transcriptome sequencing, respectively

For the methylation library, either Bislufite sequencing or 5hmc sequencing was carried out

### Methylation sequencing

With regards to the DNA obtained above, firstly, the genomic DNA obtained above was transformed with a transformation kit such as the EpiTect Fast Bisulfite Conversion Kit or NEB Enzymatic Methylation conversion kit. For example, taking the Qiagen kit as an example, the bisulfite conversion reagent was prepared according to the instructions.

The DNA above, 85 µl Bisulfite solution, 35 µl DNA protection Buffer, H₂O, with a total volume of 140 µl.

95°C for 5 min, 60°C for 10 min, 95°C for 5 min, 60°C for 10min and holding at 20°C. According to the steps in the instructions, the transformed DNA was purified by column.

### Amplification of DNA

The DNA was amplified with the following reaction system to obtain the final sequencing library: 36 µl DNA template, 10 µl 5xPCRBuffer, 1 µl 10 mM dNTP, 1 µl 10 µm primer TrueseqD501, 1 µl 10 µM primer N701, 1 µl Taq, 94°C for 2 min, 94°C for 30 sec, 55°C for 30 sec and 72°C for 30 sec, 18 cycles.

Sequence of primer TrueseqD501: AATGATACGGCGACCACCGAGATCTACACTCTTTCCCTACACGACGCTCTTCCGATCT (SEQ ID NO: 10)

Sequence of primer N701: CAAGCAGAAGACGGCATACGAGATATCGGCTAGTCTCGTGGGCTCGG (SEQ ID NO: 11)

100,000 PE150 reads were measured by Illumina Novaseq in each cell.

With regards to the 5hmC methylation sequencing, the Thermo Fisher EpiJET 5-hmC Enrichment Kit was used to enrich the recovered DNA for 5hmc, and then the library was constructed for sequencing.

The DNA was recovered by 12.5 µL 4X Enzyme Reaction Buffer, and 10 µl 5-hmC Modifying Enzyme, water was added to 50 µl, and reacted at 30°C for 1 hr. DNA was purified using magnetic beads at 1:1 volume.

40 µL elution sample, 10 µl 10xbiotin conjugation buffer, and 50 µl biotin reagent, 50°C for 5min, 100 µl elution buffer was added to stop the reaction, and then the DNA was purified by column with a kit.

### Amplification of DNA

The DNA was amplified with the following reaction system to obtain the final sequencing library: 36 µl DNA template, 10 µl 5xPCRBuffer, 1 µl 10 mM dNTP, 1 µl 10 µm primer TrueseqD501, 1 µl 10 µM primer N701, 1 µl Taq, 94°C for 2 min, 94°C for 30 sec, 55°C for 30 sec and 72°C for 30 sec, 18 cycles.

Sequence of primer TrueseqD501: AATGATACGGCGACCACCGAGATCTACACTCTTTCCCTACACGACGCTCTTCCGATCT (SEQ ID NO: 10)

Sequence of primer N701: CAAGCAGAAGACGGCATACGAGATATCGGCTAGTCTCGTGGGCTCGG (SEQ ID NO: 11)

100,000 PE150 reads were measured by Illumina Novaseq in each cell.

### Amplification of DNA

The DNA was amplified with the following reaction system to obtain the final sequencing library: 36 µl DNA template, 10 µl 5xPCRBuffer, 1 µl 10 mM dNTP, 1 µl 10 µm primer TrueseqD501, 1 µl 10 µM primer N701, 1 µl Taq, 94°C for 2 min, 94°C for 30 sec, 55°C for 30 sec and 72°C for 30 sec, 18 cycles.

Sequence of primer TrueseqD501: AATGATACGGCGACCACCGAGATCTACACTCTTTCCCTACACGACGCTCTTCCGATCT (SEQ ID NO: 10)

Sequence of primer N701: CAAGCAGAAGACGGCATACGAGATATCGGCTAGTCTCGTGGGCTCGG (SEQ ID NO: 11)

100,000 PE150 reads were measured by Illumina Novaseq in each cell.

### Amplification of cDNA and Construction of sequencing library

The DNA and cDNA/mRNA recovered above, a DNA template, 10 µl 5xPCR Buffer, 1 µl 10 mM dNTP, 1 µl 10 µm primer TrueseqD501, 1 µl 10 µM ISPCR primer, and 1 µl Taq, 94°C for 2 min, 94°C for 30 sec, 55°C for 30 sec and 72°C for 30 sec, 18 cycles.

The library was purified by AMPure at 1:1, quantified and sequenced.

### Interruption of sequencing library

1 ng cDNA, 10 µl 2xTD Buffer (the Illumina Nextera kit), and 1 µl Nextera enzyme (Illumina Nextera), 20 µl reaction system, 55°C for 7 min. 5 µl Tn5 stop buffer (the Nextera kit) was added.

### Amplification of library

25 µl reaction system above, 1 µl 10 µM primer TrueseqD501, 1 µl 10 µM primer Nextera N701 primer, 1 µl Taq enzyme, 72°C for 5 min, 94°C for 2 min, 94°C for 30 sec, 60°C for 30 sec, and 72°C for 3 sec, 18 cycles.

The library was purified by AMPure XP magnetic beads at a volume of 1: 1.

100,000 PE150 reads were measured by Illumina Novaseq in each cell.

Transcriptomes and methylations were analyzed simultaneously.

The present application is used to detect the transcriptomes and methylationss of the same cell simultaneously. Fig. 26 shows that, the transcriptomes and methylation set of the same cell can be well matched with the gene models and known methylation sites. The method of this application can be used to accurately detecting transcriptomes and methylations of the same cell simultaneously.

### Example 9 Technology platform of spatial multi-omics

(1) Spatial lattice chip: DNA oligo clusters with fixed intervals were on the chip, and the structure of the chip was as follows:
   Slide-Surface-releasable linker-PCR adaptor-barcode-linking arm

The chip hybridized with a complementary single chain to change the oligo lattice into the following structure:
Slide-Surface-releasable linker-PCR adaptor-barcode-linking arm
linking arm-complementary chain

The spatial lattice was synthesized by microarray in-situ synthesis method (Affymetrix, NimbleGene) or other methods, including transferring from the existing array by PCR, extending by sequential labeling, etc.

(2) Preparation of tissue slices: the frozen slices of non-fixed tissues were sticked onto cover glasses, 1% formaldehyde was added, and the tissues were fixed, and washed.

(3) Permeabilization treatment: the tissues were treated with a buffer containing a detergent.

(4) The reverse transcription reaction mix was added onto the tissues, and a reverse transcription primer with 5' phosphate modification and 5' extension which can be complementary to the oligo on the chip was used for in-situ RT (reverse transcription) reaction.

(5) The reverse transcription reaction system was washed off, a Tn5 enzyme with 5' phosphate modification was added on the slide to carry out an in-situ ATAC reaction.

(6) The ATAC reaction system was washed off, a DNA ligase buffer and DNA ligase were added onto the tissues, and then the tissues were sticked onto the DNA oligo lattice to make the two closely contact. The DNA oligos were released from the slide, and transferred to the tissue slices for ligation reaction, and the cDNA and Tn5 products were labeled.

(7) After the reaction, the tissues were imaged.

(8) The reaction was terminated, the tissues were digested with a protease, the DNA was recovered, and the cDNA and ATAC DNA libraries were constructed for sequencing in the manner of the previous embodiments.

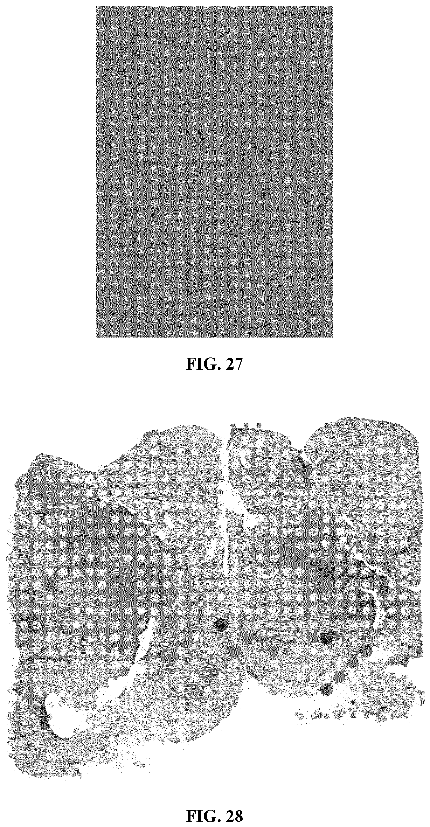

The specific steps were as follows:
A 100x100 primer lattice with a size of 5 µm and a spacing of 5 µm was synthesized on a glass/silicon mechanism using the technology of the Affymetrix company, with a total area of 1 cm x 1 cm, and a total of 10,000 DNA oligo lattices. Figure 27 shows a spatial lattice chip, in which the DNA lattices can have regularly arranged dT primer arrays, and hybridized with the FAM-AAAAAAAAAAAAAAAAAAAAAAAA (SEQ ID NO: 17) primer. The specific DNA sequence of the lattice was:
S-S-ACACTCTTTCCCTACACGACGCTCT (SEQ ID NO: 16)-NNNNNNNN-ATCCACGTGCTTGAG (SEQ ID NO: 12)

The NNNNNNNN in the DNA sequence of the lattice sequence was a specific primer sequence of 8bp, and each dot on the lattice corresponded to a specific 8bp sequence.

The CGAATGCTCTGGCCTCTCAAGCACGTGGAT (SEQ ID NO: 9) primer was added onto the above slide, and hybridized at room temperature with the glass for 1 hr in 1 M NaCl and 10 mM Tris solution, so that the primers on the lattice were annealed into partial double-stranded primers.

The OCT-embedded tissues were sliced with a freezing slicer, and affixed to polylysine surface-treated slides.

The tissues were fixed with 1% formaldehyde at room temperature for 10 minutes, and the slides were washed with PBS.

The tissues on the slides were treated with a lysis solution (10 mM Tris-Cl, pH 7.4; 10 mM NaCl; 3 mM MgCl₂; 0.01% NP-40) at room temperature for 5 min.

p-Tn5 and Tn5-B obtained in examples of this application were used to react with the slides. The reaction system was as follows:

25 µl 2xTD Buffer (Illumina), 2.5 µl 10 µM p-Tn5, 2.5 µl 10 µM Tn5-B, and 20 µl nucleus (100,000). They were reacted at 37°C for 30 min, and the slices were washed with PBS.

A RT reaction was carried out on the slices

1000/µl nucleus, 1x RT Buffer, 1 µM dNTP, 1 µM reverse transcription primer above, 1 u/µl RNase inhibitor, 1 µM TSO primer with the primer sequence of (5'-AAGCAGTGGTATCAACGCAGAGTACATrGrGrG (SEQ ID NO: 14)-3', where the G on 3 end could be rG, and the rG represents riboguanine, and 1 unit/µl RT enzyme (Superscript II reverse transcriptase); reaction condition: 50°C for 5 min, 4°C for 5 min, 42°C for 60 min, and the slices were washed with PBS.

The reacted tissues were contacted with the synthesized primer lattice slide, and 1xT4 ligase buffer and 1 unit/µl T4 DNA ligase were added, so that the adaptors with a partial double-stranded form on the slides were subjected to a ligation reaction with the RT products and AATC products on the tissue slices.

The cDNA and ATACDNA were recovered. A Proteinase K reaction buffer and Proteinase K were added onto the slices, and DNA was purified after de-crosslinking at 55-65°C. Then the genomic DNA and reverse transcript mRNA/cDNA were obtained by purification with the Qiagen kit

The ATAC DNA and mRNA/cDNA in the aqueous phase were purified with the Qiagen DNA purification kit.

The library was amplified, and ATAC DNA and mRNA/cDNA were amplified simultaneously

The DNA and cDNA were amplified with the following reaction system to obtain the final sequencing library: 36 µl DNA template, 10 µl 5xPCR Buffer, 1 µl 10 mM dNTP, 1 µl 10 µm primer TrueseqD501, 1 µl 10 µM primer N701, 1 µl 10 µM ISPCR primer, 1 µl Taq, 72°C for 5 min, 94°C for 2 min, 94°C for 30 sec, 55°C for 30 sec, and 72°C 3 min, 12 cycles.

Sequence of primer TrueseqD501: AATGATACGGCGACCACCGAGATCTACACTCTTTCCCTACACGACGCTCTTCCGATCT (SEQ ID NO: 10)

Sequence of primer N701: CAAGCAGAAGACGGCATACGAGATATCGGCTAGTCTCGTGGGCTCGG (SEQ ID NO: 11)

ISPCR primer: AAGCAGTGGTATCAACGCAGAGT (SEQ ID NO: 15)

The above mixed libraries were purified by AMPure beads at 1:1, and quantified.

### Amplification of ATAC library

1 ng the above DNA, a DNA template, 10 µl 5xPCR Buffer, 1 µl 10 mM dNTP, 1 µl 10 µm primer TrueseqD501, 1 µl 10 µM primer N701, 1 µl Taq, 94°C for 2 min, 94°C for 30 sec, 55°C for 30 sec and 72°C for 30 sec, 18 cycles. The library was purified by AMPure at 1:1, quantified and sequenced.

### Amplification of cDNA and Construction of sequencing library

1 ng the above DNA, a DNA template, 10 µl 5xPCR Buffer, 1 µl 10 mM dNTP, 1 µl 10 µm primer TrueseqD501, 1 µl 10 µM ISPCR primer, and 1 µl Taq, 94°C for 2 min, 94°C for 30 sec, 55°C for 30 sec and 72°C for 30 sec, 18 cycles. The library was purified by AMPure at 1:1, quantified and sequenced.

### Interruption of sequencing library

1 ng cDNA, 10 µl 2xTD Buffer (the Illumina Nextera kit), and 1 µl Nextera enzyme (Illumina Nextera), 20 µl reaction system, 55°C for 7 min. 5 µl Tn5 stop buffer (the Nextera kit) was added.

### Amplification of library

25 µl reaction system above, 1 µl 10 µM primer TrueseqD501, 1 µl 10 µM primer Nextera N701 primer, 1 µl Taq enzyme, 72°C for 5 min, 94°C for 2 min, 94°C for 30 sec, 60°C for 30 sec, and 72°C for 3 sec, 18 cycles.

The library was purified by AMPure XP magnetic beads at a volume of 1:1.

100,000 PE150 reads were measured by Illumina Novaseq in each cell.

Transcriptomes and genomes were analyzed simultaneously.

Fig. 28 shows the superimposing of the HE staining of the slices on the spatial lattice chip, and the color depth of each dot indicates the number of genes obtained by measurement. The method of this application can be used in the study of technology platforms of spatial multi-omics.

The foregoing detailed description is provided in an illustrative and exemplary manner, and is not intended to limit the scope of the appended claims. Various modifications of embodiments currently listed in this application are apparent for persons skilled in the art, and are encompassed within the scope of the appended claims and their equivalents.

## Claims

1. A method for analyzing a target nucleic acid from a cell, comprising:
a) providing the discrete partition comprising the following:
i. a target nucleic acid derived from a single cell, wherein at least a part of the target nucleic acids is added with oligonucleotide adapter sequences to become target nucleic acids attached; and
ii. a solid support with at least one oligonucleotide tag attached, wherein each of the oligonucleotide tags comprises a first chain and a second chain, where the first chain comprises a barcode sequence and a hybridization sequence located at the 3' end of the barcode sequence, the second chain comprises a first portion complementary to the hybridization sequence of the first chain and a second portion complementary to the oligonucleotide adapter sequence attached to the target nucleic acid, and the first chain and the second chain form a partially double-stranded structure, or the second chain and the target nucleic acid attached form a partially double-stranded structure; and
b) in the discrete partition, the oligonucleotide tag is linked to the target nucleic acid attached, thereby producing a barcoded target nucleic acid.

2. The method according to claim 1, wherein the oligonucleotide tag is releasably attached to the solid support.

3. The method according to any one of claims 1-2, comprising releasing the at least one oligonucleotide tag from the solid support, and linking the released oligonucleotide tag to the target nucleic acid attached in b), thereby producing a barcoded target nucleic acid.

4. The method according to any one of claims 1-3, wherein the oligonucleotide tag is directly or indirectly attached to the solid support through the 5' end of its first chain.

5. The method according to any one of claims 1-4, wherein a ligase is further comprised in the discrete partition, and the ligase links the oligonucleotide tag to the target nucleic acid attached.

6. The method according to claim 5, wherein the ligase comprises T4 ligase.

7. The method according to any one of claims 1-6, wherein in the barcoded target nucleic acid, the target nucleic acid sequence is located at the 3' end of the barcode sequence.

8. The method according to any one of claims 1-7, wherein the solid support is a bead.

9. The method according to claim 8, wherein the bead is a magnetic bead.

10. The method according to any one of claims 1-9, wherein the discrete partition is a hole or droplet.

11. The method according to any one of claims 1-10, wherein the barcode sequence comprises a cell barcode sequence, and each oligonucleotide tag attached to the same solid support comprises the same cell barcode sequence.

12. The method according to claims 11, wherein the cell barcode sequence comprises at least 2 cell barcode segments separated by a linker sequence.

13. The method according to any one of claims 1-12, wherein a) comprises co-distributing the target nucleic acid derived from the single cell and the solid support attached with at least one oligonucleotide tag into the discrete partition.

14. The method according to any one of claims 1-13, wherein b) comprises linking the hybridization sequence of the first chain in the oligonucleotide tag to the oligonucleotide adapter attached to the target nucleic acid, thereby producing the barcoded target nucleic acid.

15. The method according to any one of claims 1-14, wherein b) comprises hybridizing the second part of the second chain in the oligonucleotide tag with the oligonucleotide adapter attached to the target nucleic acid, and linking the hybridization sequence of the first chain in the oligonucleotide tag to the oligonucleotide adapter attached to the target nucleic acid, thereby producing the barcoded target nucleic acid.

16. The method according to any one of claims 1-15, wherein the target nucleic acid attached comprises a unique molecular identification region.

17. The method according to claim 16, wherein the unique molecular identification region is located between the oligonucleotide adapter sequence and the target nucleic acid sequence.

18. The method according to any one of claims 1-17, wherein the oligonucleotide tag further comprises an amplification primer recognition region.

19. The method according to claim 18, wherein the amplification primer recognition region is a general amplification primer recognition region.

20. The method according to any one of claims 1-19, further comprising:
c)obtaining the characterization result of the barcoded target nucleic acid; and
d)identifying the sequence of the target nucleic acid as deriving from the single cell based at least in part on the presence of the same cell barcode sequence in the characterization result obtained in c).

21. The method according to claim 20, further comprising, releasing the barcoded target nucleic acid from the discrete partition after b) and before c).

22. The method according to any one of claims 20-21, wherein c) comprises sequencing the barcoded target nucleic acid, thereby obtaining the characterization result.

23. The method according to any one of claims 20-22, further comprising assembling continuous nucleic acid sequences of at least a part of the genome of the single cell from the sequences of the barcoded target nucleic acids.

24. The method according to claim 23, wherein the single cell is characterized based on the nucleic acid sequences of at least a part of the genome of the single cell.

25. The method according to any one of claims 1-24, wherein each of the discrete partitions comprises at most the target nucleic acids derived from the single cell.

26. The method according to any one of claims 20-25, further comprising identifying the single nucleic acid sequence in the barcoded target nucleic acid as deriving from a given nucleic acid in the target nucleic acid based at least in part on the presence of the unique molecular identification region.

27. The method according to any one of claims 20-26, wherein the target nucleic acid comprises an exogenous nucleic acid comprising that linked to proteins, lipids and/or small molecule compounds which can bind to target molecules in the cell.

28. The method according to claim 27, further comprising determining the amount of a given nucleic acid in the target nucleic acid based on the presence of the unique molecular identification region.

29. The method according to any one of claims 1-28, comprising pretreatment of the cell before a).

30. The method according to claim 29, wherein the pretreatment comprises fixing the cell.

31. The method according to claim 30, wherein, the cell is immobilized using a fixing agent selected from one or more of the group consisting of formaldehyde, paraformaldehyde, methanol, ethanol, acetone, glutaraldehyde, osmic acid and potassium dichromate.

32. The method according to any one of claims 29-31, the pretreatment comprises exposing the nucleus of the cell.

33. The method according to any one of claims 29-32, wherein the pretreatment comprises treating the cell with a detergent comprising Triton, NP-40 and/or digitonin.

34. The method according to any one of claims 1-33, wherein the target nucleic acid comprises one or more selected from the group consisting of DNA, RNA and cDNA.

35. The method according to any one of claims 20-34, further comprising amplifying the barcoded target nucleic acid after b) and before c).

36. The method according to claim 35, comprising releasing the barcoded target nucleic acid from the discrete partition after b) and before c), and the amplification is performed after the barcoded target nucleic acid is released from the discrete partition.

37. The method according to any one of claims 35-36, wherein an amplification primer is used in the amplification, and the amplification primer comprises a random guide sequence.

38. The method according to claim 37, wherein the random guide sequence is a random hexamer.

39. The method according to any one of claims 35-38, wherein the amplification comprises at least partially hybridizing the random guide sequence with the barcoded target nucleic acid and extending the random guide sequence in a template-oriented manner.

40. The method according to any one of claims 1-39, comprising releasing at least a part of the target nucleic acids from the single cell in the discrete partition to the outside of the cell, and linking the released target nucleic acids to the oligonucleotide tags in b), thereby producing the barcoded target nucleic acids.

41. The method according to any one of claims 1-40, comprising introducing at least a part of the oligonucleotide tags released from the solid support into the single cell, and linking them to the target nucleic acids in b), thereby producing barcoded target nucleic acids.

42. The method according to any one of claims 1-41, comprising co-distributing the target nucleic acid derived from the single cell and the solid support attached with at least one oligonucleotide tag into the discrete partition by using a microfluidic device.

43. The method according to claim 42, wherein the discrete partition is a droplet, and the microfluidic device is a droplet generator.

44. The method according to any one of claims 42-43, wherein the microfluidic device comprises a first input channel and a second input channel, which meet at a junction in fluid communication with an output channel.

45. The method according to claim 44, wherein the method further comprises introducing a sample comprising the target nucleic acid into the first input channel, and introducing the solid support attached with at least one oligonucleotide tag into the second input channel, thereby generating a mixture of the sample and the solid support in the output channel.

46. The method according to claim 45, wherein the output channel is in fluid communication with a third input channel at a junction.

47. The method according to claim 46, further comprising introducing oil into the third input channel, so that aqueous droplets in the water-in-oil emulsion are formed as the discrete partitions.

48. The method according to claim 47, wherein each of the discrete partitions comprises at most the target nucleic acids from the single cell.

49. The method according to any one of claims 44-48, wherein the first input channel and the second input channel form a substantially perpendicular angle with each other.

50. The method according to any one of claims 1-49, wherein the target nucleic acid comprises cDNA derived from RNA in the single cell.

51. The method according to claim 50, wherein the RNA comprises mRNA.

52. The method according to any one of claims 34-51, comprising reverse transcription of the RNA before a) and generation of the target nucleic acid attached.

53. The method according to claim 52, wherein a reverse transcription primer is used in the reverse transcription, and the reverse transcription primer comprises the oligonucleotide adapter sequence and a polyT sequence in a 5' to 3' direction.

54. The method according to claim 53, wherein the reverse transcription comprises hybridizing the polyT sequence with the RNA and extending the polyT sequence in a template-oriented manner.

55. The method according to any one of claims 1-54, wherein the target nucleic acid comprises DNA derived from the single cell.

56. The method according to claim 55, wherein the DNA comprises genomic DNA, accessible chromatin DNA, protein-bound DNA regions and/or exogenous nucleic acids that linked to proteins, lipids and/or small molecule compounds which can bind to target molecules in the cell.

57. The method according to claim 56, comprising fragmenting the DNA derived from the single cell before a).

58. The method according to claim 57, wherein the target nucleic acid attached is generated after or during the fragmentation.

59. The method according to any one of claims 57-58, wherein the fragmentation comprises breaking with ultrasound, and then adding a sequence comprising the oligonucleotide adapter to the broken DNA, thereby obtaining the target nucleic acid attached.

60. The method according to any one of claims 57-59, wherein the fragmentation comprises interrupting with DNA endonuclease and exonuclease, and then adding a sequence comprising the oligonucleotide adapter to the broken DNA, thereby obtaining the target nucleic acid attached.

61. The method according to any one of claims 57-60, wherein the fragmentation comprises integrating the sequence comprising the oligonucleotide adapter into the DNA with a transposase-nucleic acid complex, and releasing the transposase to obtain the target nucleic acid attached.

62. The method according to claim 61, wherein the transposase-nucleic acid complex comprises transposase and a transposon terminal nucleic acid molecule, wherein the transposon terminal nucleic acid molecule comprises the oligonucleotide adapter sequence.

63. The method according to any one of claims 61-62, wherein the transposase comprises Tn5.

64. The method according to any one of claims 61-63, wherein the DNA comprises a protein-bound DNA region, and the transposase-nucleic acid complex further comprises a moiety that directly or indirectly recognizes the protein.

65. The method according to claim 64, wherein the moiety that directly or indirectly recognizes the protein comprises one or more of the following groups: an antibody that specifically binds to the protein and Protein A or Protein G.

66. A composition, which comprises a plurality of solid supports each attached with at least one oligonucleotide tag, wherein each of the oligonucleotide tags comprises a first chain and a second chain, where the first chain comprises a barcode sequence and a hybridization sequence located at the 3' end of the barcode sequence, the second chain comprises a first portion complementary to the hybridization sequence of the first chain and a second portion complementary to the sequence in the nucleic acid to be tested, and the first chain and the second chain form a partially double-stranded structure, or the second chain and the target nucleic acid attached form a partially double-stranded structure; and the barcode sequence of the oligonucleotide tag comprises a common barcode domain and a variable domain, where the common barcode domains are the same in the oligonucleotide tags attached to the same solid support, and the common barcode domains are different between two or more solid supports in the plurality of solid supports.

67. A kit for analyzing a target nucleic acid from a cell, comprising the composition of claim 66.

68. The kit according to claim 67, comprising a transposase.

69. The kit according to any one of claims 67-68, further comprising at least one of a nucleic acid amplification agent, a reverse transcription agent, a fixing agent, a permeabilization agent, a ligation agent and a lysis agent.

70. A method for amplifying a target nucleic acid from a cell, comprising:
a) providing a discrete partition comprising: i. a target nucleic acid derived from a single cell, wherein at least a part of the target nucleic acids is added with an oligonucleotide adapter sequence to become a target nucleic acid attached; and ii. a solid support with at least one oligonucleotide tag attached, wherein each of the oligonucleotide tags comprises a first chain and a second chain, where the first chain comprises a barcode sequence and a hybridization sequence located at the 3' end of the barcode sequence, the second chain comprises a first portion complementary to the hybridization sequence of the first chain and a second portion complementary to the oligonucleotide adapter sequence attached to the target nucleic acid, and the first chain and the second chain form a partially double-stranded structure, or the second chain and the target nucleic acid attached form a partially double-stranded structure;
b) in the discrete partition, the oligonucleotide tag is linked to the target nucleic acid attached, thereby producing a barcoded target nucleic acid; and
c) amplifying the barcoded target nucleic acid.

71. The method according to claim 70, wherein the oligonucleotide tag is releasably attached to the solid support.

72. The method according to claim 71, comprising releasing the at least one oligonucleotide tag from the solid support, and linking the released oligonucleotide tag to the target nucleic acid attached in b), thereby producing a barcoded target nucleic acid.

73. The method according to any one of claims 70-72, wherein the oligonucleotide tag is directly or indirectly attached to the solid support through the 5' end of its first chain.

74. The method according to any one of claims 70-73, wherein a ligase is further comprised in the discrete partition, and the ligase links the oligonucleotide tag to the target nucleic acid attached.

75. The method according to claim 74, wherein the ligase comprises T4 ligase.

76. The method according to any one of claims 70-75, wherein in the barcoded target nucleic acid, the target nucleic acid sequence is located at the 3' end of the barcode sequence.

77. The method according to any one of claims 70-76, wherein the solid support is a bead.

78. The method according to any one of claims 70-77, wherein the discrete partition is a hole or droplet.

79. The method according to any one of claims 70-78, wherein the barcode sequence comprises a cell barcode sequence, and each oligonucleotide tag attached to the same solid support comprises the same cell barcode sequence.

80. The method according to claims 79, wherein the cell barcode sequence comprises at least 2 cell barcode segments separated by a linker sequence.

81. The method according to any one of claims 70-80, wherein a) comprises co-distributing the target nucleic acid derived from the single cell and the solid support attached with at least one oligonucleotide tag into the discrete partition.

82. The method according to any one of claims 70-81, wherein b) comprises linking the hybridization sequence of the first chain in the oligonucleotide tag to the oligonucleotide adapter attached to the target nucleic acid, thereby producing the barcoded target nucleic acid.

83. The method according to any one of claims 70-82, wherein b) comprises hybridizing the second part of the second chain in the oligonucleotide tag with the oligonucleotide adapter attached to the target nucleic acid, and linking the hybridization sequence of the first chain in the oligonucleotide tag to the oligonucleotide adapter attached to the target nucleic acid, thereby producing the barcoded target nucleic acid.

84. The method according to any one of claims 70-83, wherein the target nucleic acid attached comprises a unique molecular identification region.

85. The method according to claim 84, wherein the unique molecular identification region is located between the oligonucleotide adapter sequence and the target nucleic acid sequence.

86. The method according to any one of claims 70-85, wherein the oligonucleotide tag further comprises an amplification primer recognition region.

87. The method according to claim 86, wherein the amplification primer recognition region is a general amplification primer recognition region.

88. The method according to claim 87, comprising releasing the barcoded target nucleic acid from the discrete partition after b) and before c), and the amplification is performed after the barcoded target nucleic acid is released from the discrete partition.

89. The method according to any one of claims 70-88, wherein an amplification primer is used in the amplification, and the amplification primer comprises a random guide sequence.

90. The method according to claim 89, wherein the random guide sequence is a random hexamer.

91. The method according to any one of claims 70-90, wherein the amplification comprises at least partially hybridizing the random guide sequence with the barcoded target nucleic acid and extending the random guide sequence in a template-oriented manner.

92. A method for sequencing a target nucleic acid from a cell, comprising:
a) providing a discrete partition comprising: i. a target nucleic acid derived from a single cell, wherein at least a part of the target nucleic acids is added with an oligonucleotide adapter sequence to become a target nucleic acid attached; and ii. a solid support with at least one oligonucleotide tag attached, wherein each of the oligonucleotide tags comprises a first chain and a second chain, where the first chain comprises a barcode sequence and a hybridization sequence located at the 3' end of the barcode sequence, the second chain comprises a first portion complementary to the hybridization sequence of the first chain and a second portion complementary to the oligonucleotide adapter sequence attached to the target nucleic acid, and the first chain and the second chain form a partially double-stranded structure, or the second chain and the target nucleic acid attached form a partially double-stranded structure;
b) in the discrete partition, the oligonucleotide tag is linked to the target nucleic acid attached, thereby producing a barcoded target nucleic acid; and
c) sequencing the barcoded target nucleic acid.

93. The method according to claim 92, wherein the oligonucleotide tag is releasably attached to the solid support.

94. The method according to claim 93, comprising releasing the at least one oligonucleotide tag from the solid support, and linking the released oligonucleotide tag to the target nucleic acid attached in b), thereby producing a barcoded target nucleic acid.

95. The method according to any one of claims 92-94, wherein the oligonucleotide tag is directly or indirectly attached to the solid support through the 5' end of its first chain.

96. The method according to any one of claims 92-95, wherein a ligase is further comprised in the discrete partition, and the ligase links the oligonucleotide tag to the target nucleic acid attached.

97. The method according to claim 96, wherein the ligase comprises T4 ligase or T7 ligase.

98. The method according to any one of claims 92-97, wherein in the barcoded target nucleic acid, the target nucleic acid sequence is located at the 3' end of the barcode sequence.

99. The method according to any one of claims 92-98, wherein the solid support is a bead.

100. The method according to any one of claims 92-99, wherein the discrete partition is a hole or droplet.

101. The method according to any one of claims 92-100, wherein the barcode sequence comprises a cell barcode sequence, and each oligonucleotide tag attached to the same solid support comprises the same cell barcode sequence.

102. The method according to claims 101, wherein the cell barcode sequence comprises at least 2 cell barcode segments separated by a linker sequence.

103. The method according to any one of claims 92-102, wherein a) comprises co-distributing the target nucleic acid derived from the single cell and the solid support attached with at least one oligonucleotide tag into the discrete partition.

104. The method according to any one of claims 92-103, wherein b) comprises linking the hybridization sequence of the first chain in the oligonucleotide tag to the oligonucleotide adapter attached to the target nucleic acid, thereby producing the barcoded target nucleic acid.

105. The method according to any one of claims 92-104, wherein b) comprises hybridizing the second part of the second chain in the oligonucleotide tag with the oligonucleotide adapter attached to the target nucleic acid, and linking the hybridization sequence of the first chain in the oligonucleotide tag to the oligonucleotide adapter attached to the target nucleic acid, thereby producing the barcoded target nucleic acid.

106. The method according to any one of claims 92-105, wherein the target nucleic acid attached comprises a unique molecular identification region.

107. The method according to claim 106, wherein the unique molecular identification region is located between the oligonucleotide adapter sequence and the target nucleic acid sequence.

108. The method according to any one of claims 92-107, wherein the oligonucleotide tag further comprises an amplification primer recognition region.

109. The method according to claim 108, wherein the amplification primer recognition region is a general amplification primer recognition region.

110. The method according to any one of claims 92-109, further comprising assembling continuous nucleic acid sequences of at least a part of the genome of the single cell from the sequences of the barcoded target nucleic acids.

111. The method according to claim 110, wherein the single cell is characterized based on the nucleic acid sequences of at least a part of the genome of the single cell.

112. The method according to any one of claims 92-111, wherein each of the discrete partitions comprises at most the target nucleic acids derived from the single cell.

113. The method according to any one of claims 92-112, further comprising identifying the single nucleic acid sequence in the barcoded target nucleic acid as deriving from a given nucleic acid in the target nucleic acid based at least in part on the presence of the unique molecular identification region.

114. The method according to claim 113, further comprising determining the amount of a given nucleic acid in the target nucleic acid based on the presence of the unique molecular identification region.
